# EUROPEAN PATENT APPLICATION

(11) **EP 1 275 301 A1**
(43) Date of publication of application: **15.01.2003**
(21) Application number: 01420154.5
(22) Date of filing: 10.07.2001
(51) Int. Cl.: A01N 47/00, C07D 333/38, C07D 207/36, C07D 213/81, C07D 261/18, C07D 275/02, C07D 231/44, C07D 231/16, C07D 213/82, C07D 237/24, C07D 239/48, C07D 311/24, A01N 43/08, A01N 43/10, A01N 43/40, A01N 43/56, A01N 43/54, A01N 43/58, A01N 43/80, A01N 43/36, A01N 43/16

(54) **Trisubstituted heterocyclic compounds and their use as fungicides**

(71) Applicant: Bayer CropScience S.A., 69009 Lyon (FR)
(72) Inventor: Gary, Stéphanie, 69009 Lyon (FR); Lange, Tim, 69007 Lyon (FR); Muller, Benoit, 69002 Lyon (FR); Steele, Chris Richard, 69001 Lyon (FR); Briggs, Geoffrey Gower, Harpenden Herts AL5 5QR (GB); Perez, Jose, 69009 Lyon (FR); Villier, Alain, 69370 Saint-Didier-au-Mont-D'or (FR)
(74) Representative: Mérigeault, Shona

(57) **Abstract**

Compounds of general formula (I): 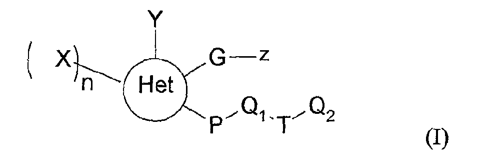 in which:
**Het** represents a five or six membered saturated, partially unsaturated or aromatic ring containing between one and six heteroatoms of the group N, O, S, in which the heterocycle is substituted in an adjacent manner with -P-Q¹-T-Q², -GZ and Y, such that the substituant -GZ is adjacent to both.
the other substituants being as defined in the description,
process for preparing these compounds,
fungicidal compositions comprising these compounds,
processes for treating plants by applying these compounds or compositions.

## Description

The present invention relates to new substituted heterocycles, their process of preparation, their use as fungicides, particularly in the form of fungicidal compositions, and methods for the control of phytopathogenic fungi of plants using these compounds or their compositions.

From the literature, substituted heterocycles with fungicidal action are known. Thus, substituted heterocycles, disclosed in particular in patent application WO-A2-01/05769 and by M. Shimano et al. (*Tetrahedron Lett.* 1998, 12745-12774), are presented as being effective against phytopathogenic fungi of plants. These compounds, and also those disclosed in patent WO-A2-01/143339, only present heterocycles that are substituted in an adjacent manner by a) amide, b) hydroxy (or a processor thereof and c) eventually an alkoxy (or thioalkoxy) radical.

Patent application WO-A-00/26191 presents picolinamide derivatives that are optionally substituted in position 4 with a methoxy radical. Patent application WO-A-95/25723 proposes 3-pyridylcarboxylic acid derivatives.

Picolinamide derivatives are also known from patent application publication JP-11 228 542. These derivatives are presented as having potential antifungal activities and low toxicity, for use in pharmaceutical products. Other picolinic acid derivatives are also known from patent application EP-A-0 690 061, in which such compounds are used as synthetic intermediates for the preparation of pyridothiadiazoles.

A first object of the present invention is to propose a new family of compounds having an improved activity, both quantitatively and qualitatively, in comparison with the known fungicides, antimycin or derivatives. By activity, is meant fungicidal activity; by quantitative improvement, is understood a better control of phytopathogenic fungi on plants and, by qualitative improvement, a broader spectrum of activity, that is to say control of a greater variety of phytopathogenic fungi of plants.

Another object of the present invention is to provide a new family of compounds having a reduced toxicity and/or phytotoxicity and/or ecotoxicity in comparison with known fungicides, particularly antimycin and its derivatives as well as the compounds disclosed in the above mentioned patents.

Another object of the present invention is to provide a new family of substituted heterocycles possessing the characteristics indicated above, notably on crops such as cereals, rice, corn, fruit trees, woodland trees, vine, oil-yielding plants, protein-yielding plants, market garden plants, solanaceous plants, beet, flax, citrus fruits, banana and ornamental plants and tobacco.

Another object of the present invention consists equally in proposing a new family of substituted heterocycles useful for the treatment and protection of lumber against fungal disease. In effect, lumber used for example for the fabrication of buildings and furniture (framework, walls, ceilings, flooring etc.), may suffer different injuries, among others, due to phytopathogenic fungi. The compounds according to the present invention allow these attacks to be combated.

Another object of the present invention comprises proposing a new family of substituted heterocycles useful in human and animal therapy. In fact, by virtue of their antifungal properties, heterocyclic salicylic acid derivatives which are the object of the present invention can prove useful for the treatment, in man and animals, of fungal diseases, such as for example mycosis and candidiasis.

Surprisingly it has been found that these objects can be achieved in whole or in part, by substituted heterocycles such as those described in the present invention.

### General definition of the invention

The invention relates to substituted heterocycles of general formula (I): 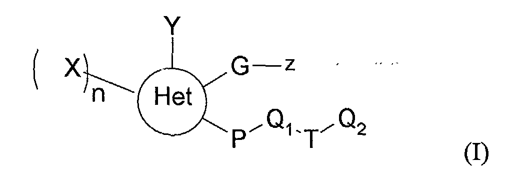 in which:
**Het** represents a five or six membered saturated, partially unsaturated or aromatic ring containing between one and six heteroatoms of the group N, O, S, in which the heterocycle is substituted in an adjacent manner with -P-Q¹-T-Q², -GZ and Y, such that the substituant -GZ is adjacent to both. Further ring members may each be substituted by Xₙ;
**n** represents the integer 0, 1, 2 or 3;
**P** is chosen from among -C(O)-, -C(S)-, -SO₂-, -C(NR¹)-, -C(N-NR¹R²)-, -P(O)(OR¹)-, -P(O)(NR¹R²)-, and -P(O)R¹-;
**R**^{**1**} **and R**^{**2**} are the same or different and are each independently chosen from hydrogen, alkyl, cycloalkyl, aryl, heteroaryl, alkenyl, alkynyl, and sulphonyl, all optionally and independently from another substituted by one or several R⁵ and/or R⁶;
**G** represents oxy (-O-), thio (-S-), sulphinyl (-SO-) or a direct bond;
**Q**^{**1**} is chosen from -OR³, -SR³ and -NR³R⁴
**P and Q**^{**1**} may furthermore together form a ring containing from 5 to 7 atoms, 2 to 3 of which being chosen from nitrogen, oxygen and sulphur, and in which ring members may optionally be substituted with one or several R⁵;
**Q**^{**2**} represents R³, which is optionally and independently from another substituted by one or several R⁵ and/or R⁶;
**R**^{**3**} **and R**^{**4**}, which are the same or different, are each chosen, independently from one another, from:
   * hydrogen;
   * aryl, alkylaryl, cycloalkylaryl, bisaryl, heteroarylaryl, cycloalkylalkylaryl, arylalkylaryl, heteroarylalkylaryl, alkanoylaryl, cycloalkylcarbonylaryl, aroylaryl, hetaroylaryl, alkoxyaryl, cycloalkoxyaryl, aryloxyaryl, heteroaryloxyaryl, alkylaminoaryl, cycloalkylaminoaryl, arylaminoaryl, heteroarylaminoaryl, alkylthioaryl, cycloalkylthioaryl, arylthioaryl, heteroarylthioaryl, where all aryl, alkyl, cycloalkyl, heteroaryl may optionally and independently from another be substituted by one or several R⁵ and/or R⁶;
   * heteroaryl, alkylheteroaryl, cycloalkylheteroaryl, arylheteroaryl, bisheteroaryl, cycloalkylalkylheteroaryl, arylalkylheteroaryl, heteroarylalkylheteroaryl, alkanoylheteroaryl, cycloalkylcarbonylheteroaryl, aroylheteroaryl, hetaroylheteroaryl, alkoxyheteroaryl, cycloalkoxyheteroaryl, aryloxyheteroaryl, heteroaryloxyheteroaryl, alkoxyalkylheteroaryl, cycloalkoxyalkylheteroaryl, aryloxyalkylheteroaryl, heteroaryloxyalkylheteroaryl, alkylaminoalkylheteroaryl, cycloalkylaminoalkylheteroaryl, arylaminoalkylheteroaryl, heteroarylaminoalkylheteroaryl, alkylaminoalkylheteroaryl, cycloalkylaminoalkylheteroaryl, arylaminoalkylheteroaryl, heteroarylaminoalkylheteroaryl, alkylthioheteroaryl, cycloalkylthioheteroaryl, arylthioheteroaryl, heteroarylthioheteroaryl, alkylthioalkylheteroaryl, cycloalkylthioalkylheteroaryl, arylthioalkylheteroaryl, heteroarylthioalkylheteroaryl, where all aryl, alkyl, cycloalkyl, heteroaryl may optionally and independently from another be substituted by one or several R⁵ and/or R⁶;
   * alkyl, cycloalkylalkyl, arylalkyl, heteroarylalkyl, alkanoylalkyl, cycloalkylcarbonylalkyl, aroylalkyl, hetaroylalkyl, alkoxyalkyl, cycloalkoxyalkyl, aryloxyalkyl, heteroaryloxyalkyl, alkylaminoalkyl, cycloalkylaminoalkyl, arylaminoalkyl, heteroarylaminoalkyl, alkylthioalkyl, cycloalkylthioalkyl, arylthioalkyl, heteroarylthioalkyl, where all aryl, alkyl, cycloalkyl, heteroaryl may optionally and independently from another be substituted by one or several R⁵ and/or R⁶;
   * cycloalkyl, alkylcycloalkyl, spirocycloalkyl, biscycloalkyl, arylcycloalkyl, heteroarylcycloalkyl, cycloalkylalkylcycloalkyl, arylalkylcycloalkyl, heteroarylalkylcycloalkyl, alkanoylcycloalkyl, cycloalkylcarbonylcycloalkyl, arylalkylcycloalkyl, heteroarylalkylcycloalkyl, alkoxycycloalkyl, cycloalkoxycycloalkyl, aryloxycycloalkyl, heteroaryloxycycloalkyl, alkoxyalkylcycloalkyl, cycloalkoxyalkylcycloalkyl, aryloxyalkylcycloalkyl, aryloxyalkylcycloalkyl, alkylaminoalkylcycloalkyl, cycloalkylaminoalkylcycloalkyl, arylaminoalkylcycloalkyl, heteroarylaminoalkylcycloalkyl, alkylaminoalkylcycloalkyl, cycloalkylaminoalkylcycloalkyl, arylaminoalkylcycloalkyl, heteroarylaminoalkylcycloalkyl, alkylthiocycloalkyl, cycloalkylthiocycloalkyl, arylthiocycloalkyl, heteroarylthiocycloalkyl, alkylthioalkylcycloalkyl, cycloalkylthioalkylcycloalkyl, arylthioalkylcycloalkyl, and heteroarylthioalkylcycloalkyl, where all aryl, alkyl, cycloalkyl, heteroaryl may optionally and independently from another be substituted by one or several R⁵ and/or R⁶;
**Z** is chosen from hydrogen, halogen, cyano, formyl, alkyl, cycloalkyl, allyl, aryl, heteroaryl, arylalkyl, propargyl, alkenyl, alkynyl, alkoxyalkyl, alkylthioalkyl, N-alkylaminoalkyl, N,N-dialkylaminoalkyl, alkanoylaminoalkyl, acyl, thioacyl, C₃-C₁₀ alkanoyl, aroyl, hetaroyl, alkoxycarbonyl, alkenoxycarbonyl, alkynyloxycarbonyl, aryloxycarbonyl, heteroaryloxycarbonyl, N-alkylaminocarbonyl, N,N-dialkylaminocarbonyl, alkylthiocarbonyl, N-alkylamidinyl, alkoxythiocarbonyl, C₁-C₆ alkylaminothiocarbonyl, alkylcarbonyl, alkoxycarbonyl, N-alkylaminocarbonyl, N,N-dialkylaminocarbonyl, N,N-dialkylaminothiocarbonyl, arylcarbonyl, heteroarylcarbonyl, aryloxycarbonyl, heteroaryloxycarbonyl, alkenylcarbonyl, alkenyloxycarbonyl, alkynylcarbonyl, alkynyloxycarbonyl, dialkylphosphatyl, diarylphosphatyl, alkylarylphosphatyl, trialkylsilyl, alkylarylsilyl, triarylsilyl, alkylsulphonyl, alkoxysulphonyl, aryloxysulphonyl, aminosulphonyl, N-alkylaminosulphonyl, N,N-dialkylaminosulphonyl, N-arylaminosulphonyl, N,N-diarylaminosulphonyl, arylsulphonyl, alkoxysulphinyl, alkylaminosulphinyl, alkylsulphinyl, and arylsulphinyl, where all alkyl, alkoxy, alkenyl, alkynyl, cycloalkyl, heteroaryl and aryl group may optionally be substituted by one or several R⁵ and/or R⁶;
**R**^{**5**} is chosen from cyano, cyanoalkyl, nitroalkyl, halogen, haloalkyl, hydroxy, hydroxyalkyl, mercapto, mercaptoalkyl, amino, aminoalkyl, hydrazino, nitrido, hydroxylamino, alkylhydroxylamino, alkoxamino, cycloalkoxamino, formyl, trialkylsilyl, triarylsilyl, thiocyanato, azido, dihydroxyphosphanyl, dialkoxyphosphanyl, alkoxy, haloalkoxy, cycloalkoxy, alkylthio, haloalkylthio, N-alkylamino, N,N-dialkylamino, N-haloalkylamino, N,N-halodialkylamino, alkoxylamino, haloalkoxamino, oxo, imino, alkoxyimino, haloalkoximino, hydrazono, alkylsulphonyl, arylsulphonyl, mono- or disubstituted aminosulphonyl, aroyl, alkanoyl, and mono- or disubstituted aminocarbonyl;
**R**^{**6**} is chosen form alkyl, cycloalkyl, alkoxyalkyl, alkylthioalkyl, N-alkylaminoalkyl, N,N-dialkylaminoalkyl, alkenyl, cycloalkenyl, alkoxyalkenyl, alkylthioalkenyl, N-alkylaminoalkenyl, N,N-dialkylaminoalkenyl, alkynyl, cycloalkynyl, alkoxyalkynyl, alkylthioalkynyl, N-alkylaminoalkynyl, N,N-dialkylaminoalkynyl, oxo, alkylimino, cycloalkylimino, alkoxyimino, alkoxyalkylimino, N-alkylaminoalkylimino, N,N-dialkylaminoalkylimino, hydrazono, alkylhydrazono, cycloalkylhydrazono, arylhydrazono, heteroarylhydrazono, alkanoyl, cycloalkanoyl, alkoxyalkanoyl, alkylthioalkanoyl, N-alkylaminoalkanoyl, N,N-dialkylaminoalkanoyl, aroyl, cycloaroyl, alkoxyaroyl, alkylthioaroyl, N-alkylaminoaroyl, N,N-dialkylaminoaroyl, aryl, cycloalkylaryl, alkoxyaryl, alkylthioalkyl, diaminoalkylaryl, alkylthio, cycloalkylthio, haloalkylthio, hydroxyalkylthio, aminoalkylthio, thioalkylthio, alkoxyalkylthio, alkylthioalkylthio, N-alkylaminoalkylthio, N,N-dialkylaminoalkylthio, alkylamino, cycloalkylamino, haloalkylamino, hydroxyalkylamino, thioalkylamino, aminoalkylamino, alkoxyalkylamino, alkoxamino, N-alkylaminoalkylamino, N,N-dialkylaminoalkylamino, alkoxylamino, alkylhydrazino, alkylsulphonylamino, cycloalkylsulphonylamino, haloalkylsulphonylamino, arylsulphonylamino, heteroaryloxy, heteroarylthio, heteroarylamino, N-alkylaminocarbonyl, N-alkylaminocarbonyl, N,N-dialkylaminocarbonyl, alkoxycarbonyl, cycloalkoxycarbonyl, haloalkoxycarbonyl, hydroxyalkoxycarbonyl, N-alkylaminoalkoxycarbonyl, N,N-diaminoalkoxycarbonyl, alkylsulphonyl, cycloalkylsulphonyl, haloalkylsulphonyl, pentafluoroalkylsulphonyl, arylsulphonyl, N-alkylaminosulphonyl, N,N-dialkyaminosulphonyl, alkysulphoxidyl, cycloalkylsulphoxidyl, arylsulphinyl, and heteroarylsulphinyl, where all aryl, alkyl, cycloalkyl, heteroaryl may optionally and independently from another be substituted by one or several R⁵;
**Y** is chosen from
   * azido, formyl, -C(O)R⁷, -SH, thiocyanato, -SiR⁷R⁸R⁹;
   * -NH₂, -NR⁷R⁸, -NR⁷-NR⁸R⁹, -ONR⁷R⁸, -ON=CR⁷R⁸, -NR⁷NR⁸(CO)R⁹, -NR⁷N=CR⁸R⁹;
   * -NR⁷C(O)OR⁸, -NR⁷C(O)SR⁸, -NR⁷C(O)NR⁸R⁹, -NR⁷C(S)OR⁸, -NR⁷C(S)SR⁸;
   * -NR⁷C(S)NR⁸R⁹, -NR⁷C(NR⁸)OR⁹, -NR⁷C(NR⁸)SR⁹, -NR⁷C(NR⁸)NR⁹R¹⁰, -NR⁷SOₙR⁷, -NR⁷SOₙOR⁷, -NR⁷SOₙNR⁷R⁸, -NR⁷SOₙSR⁷, wherein n represents 1 or 2;
   * -P(O)(OR⁷)₂, -P(O)(NR⁷R⁸)₂, -P(O)R⁷R⁸;
   * cyclobutyl, cyclopropyl, cyclohexyl;
   * -(CR⁷R⁸)ₙ-R⁵, -(CR⁷R⁸)ₙ-R⁶ wherein represents an integer from 1 to 10, except when R⁵ represents halogen and R⁶ represents alkenyl or alkynyl;
   * alkoxycarbonylaminoalkyl, haloalkoxycarbonylaminoalkyl, cycloalkoxycarbonylaminoalkyl;
   * cycloalkyloxy, alkenyloxy, alkenylamino, alkynyloxy, alkenylthio, alkynylthio;
   * R⁷OC(O)O-, R⁷SC(O)O-, R⁷R⁸NC(O)O-, R⁷OC(S)O-, R⁷SC(S)O-, R⁷R⁸NC(S)O-, R⁷OC(NR⁹)O-, R⁷SC(NR⁹)O-, R⁷R⁸NC(NR⁹)O-, alkanoyloxy, alkenoyloxy, alkynoyloxy, aroyloxy, hetaroyloxy;
   * alkoxycarbonylaminoalkyl;
   * -C(O)OH, -C(O)OR⁷, -C(O)SR⁷, -C(O)NR⁷R⁸, -C(S)OR⁷, -C(S)SR⁷, -C(S)NR⁷R⁸;
   * -C(=NR⁷R⁸)H, -C(=NR⁷)OR⁸, -C(=NR⁷)SR⁸, -C(=NR⁷)NR⁸R⁹
   * -S(O)R⁷, -SO₂R⁷, -SO₃R⁷, -SO₂NR⁷R⁸, -SO₂SR⁷, pentafluorophenylsulphonyl, alkoxysulphinyl;
   * N-alkylaminosulphinyl, and N,N-dialkylaminosulphinyl; and
   * when **Het** represents an unsaturated or partially saturated ring, Y may also be selected from =O, =NR⁶ and =S;
   * when G represents oxygen and Z represents alkyl, or when G is a direct bond and Z represents hydrogen, Y may also be chosen from halogen, nitro, cyano and alkylthio, cycloalkyl, alkenyl, alkynyl, aryl and heteroaryl;
all alkyl, cycloalkyl, alkenyl, alkynyl, aryl and heteroaryl may optionally be substituted with one or several R⁵ and/or R⁶;
**R**^{**7**}**, R**^{**8**}**, R**^{**9**} **and R**^{**10**}, which may be the same or different, are each chosen from hydrogen, linear or branched C₁-C₁₀ alkyl, cycloalkyl, alkenyl, alkynyl, aryl, heteroaryl, heterocyclyl, each being optionally substituted independently from another with one or several R⁵ and/or R⁶;
R⁷ and R⁸ on the one hand and R⁹ and R¹⁰ on the other hand, may further together form a saturated, partially saturated or unsaturated 3-8 membered ring containing optionally 0 to 3 heteroatoms chosen from among O, N, and S;
**X**_{**n**} is chosen from
   * hydrogen, halogen, -OH, -OR⁷, -ONR⁷R⁸, -SH, -SR⁷, -S(O)R⁷, -SO₂R⁷, -SO₂NR⁷R⁸, -C(O)R⁷, -C(O)OR⁷, -C(O)NR⁷R⁸, -NR⁷R⁸, nitro, thiocyanato, azido, cyano, pentafluorosulphonyl, -P(O)(OR⁷)₂, -P(O)(NR⁷R⁸)₂, -P(O)R⁷R⁸, oxo (=O) and imino (=R⁷),
   * linear or branched C₁-C₁₀ alkyl, cycloalkyl, alkenyl, alkynyl, aryl, heteroaryl, and heterocyclyl, each of them being optionally substituted independently from another with one or several R⁵ and/or R⁶;
   * Xₙ and Xₙ₊₁ may also be joined together, thus forming a saturated, partially unsaturated or totally unsaturated 4- to 8-membered ring optionally comprising one or more heteroatoms chosen from sulphur, oxygen, nitrogen and phosphorus;
all alkyl, cycloalkyl, alkenyl, alkynyl, aryl and heteroaryl may optionally be substituted with one or several R⁵ and/or R⁶;
**T** is chosen from
   * a direct bond,
   * a divalent radical chosen from -(C[R₃]₂)ₘ-, -(C[R⁵]₂)ₘ-or -(CH₂)ₘ, where m can take a value between 1 and 12, limits included, and where the said radical can optionally be interrupted or ending with one or two heteroatoms chosen from nitrogen, oxygen and/or sulphur,
   * oxyalkylene, alkoxyalkylene, carbonyl (-CO-), oxycarbonyl (-O-CO-), carbonyloxy (-CO-O-), sulphinyl (-SO-), sulphonyl (-SO₂-), oxysulphonyl (-O-SO₂-), sulphonyloxy (-SO₂-O-), oxysulphinyl (-O-SO-), sulphinyloxy (-SO-O-), thio (-S-), oxy (-O-), amino (-NR¹-), vinyl (-C=C-), ethynyl (-C≡C-), -NR¹-, -NR¹O-, -ONR¹-, -N=N-, -NR¹-NR²-, -NR¹-S-, -NR¹-SO-, -NR¹-SO₂-, -S-NR¹-, -SO-NR¹-, -SO₂-NR¹-, -CO-NR¹-O- or -O-NR¹-CO-;
as well as all eventual N-oxides, geometrical and/or optical isomers, enantiomers and/or diastereoisomers, tautomeric forms, salts and metal and metalloid complexes thereof.

Unless otherwise specified, "alkyl" refers to C₁-C₁₈ alkyl, alkenyl to C₂-C₁₈ alkenyl, alkynyl to C₂-C₁₈ alkynyl, cycloalkyl means C₃-C₁₈ cycloalkyl containing 0 to 3 heteroatoms and 0 to 2 unsaturations, bi- or tricyclic ring systems containing from 0 to 2 heteroatoms and from 0 to 2 unsaturations included, all optionally substituted by one or several R⁵ and/or R⁶.

The term alkyl, alkenyl, alkynyl and the like, as used herein, include within their scope both straight and branched groups; the term heteroaryl refers to any 5 or 6 membered aromatic ring containing one or more heteroatoms. These heteroatoms may also be fused to other aromatic systems.

The tautomeric forms of the compounds of formula (I) such as those defined above are also included in the invention. By tautomeric forms there are to be understood all of the isomeric forms described in the work "The Tautomerism of Hetero-cycles, Advances in Heterocyclic Chemistry, Supplement 1, by J Elguero, C. Martin, A.R. Katritsky and P Linda, published by Academic Press, New York, 1976, pp. 1-4.

The following generic terms are used with the following meanings:
* halogen signifies fluorine, chlorine, bromine or iodine,
* the alkyl radicals as well as groups including these alkyl radicals (alkoxy, alkylcarbonyl or acyl, etc.) include, unless indicated to the contrary, from 1 to 6 carbons atoms in a linear or branched chain and are optionally substituted,
* the halogenated alkyl, alkoxy and halocycloalkyl radicals may comprise one or more identical or different halogen atoms,
* the cycloalkyl radicals comprise from 3 to 6 carbon atoms and are optionally substituted,
* the alkenyl and alkynyl radicals, as well as groups including such radicals, comprise, unless indicated to the contrary, from 2 to 6 carbon atoms in a straight or branched chain and are optionally substituted,
* the acyl radical signifies alkylcarbonyl or cycloalkylcarbonyl, the alkyl part containing from 1 to 6 carbon atoms and the cycloalkyl part containing 3 to 6 carbon atoms, unless indicated to the contrary and are optionally substituted,
* the alkylene radical designates the divalent -(CH₂)ₘ- radical where m represents an integer equal to 1, 2, 3, 4, 5 or 6,
* the term "aryl" in "aryl" and "arylalkyl" signifies phenyl or naphthyl, optionally substituted,
* the term "heterocyclyl" in "heterocyclyl" and "heterocyclylalkyl" signifies a ring of 4 to 10 members, saturated, partially unsaturated or unsaturated, optionally substituted, comprising one or more heteroatoms, identical or different, chosen from nitrogen, oxygen, sulphur, silicon and phosphorus,
* when the amino radical is disubstituted, the two substituants are identical or different or may together with the nitrogen atom which carries them form a saturated, partially unsaturated or unsaturated nitrogen-containing heterocycle, containing 5 or 6 atoms in total,
* when the carbamoyl radical is disubstituted, the two substituants are identical or different or may together with the nitrogen atom which carries them form a saturated, partially unsaturated or unsaturated nitrogen-containing heterocycle of 5 to 6 carbon atoms in total,
* unless indicated to the contrary, the expression "optionally substituted" qualifying an organic group applies to different radicals constituting the group and indicates that the different radicals are optionally substituted by one or more radicals R⁵ and/or aryl and/or arylalkyl, identical or different,
* all aryl, alkyl, cycloalkyl, heteroaryl may optionally and independently from another be substituted by one or several R⁵ and/or R⁶.

According to a further aspect, the present invention relates to substituted heterocycles of general formula (I) as defined above and for which **Het** (representing "heterocycle") represents saturated, partially unsaturated or aromatic monocycle or bicycles containing from 4 to 10 ring units, comprising at least one heteroatom chosen from nitrogen, oxygen, sulphur, silicon and phosphorus. In case of bicyclic heterocycles, it has to be understood that the three substituants adjacent to each other (-Y, -GZ, -PQ¹TQ²) are attached to a ring bearing at least one of the heteroatoms chosen from O, N, S and P.

More particularly, the term "heterocycle" is understood as being one of the rings (i) to (v) below, provided that the rings may be substituted in the fashion according to formula (I) such that the valency rules are obeyed and that the three substituants (-Y, -GZ and -PQ¹TQ²) are adjacent to each other:
* a 5-membered ring described by formula (i): 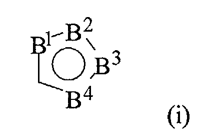 in which each of the groups of the list B¹, B², B³, B⁴ is chosen from carbon, nitrogen, oxygen and sulphur atoms such that the said list comprises from 0 to 3 carbon atoms, from 0 to 1 sulphur atom, from 0 to 1 oxygen atom and from 0 to 4 nitrogen atoms;
* a 6-membered ring described by formula (ii): 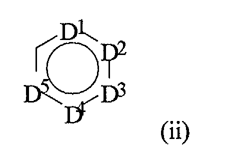 in which each of the groups of the list D¹, D², D³, D⁴, D⁵ is chosen from carbon and nitrogen atoms such that the said list comprises from 1 to 4 carbon atoms and from 1 to 4 nitrogen atoms;
* two fused rings, each being 6-membered, described by formula (iii): 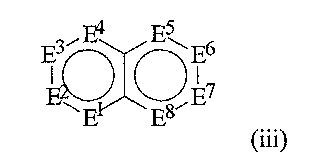 in which each of the groups in the list E¹, E², E³, E⁴, E⁵, E⁶, E⁷, E⁸ is chosen from carbon and nitrogen atoms such that the said list comprises from 4 to 7 carbon atoms and from 1 to 4 nitrogen atoms;
* a 6-membered ring and a 5-membered ring fused together, described by formula (iv): 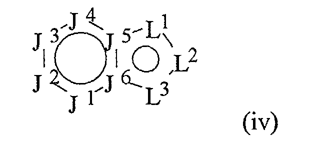 in which:
   each of the groups in the list J¹, J², J³, J⁴, J⁵, J⁶ is chosen from carbon and nitrogen atoms such that the said list comprises from 3 to 6 carbon atoms and from 0 to 3 nitrogen atoms; and
   each of the groups in the list L1, L2, L3 is chosen from carbon, nitrogen, oxygen and sulphur atoms such that the said list comprises from 0 to 3 carbon atoms, from 0 to 1 sulphur atom, from 0 to 1 oxygen atom and from 0 to 3 nitrogen atoms; and
   each of the groups in the list J¹, J², J³, J⁴, J⁵, J⁶, L¹, L², L³ is chosen such that the said list comprises from 3 to 8 carbon atoms;
* two fused rings, each being 5-membered, described by formula (v): 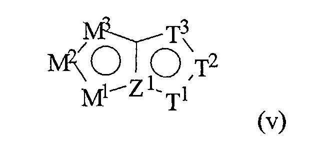 in which:
   each of the groups in the list M¹, M², M³ represents, carbon, nitrogen, oxygen or sulphur atoms such that the said list comprises from 0 to 3 carbon atoms, from 0 to 1 sulphur atom, from 0 to 1 oxygen atom and from 0 to 3 nitrogen atoms;
   each of the groups in the list T¹, T², T³ represents carbon, nitrogen, oxygen or sulphur atoms such that the said list comprises from 0 to 3 carbon atoms, from 0 to 1 sulphur atom, from 0 to 1 oxygen atom and from 0 to 3 nitrogen atoms;
   Z¹ represents a carbon or nitrogen atom;
each of the groups in the list M¹, M², M³, T¹, T², T³ is chosen such that the said list comprises from 0 to 6 carbon atoms.

In the present invention, the term "heterocycle" more specifically means:
* furan, pyrrole, thiophene,
* pyrazole, imidazole, oxazole, isoxazole, thiazole, isothiazole,
* 1,2,3-oxadiazole, 1,2,4-oxadiazole, 1,2,5-oxadiazole, 1,3,4-oxadiazole, 1,2,3-thiadiazole,
* 1,2,4-thiadiazole, 1,2,5-thiadiazole, 1,3,4-thiadiazole, 1,2,3-triazole, 1,2,4-triazole, tetrazole
* 2H-pyran, 4H-pyran, thiopyran, 1,4-dihydropyridine, N-alkyldihydropyridine,
* N-cycloalkyldihydropyridine, N-aryldihydropyridine,
* pyridine, pyrimidine, pyrazine, pyridazine, 1,2,3-triazine, 1,2,4-triazine, 1,3,5-triazine,
* 1,2,3,4-tetrazine, 1,2,3,5-tetrazine, 1,2,4,5-tetrazine,
* indazole, benzotriazole, benzoxazole, 1,2-benzisoxazole, 2,1-benzisoxazole, benzothiazole, 1,2-benzisothiazole, 2,1-benzisothiazole, 1,2,3-benzoxadiazole, 1,2,5-benzoxadiazole, 1,2,3-benzothiadiazole, 1,2,5-benzothiadiazole, quinole, isoquinole, quinoxazoline, quinazoline, phthalazol,
* pteridine, benzotriazine, 1,5-naphthyridine, 1,6-naphthyridine, 1,7-naphthyridine,
* 1,8-naphthyridine, imidazo[2,1-b]thiazole, thieno[3,4-b]pyridine, purine and pyrrolo[1,2-b]thiazole,
where every one of these heterocycles may optionally be partially or fully hydrated to partially or fully saturated compounds respectively,
where all nitrogen bearing heterocycles may optionally be present in form of N-oxides,
where the nitrogen in all nitrogen bearing heterocycles may optionally be substituted with -Y, -GZ or -PQ¹TQ².

Even more specifically, the term "heterocycle" means:
* furan, pyrrole, thiophene,
* pyrazole, imidazole, oxazole, isoxazole, thiazole, isothiazole,
* pyridine, pyrimidine, pyrazine, pyridazine, 1,2,3-triazine, 1,2,4-triazine, 1,3,5-triazine.

Most specifically, the term "heterocycle" means:
* furan, pyrrole, thiophene,
* pyrazole, imidazole, oxazole, isoxazole, thiazole, isothiazole,
* pyridine, pyrimidine.

According to another aspect, the present invention relates to substituted heterocycles of general formula (I) as defined above and for which the sub-unit P-Q¹-T-Q² is more precisely defined such that:
* P is chosen from -C(O)-, -C(S)-, -SO₂-, and -P(O)(OR¹)-,
* Q¹ represents -NR³R⁴, where R³ represents hydrogen and where R⁴ is chosen from alkyl, cycloalkyl, aryl, heteroaryl optionally substituted with one or several identical or different R⁵ and/or R⁶,
* T is chosen from a direct bond, -(CH₂)ₘ-, where m can take a value between 1 and 6, limits included, and where the said radical can optionally be interrupted or ending with one or two heteroatoms chosen from nitrogen, oxygen and/or sulphur, an oxyalkylene, alkoxyalkylene, carbonyl (-CO-), oxycarbonyl (-O-CO-), carbonyloxy (-CO-O-), sulphinyl (-SO-), sulphonyl (-SO₂-), oxysulphonyl (-O-SO₂-), sulphonyloxy (-SO₂-O-), oxysulphinyl (-O-SO-), sulphinyloxy (-SO-O-), thio (-S-), oxy (-O-), amino (-NR¹-), vinyl (-C=C-), ethynyl (-C≡C-), -NR¹-, -NR¹O-, -ONR¹-, -N=N-, -NR¹-NR²-, -NR¹-S-, -NR¹-SO-, -NR¹-SO₂-, -S-NR¹-, -SO-NR¹-, -SO₂-NR¹-, -CO-NR¹-O- and -O-NR¹-CO- radical,
* Q² is chosen from hydrogen, alkyl, cycloalkyl, aryl, and heteroaryl optionally substituted with one or several R⁵ and/or R⁶ independently chosen from another,
the other substituants being as defined above, as well as the eventual N-oxides, geometrical and/or optical isomers and enantiomers, tautomeric forms, salts and metal and metalloid complexes thereof.

According to an even further aspect, the present invention more specifically relates to substituted heterocycles of general formula (I) as defined above wherein Y is chosen from -SiR⁷R⁸R⁹, -NH₂, -NR⁷R⁸, -NR⁷-NR⁸R⁹, -ONR⁷R⁸, -ON=CR⁷R⁸, -NR⁷NR⁸(CO)R⁹, -NR⁷N=CR⁸R⁹, -NR⁷C(O)OR⁸, -NR⁷C(O)SR⁸, -NR⁷C(O)NR⁸R⁹, -NR⁷C(S)OR⁸, -NR⁷C(S)SR⁸, -NR⁷C(S)NR⁸R⁹, -NR⁷C(NR⁸)OR⁹, -NR⁷C(NR⁸)SR⁹, -NR⁷C(NR⁸)NR⁹R¹⁰, -NR⁷SOₙR⁷, -NR⁷SOₙOR⁷, -NR⁷SOₙNR⁷R⁸, -NR⁷SOₙSR⁷, where n represents 0, 1 or 2, cycloalkyloxy, alkenyloxy, alkenylamino, alkynyloxy, alkenylthio, alkynylthio, R⁷OC(O)O-, R⁷SC(O)O-, R⁷R⁸NC(O)O-, R⁷OC(S)O-, R⁷SC(S)O-, R⁷R⁸NC(S)O-, R⁷OC(NR⁹)O-, R⁷SC(NR⁹)O-, R⁷R⁸NC(NR⁹)O-, alkanoyloxy, alkenoyloxy, alkynoyloxy, aroyloxy, hetaroyloxy, -C(O)OH, -C(O)OR⁷, -C(O)SR⁷, -C(O)NR⁷R⁸, -C(S)OR⁷, -C(S)SR⁷, -C(S)NR⁷R⁸, -C(=NR⁷R⁸)H, -C(=NR⁷)OR⁸, -C(=NR⁷)SR⁸, -C(=NR⁷)NR⁸R⁹, -S(O)R⁷, -SO₂R⁷, -SO₃R⁷, -SO₂NR⁷R⁸, -SO₂SR⁷, pentafluorophenylsulphonyl, alkoxysulphinyl, N-alkylaminosulphinyl, N,N-dialkylaminosulphinyl, arylsulphinyl, and alkoxycarbonyloxy,
when Het represents an unsaturated ring, Y may also be selected from =O, =NR⁶ and =S;
* when G represents oxygen and Z represents alkyl, or when G is a direct bond and Z represents hydrogen, Y may also be chosen from halogen, nitro, cyano, alkylthio, cycloalkyl, alkenyl, alkynyl, aryl and heteroaryl;
the other substituants being as previously defined,
as well as the eventual N-oxides, geometrical and/or optical isomers and enantiomers, tautomeric forms, salts and metal and metalloid complexes thereof.

More specifically, the present invention relates to substituted heterocycles of general formula (I) as defined above for which Y is chosen from
-SiR⁷R⁸R⁹,
-NH₂, -NR⁷R⁸, -NR⁷-NR⁸R⁹, -ONR⁷R⁸, -ON=CR⁷R⁸, -NR⁷NR⁸(CO)R⁹,
-NR⁷N=CR⁸R⁹,
-NR⁷C(O)OR⁸, -NR⁷C(O)SR⁸, -NR⁷C(O)NR⁸R⁹, -NR⁷C(S)OR⁸, -NR⁷C(S)SR⁸,
-NR⁷C(S)NR⁸R⁹, -NR⁷C(NR⁸)OR⁹, -NR⁷C(NR⁸)SR⁹, -NR⁷C(NR⁸)NR⁹R¹⁰,
-C(O)OH, -C(O)OR⁷, -C(O)SR⁷, -C(O)NR⁷R⁸, -C(S)OR⁷, -C(S)SR⁷, -C(S)NR⁷R⁸,
-C(=NR⁷R⁸)H, -C(=NR⁷)OR⁸, -C(=NR⁷)SR⁸, -C(=NR⁷)NR⁸R⁹,
-S(O)R⁷, -SO₂R⁷, -SO₃R⁷, -SO₂NR⁷R⁸, -SO₂SR⁷, pentafluorophenylsulphonyl, alkoxysulphinyl, N-alkylaminosulphinyl, N,N-dialkylaminosulphinyl, arylsulphinyl, alkoxycarbonyloxy, substituants R⁷, R⁸, R⁹ as being as previously defined,
the other substituants being as previously defined,
as well as the eventual N-oxides, geometrical and/or optical isomers and enantiomers, tautomeric forms, salts and metal and metalloid complexes thereof.

More particularly, the present invention relates to substituted heterocycles of general formula (I) as defined above, which have the following characteristics, taken separately or in combination:
Xₙ is chosen from hydrogen and alkyl,
G represents oxygen, sulphur or a direct bond,
Z is chosen from hydrogen, halogen, alkyl, allyl, propargyl, alkanoyl, aroyl, hetaroyl, alkoxycarbonyl, alkenoxycarbonyl, aryloxycarbonyl, heteroaryloxycarbonyl, N-alkylaminocarbonyl, N,N-dialkylaminocarbonyl, alkylthiocarbonyl, alkoxythiocarbonyl, N-alkylaminothiocarbonyl, alkylcarbonyl, alkoxycarbonyl, N-alkylaminocarbonyl, N,N-dialkylaminocarbonyl, N,N-dialkylaminothiocarbonyl, dialkylphosphatyl, diarylphosphatyl, alkylarylphosphatyl, trialkylsilyl, alkylarylsilyl, triarylsilyl, alkylsulphonyl, arylsulphonyl, alkoxysulphonyl, aryloxysulphonyl, aminosulphonyl, N-alkylaminosulphonyl, N,N-dialkylaminosulphonyl, N-arylaminosulphonyl, N,N-diarylaminosulphonyl, alkoxysulphinyl, alkylaminosulphinyl, alkylsulphinyl and arylsulphinyl, where all alkyl, alkoxy, alkenyl, alkynyl, cycloalkyl, heteroaryl and aryl group may optionally be substituted by one or several R⁵ and/or R⁶,
Y is chosen from trialkylsilyl, amino, -NR⁷C(O)OR⁸, -NR⁷C(O)NR⁸R⁹, -NR⁷C(O)OR⁸, -NR⁷SOₙR⁷ with n = 1 or 2, S(O)R⁷, -SO₂R⁷, -SO₃R⁷, -CO₂H, C(O)OR⁷, -C(=NR⁷)H, -C(O)NR⁷R⁸; Y can also be =O, =NR⁶, =S, where Het signifies an unsaturated ring;
Y can also be halogen, nitro, alkylthio and alkoxy when G = oxygen and Z = alkyl, cycloalkyl, alkenyl, alkynyl, aryl, heteroaryl or when G = bond and Z = H,
P represents -C(O)- or -SO₂-,
T represents a direct bond, or is chosen from oxy (-O-), thio (-S-), carbonyl -C(O)-and -(CH₂)ₘ, m being an integer from 1 to 6,
Q¹ represents -NR³R⁴, where R³ represents hydrogen and R⁴ is chosen from alkyl, cycloalkyl, aryl, heteroaryl optionally substituted with one or several R⁵ and/or R⁶ independently chosen from another,
Q² is chosen from hydrogen, alkyl, cycloalkyl, aryl, heteroaryl optionally substituted with one or several R⁵ and/or R⁶ independently chosen from another,
the other substituants being as defined above, as well as the optional N-oxides, geometrical and/or optical isomers, enantiomers and/or diastereoisomers, tautomeric forms, salts and metal and metalloid complexes thereof.

Even more particularly, the present invention relates to substituted heterocycles of general formula (I) as defined above, which have the following characteristics, taken separately or in combination:
Xₙ is chosen from hydrogen, alkyl, -SO₂R⁷, -C(O)OR⁷, and -P(O)(OR⁷)₂,
G represents a direct bond or oxygen,
Z is chosen from hydrogen, halogen, alkyl, alkoxycarbonyl, and alkanoyl,
Y is chosen from trialkylsilyl, amino, -NR⁷C(O)OR⁸, -SR⁷, -SO₂R⁷, -CO₂H, C(O)OR⁷, -C(=NR⁷)H, -C(O)NR⁷R⁸,
Y can also be =O, =NR⁶, =S, where Het signifies an unsaturated ring,
Y can also be halo, nitro, alkylthio and alkoxy when G = oxygen and Z = alkyl, cycloalkyl, alkenyl, alkynyl, aryl or heteroaryl or when G = bond and Z = H,
P represents -C(O)- or -SO₂-,
Q¹ represents -NR³R⁴, where R³ represents hydrogen and R⁴ is chosen from alkyl, cycloalkyl, aryl, and heteroaryl optionally substituted with one or several R⁵ and/or R⁶ independently chosen from another,
T represents a direct bond, or is chosen from oxygen, sulphur, -C(O)-, and -(CH₂)ₘ, m having a value of between 1 and 6,
Q² is chosen from hydrogen, alkyl, cycloalkyl, aryl, and heteroaryl optionally substituted with one or several R⁵ and/or R⁶ independently chosen from another,
the other substituants being as defined above, as well as the optional N-oxides, geometrical and/or optical isomers, enantiomers and/or diastereoisomers, tautomeric forms, salts and metal and metalloid complexes thereof.

A preferred class of compounds of general formula (I) comprises the compounds having the following characteristics:
Het represents thiophene,
Y is chosen from bromine, alkylthio, -SiR⁷R⁸R⁹, -SO₂R⁷, NH₂, -N=CR⁷R⁸, -NC(O)OR⁷, CO₂H, -C(=NR⁷)H, and -SO₂R⁷,
n is equal to 1,
X¹ represents hydrogen or alkylthio,
G represents oxygen,
Z is chosen from H, alkyl, alkanoyl, alkylsulphonyl, and arylsulphonyl,
P represents -C(O)-,
Q¹ represents -NR³R⁴,
T is a direct bond or represents -O-,
Q² is chosen from hydrogen and R³, R³ being alkyl or aryl,
R⁴ represents hydrogen,
R⁷ represents alkyl or aryl,
the other substituants being as defined above, as well as the optional N-oxides, geometrical and/or optical isomers, enantiomers and/or diastereoisomers, tautomeric forms, salts and metal and metalloid complexes thereof.

Another preferred class of compounds of general formula (I) comprises the compounds having the following characteristics:
Het represents pyrrole,
Y is chosen from -NH₂, -NR⁷R⁸, -NC(O)OR⁷, CO₂H, -C(O)OR⁷, -C(O)NR⁷R⁸, -SO₂R⁷, and -CO₂H,
n represents 1 or 2,
X¹ represents hydrogen,
X² represents alkyl, -SO₂R⁷, -C(O)OR⁷, and -P(O)(OR⁷)₂,
G represents -O-,
Z is chosen from hydrogen, alkyl, alkanoyl, alkylsulphonyl, and arylsulphonyl,
P represents -C(O)-,
Q¹ represents -NR³R⁴, or -OR³,
T represents a direct bond, or -O-,
Q² is chosen from hydrogen and R³,
R³ is chosen from alkyl, and aryl,
R⁴ represents hydrogen,
R⁷ and R⁸ which are the same or different are independently chosen from alkyl, alkenyl, and aryl, R⁷ and R⁸ optionally forming an unsaturated or saturated cycle,
the other substituants being as defined above, as well as the optional N-oxides, geometrical and/or optical isomers, enantiomers and/or diastereoisomers, tautomeric forms, salts and metal and metalloid complexes thereof.

Another preferred class of compounds of general formula (I) comprises the compounds having the following characteristics:
Het represents pyrazole,
n represents 1,
X¹ is chosen from alkyl, -SO₂R⁷, -C(O)OR⁷, and -P(O)(OR⁷)₂,
Y is chosen from -NH₂, -NO₂, -NHC(O)OR⁷, -NHC(O) R⁷, -NR⁷R⁸, -SO₂R⁷, and -CO₂H,
G represents a direct bond or -O-,
Z is chosen from hydrogen, bromine, alkyl, alkanoyl, alkylsulphonyl, and arylsulphonyl,
P represents -C(O)-,
Q¹ is chosen from -NR³R⁴, and -OR³,
T represents a direct bond or -O-,
Q² is chosen from hydrogen and R³,
R³ is chosen from hydrogen, alkyl, and aryl,
R⁴ represents hydrogen,
R⁷ and R⁸ which are the same or different are independently chosen from hydrogen, alkyl, alkenyl, and aryl,
the other substituants being as defined above, as well as the optional N-oxides, geometrical and/or optical isomers, enantiomers and/or diastereoisomers, tautomeric forms, salts and metal and metalloid complexes thereof.

Another preferred class of compounds of general formula (I) comprises the compounds having the following characteristics:
Het represents isothiazole,
n represents 0,
Y is chosen from -NH₂, -NO₂, -NHC(O)R⁷, -NR⁷R⁸, -SO₂R⁷, -NHC(O)OR⁷, and -CO₂H,
G represents a direct bond or -O-,
Z is chosen from hydrogen, bromine, alkyl, alkanoyl, alkylsulphonyl, and arylsulphonyl,
P represents -C(O)-,
Q¹ is chosen from -NR³R⁴, and -OR³,
T represents a direct bond or -O-,
Q² is chosen from hydrogen and R³,
R³ is chosen from hydrogen, alkyl, and aryl,
R⁴ represents hydrogen,
R⁷ and R⁸ which are the same or different are independently chosen from hydrogen, alkyl, alkenyl, and aryl,
the other substituants being as defined above, as well as the optional N-oxides, geometrical and/or optical isomers, enantiomers and/or diastereoisomers, tautomeric forms, salts and metal and metalloid complexes thereof.

Another preferred class of compounds of general formula (I) comprises the compounds having the following characteristics:
Het represents isoxazole,
n is 0,
Y is chosen from -NH₂, -CO₂H, -NHC(O)OR⁷, -CO₂R⁷, -NR⁷R⁸, -SO₂R⁷, and -CO₂H,
G represents -O-,
Z is chosen from hydrogen, alkyl, alkanoyl, alkylsulphonyl, and arylsulphonyl,
P represents -C(O)-
Q¹ is chosen from -NR³R⁴, and OR³,
T is a direct bond or is -O-,
Q² is chosen from hydrogen and R³,
R³ is chosen from alkyl and aryl,
R⁴ represents hydrogen,
R⁷ and R⁸, which are identical or different, are independently chosen from hydrogen, alkyl, alkenyl and aryl,
the other substituants being as defined above, as well as the optional N-oxides, geometrical and/or optical isomers, enantiomers and/or diastereoisomers, tautomeric forms, salts and metal and metalloid complexes thereof.

Another preferred class of compounds of general formula (I) comprises the compounds having the following characteristics:
Het represents pyridine,
n is 1 or 2,
X¹ and X² identical or different are independently chosen from hydrogen, -SO₂R⁷, and -C(O)OR⁷,
Y is chosen from -NH₂, -NR⁷R⁸, -C(O)NR⁷R⁸, -SO₂R⁷, -NHC(O)OR⁷, -CO₂H, -CO₂R⁷, and -NHC(O)R⁷,
G represents -O-,
Z is chosen from hydrogen, alkyl, alkanoyl, alkylsulphonyl, and arylsulphonyl,
P is chosen from -C(O)-, -SO₂-, and -P(O)(OR⁷)-,
Q¹ is chosen from -NR₃R⁴, and O-R³,
T is a direct bond or is -O-,
Q² represents R³,
R³ is chosen from alkyl, and aryl,
R⁴ represents hydrogen,
R⁷ and R⁸, which are identical or different, are independently chosen from alkyl, alkenyl and aryl, or R⁷ and R⁸ may together form an unsaturated or saturated cycle,
the other substituants being as defined above, as well as the optional N-oxides, geometrical and/or optical isomers, enantiomers and/or diastereoisomers, tautomeric forms, salts and metal and metalloid complexes thereof.

Another preferred class of compounds of general formula (I) comprises the compounds having the following characteristics:
Het is chosen from pyrimidine and pyridazine,
n represents 1,
X¹ is chosen from hydrogen, alkyl, -SR⁷ and -SO₂R⁷,
Y is chosen from -NH₂, -NR⁷R⁸, CO₂H, -C(O)OR⁷, -C(O)NR⁷R⁸, -SO₂R⁷, -NHC(O)OR⁷,
G represents a direct bond or is -O-,
Z is chosen from hydrogen, alkyl, alkanoyl, alkylsulphonyl, and arylsulphonyl,
P represents -C(O)-,
Q¹ is chosen from -NR³R⁴, and -OR³,
T represents a direct bond or is -O-,
Q² is chosen from hydrogen and R³,
R³ is chosen from alkyl and aryl,
R⁴ represents hydrogen,
R⁷ and R⁸, which are identical or different, are independently chosen from alkyl, alkenyl and aryl, or R⁷ and R⁸ may together form an unsaturated or saturated cycle,
the other substituants being as defined above, as well as the optional N-oxides, geometrical and/or optical isomers, enantiomers and/or diastereoisomers, tautomeric forms, salts and metal and metalloid complexes thereof.

Another preferred class of compounds of general formula (I) comprises the compounds having the following characteristics:
Het represents pyranone,
n is chosen from 1 and 2,
X¹,X² which are identical or different, are independently chosen from hydrogen, alkyl, and alkylthio,
Y is chosen from =O, and =NR⁷,
G represents a direct bond or is -O-,
Z is chosen from hydrogen, alkyl, alkanoyl, alkylsulphonyl, and arylsulphonyl,
P represents -C(O)-,
Q¹ represents -NR³R⁴,
T represents a direct bond or is -O-,
Q² is chosen from hydrogen and R³,
R³ is chosen from alkyl and aryl,
R⁴ represents hydrogen,
the other substituants being as defined above, as well as the optional N-oxides, geometrical and/or optical isomers, enantiomers and/or diastereoisomers, tautomeric forms, salts and metal and metalloid complexes thereof.

The compounds of general formula (I) and the compounds which may be used as intermediates in the processes of preparation, and which will be defined in the description of these processes, can exist in one or more forms of geometrical isomers according to the number of double bonds in the compound. As an example, compounds of general formula (I) where Q¹ is -NR¹ or -N-NR₄R⁵ can comprise 2 different geometrical isomers denoted (*E*) or (*Z*) depending on the configuration of the two double bonds. The *E* and *Z* notation can be replaced, respectively, by the term "syn" and "anti", or "cis" and "trans". Reference is made particularly to the work of E. Eliel and S. Wilen "Stereochemistry of Organic Compounds", published by Wiley (1994), for the description and use of these notations.

The compounds of general formula (I) and compounds which may be used as intermediates in the processes of preparation, and which will be defined in the description of the processes, can exist in one or more optical isomeric or chiral forms according to the number of asymmetric centres in the compound. The invention thus also relates to all the optical isomers and their racemic or scalemic (scalemic designates a mixture of enantiomers in different proportions), as well as the mixtures of all possible stereoisomers in all proportions, including the racemic mixture. The separation of the diastereoisomers and/or optical isomers can be effected by known methods (E. Eliel *ibid*.).

The invention also relates to fungicidal compositions comprising an effective amount of at least one active material of formula (I). The fungicidal compositions according to the invention comprise, besides the active material of formula (I), agriculturally acceptable solid or liquid supports and/or surfactants that are also agriculturally acceptable. In particular, common inert supports and common surfactants can be used. These compositions include not only compositions which are ready to be applied to the plant or seed to be treated by means of a suitable device, such as a spraying or dusting device, but also concentrated commercial compositions which must be diluted before they are applied to the crop.

These fungicidal compositions according to the invention can also contain other ingredients of any kind, such as, for example, protective colloids, adhesives, thickeners, thixotropic agents, penetration agents, stabilisers, sequestering agents, etc. More generally, the active materials can be combined with any solid or liquid additive, which complies with the usual formulation techniques.

In general, the compositions according to the invention usually contain from 0.05 to 99% (by weight) of active material, one or more solid or liquid supports and, optionally, one or more surfactants.

In the present specification, the term "support" denotes a natural or synthetic, organic or inorganic material with which the active material is combined to make it easier to apply to the parts of the plant. This support is thus generally inert and should be agriculturally acceptable. The support can be solid (clays, natural or synthetic silicates, silica, resins, waxes, solid fertilisers, etc.) or liquid (water, alcohols, in particular butanol, etc., organic solvents, mineral and plant oils and derivatives thereof). Mixtures of such supports may also be used.

The surfactant can be an emulsifier, a dispersing agent or a wetting agent of ionic or nonionic type or a mixture of such surfactants. Mention may be made, for example, of polyacrylic acid salts, lignosulphonic acid salts, phenolsulphonic or naphthalenesulphonic acid salts, polycondensates of ethylene oxide with fatty alcohols or with fatty acids or with fatty amines, substituted phenols (in particular alkylphenols or arylphenols), salts of sulphosuccinic acid esters, taurine derivatives (in particular alkyl taurates), phosphoric esters of polyoxyethylated alcohols or phenols, fatty acid esters of polyols, and derivatives of the above compounds containing sulphate, sulphonate and phosphate functions. The presence of at least one surfactant is generally essential when the active material and/or the inert support are water-insoluble and when the vector agent for the application is water.

Thus, the compositions for agricultural use according to the invention can contain the active material in a very wide range, from 0.05% to 99% (by weight). Their surfactant content is advantageously between 5% and 40% by weight. Except where otherwise indicated, the percentages given in this description are weight percentages.

These compositions according to the invention are themselves in quite diverse, solid or liquid forms. As solid composition forms, mention may be made of powders for dusting (with an active material content which can be up to 100%) and granules, in particular those obtained by extrusion, by atomisation, by compacting, by impregnation of a granulated support or by granulation from a powder (the active material content in these granules being between 0.5 and 80% for the latter cases).

The fungicidal compositions according to the invention can also be used in the form of powders for dusting; compositions comprising 50 g of active material and 950 g of talc can also be used; compositions comprising 20 g of active material, 10 g of finely divided silica and 970 g of talc can also be used; these constituents are mixed together and ground and the mixture is applied by dusting.

As liquid composition forms or forms intended to constitute liquid compositions when applied, mention may be made of solutions, in particular water-soluble concentrates, emulsifiable concentrates, emulsions, concentrated suspensions and wettable powders (or powders for spraying).

The concentrated suspensions, which can be applied by spraying, are prepared so as to obtain a stable fluid product which does not become deposited and which gives good bioavailability of the active material(s). These suspensions usually contain from 5% to 75% of active material, preferably from 10% to 25%, from 0.5% to 75% of surfactants, preferably from 5% to 50%, from 0% to 10% of suitable additives, such as thickeners of organic or mineral origin, antifoaming agents, corrosion inhibitors, adhesives, preserving agents, such as, for example, Proxel GXL®, antifreezes and, as support, water or an organic liquid in which the active material is insoluble or only sparingly soluble: certain organic solid materials or mineral salts may be dissolved in the support to help prevent sedimentation or as antifreezes for the water. In certain cases, and in particular for formulations intended for treating seeds, one or more colorants may be added.

For foliar applications, the choice of surfactants is paramount for obtaining good bioavailability of the active material(s); thus, a combination of a surfactant of hydrophilic nature (HLB>10) and a surfactant of lipophilic nature (HLB<5) will preferably be used. Such combinations of surfactants are disclosed, for example, in the yet unpublished French patent No. 00/04015.

By way of example, here are 3 possible formulations of concentrated suspension type that are suitable for various crops:

### Example CS 1 (in g/kg):

This example is rather suited for monocotyledon crops (cereals, rice, etc.)
- active material 150
- surfactant of hydrophilic nature (for example Rhodasurf 860P) 300
- surfactant of lipophilic nature (for example Plurafac LF 700) 150
- ethoxylated tristyrylphenol phosphate 50
- antifoam 5
- propylene glycol 30
- Aerosil 200 20
- Attagel 50 40
- water (completion to 1 kg) 255

### Example CS 2 (in g/kg):

This example is suited rather to dicotyledon crops (vines, fruit trees, etc.)
- active material 150
- surfactant of hydrophilic nature (for example Rhodasurf 860P) 150
- ethoxylated tristyrylphenol phosphate 50
- antifoam 5
- propylene glycol 30
- Aerosil 200 20
- Attagel 50 40
- water (completion to 1 kg) 555

### Example CS 3 (in k/kg):

This example is more specifically suited to the treatment of seeds.
- active material 50
- surfactant of hydrophilic nature (for example Rhodasurf 860P) 5
- ethoxylated tristyrylphenol phosphate 15
- antifoam 1
- propylene glycol 30
- colorant 20
- Rhodopol G 1.5
- Proxel GXL 1.5
- water (completion to 1 kg) 876

The following procedure will preferably be carried out to prepare these formulations: The surfactants selected (surfactant of hydrophilic nature + surfactant of lipophilic nature + ethoxylated tristyrylphenol phosphate) are mixed with the required amount of water, using a turbo mixer; after homogenisation, the other constituents of the formulation apart from the active material are then mixed together.

Next, the active material and optionally the thickener of mineral origin (Aerosol 200 and Attagel 50) are added to give a medium of viscous consistency. The mixture obtained is then ground using a turbo mixer mill at high speed and then a ball mill until a D50 of about 1 to 3 µm and a D90 of between 3 and 8 µm are obtained. When no thickener of mineral origin is used, the thickener of natural origin (Rhodopol G) is then added and the mixture is stirred until a suitable viscosity is obtained.

The wettable powders (or powders for spraying) are usually prepared such that they contain 20 to 95% of active material, and they usually contain, in addition to the solid support, from 0 to 30% of a wetting agent, from 3 to 20% of a dispersing agent and, when necessary, from 0.1 to 10% of one or more stabilisers and/or other additives, such as penetration agents, adhesives, anticaking agents, dyes, etc.

In order to obtain the powders for spraying or wettable powders, the active materials are intimately mixed with the additional substances in suitable mixers and are ground with mills or other suitable blenders. Powders for spraying with advantageous wettability and suspension formation are thus obtained; they can be placed in suspension with water at any desired concentration and these suspensions can be used very advantageously, in particular for application, for example, to plant leaves or to seeds.

By way of example, there follow various wettable powder compositions (or powders for spraying):

### Example WP1

- Active material 50%
- Ethoxylated fatty alcohol (wetting agent) 2.5%
- Ethoxylated phenylethylphenol (dispersant) 5%
- Chalk (inert support) 42.5%

### Example WP2:

- Active material 10%
   Synthetic C13 oxo alcohol of branched type, ethoxylated with 8 to 10
   ethylene oxide (wetting agent) 0.75%
- Neutral calcium lignosulphonate (dispersant) 12%
- Calcium carbonate (inert filler) completion to 100%

### Example WP3:

This wettable powder contains the same ingredients as in the above example, in the following proportions:
- Active material 75%
- Wetting agent 1.50%
- Dispersant 8%
- Calcium carbonate (inert filler) completion to 100%

### Example WP4:

- Active material 90%
- Ethoxylated fatty alcohol (wetting agent) 4%
- Ethoxylated phenylethylphenol (dispersant) 6%

### Example WP5:

- Active material 50%
- Mixture of anionic and nonionic surfactants (wetting agent) 2.5%
- Sodium lignosulphonate (dispersant) 5%
- Kaolinic clay (inert support) 42.5%

The aqueous dispersions and emulsions, for example the compositions obtained by diluting a wettable powder according to the invention with water, are included within the general scope of the present invention. The emulsions can be of the water-in-oil or oil-in-water type and they can have a thick consistency, such as that of a "mayonnaise".

The fungicidal compositions according to the invention can be formulated in the form of water-dispersible granules, which are also included within the scope of the invention. These dispersible granules, with an apparent density generally of between about 0.3 and about 0.6, have a particle size generally of between about 150 and about 2000 and preferably between 300 and 1500 microns.

The active material content of these granules is generally between about 1% and about 90% and preferably between 25% and 90%. The rest of the granule is essentially composed of a solid support and optionally of surfactant adjuvants, which give the granule water-dispersibility properties. These granules can be essentially of two different types depending on whether the support selected is soluble or insoluble in water. When the support is water-soluble, it can be inorganic or, preferably, organic. Excellent results have been obtained with urea. In the case of an insoluble support, it is preferably inorganic, for example such as kaolin or bentonite. It is then advantageously accompanied by surfactants (in a proportion of from 2 to 20% by weight of the granule) more than half of which consists, for example, of at least one dispersant, which is essentially anionic, such as an alkali metal or alkaline-earth metal polynaphthalene sulphonate or an alkali metal or alkaline-earth metal lignosulphonate, the remainder consisting of nonionic or anionic wetting agents such as an alkali metal or alkaline-earth metal alkylnaphthalene sulphonate. Moreover, although this is not essential, other adjuvants can be added, such as antifoaming agents.

The granule according to the invention can be prepared by mixing together the required ingredients, followed by granulation according to several techniques, which are known per se (granulator, fluid bed, sprayer, extrusion, etc.). The process generally ends by a crushing operation, followed by an operation of screening to the particle size chosen within the limits mentioned above. Granules obtained as above and then impregnated with a composition containing the active material can also be used.

Preferably, it is obtained by extrusion, by performing the process as indicated in the examples below.

### Example DG1: Dispersible granules

90% by weight of active material and 10% of urea pellets are mixed together in a mixer. The mixture is then ground in a toothed roll crusher. A powder is obtained, which is moistened with about 8% by weight of water. The wet powder is extruded in a perforated-roller extruder. A granulate is obtained, which is dried and then crushed and screened, so as to retain, respectively, only the granules between 150 and 2000 microns in size.

### Example DG2: Dispersible granules

The constituents below are mixed together in a mixer:
- Active material 75%
- Wetting agent (sodium alkylnaphthalene sulphonate) 2%
- Dispersant (polysodium naphthalene sulphonate) 8%
- Water-insoluble inert filler (kaolin) 15%

This mixture is granulated in a fluid bed, in the presence of water, and then dried, crushed and screened so as to obtain granules between 0.15 and 0.80 mm in size.

These granules can be used alone, or as a solution or dispersion in water so as to obtain the desired dose. They can also be used to prepare combinations with other active materials, in particular fungicides, these being in the form of wettable powders, granules or aqueous suspensions.

The compounds of the invention can also be mixed with one or more insecticides, fungicides, bactericides, attractant acaricides or pheromones or other compounds with biological activity. The mixtures thus obtained have a broadened spectrum of activity. In particular the compounds of the present invention do not exhibit the problem of cross-resistance with strobilurin derivatives. In fact, the compounds of the present invention are active on a different biochemical site to strobilurin derivatives.

The mixtures with other fungicides are particularly advantageous, especially the mixtures with acibenzolar-S-methyl, benalaxyl, benomyl, blasticidin-S, bromuconazole, captafol, captan, carbendazim, carboxin, carpropamide, chlorothalonil, fungicidal compositions based on copper, derivatives of copper such as copper hydroxide and copper oxychloride, cyazofamide, cymoxanil, cyproconazole, cyprodinil, dichloran, diclocymet, diethofencarb, difenoconazole, diflumetorim, dimethomorph, diniconazole, dodemorph, dodine, edifenphos, epoxyconazole, ethaboxam, ethirimol, famoxadone, fenamidone, fenarimol, fenbuconazole, fenhexamide, fenpiclonil, fenpropidine, fenpropimorph, ferimzone, fluazinam, fludioxonil, flumetover, fluquinconazole, flusilazole, flusulphamide, flutolanil, flutriafol, folpel, furalaxyl, furametpyr, guazatine, hexaconazole, hymexazol, imazalil, iprobenphos, iprodione, isoprothiolane, kasugamycin, mancozeb, maneb, mefenoxam, mepanipyrim, metalaxyl and their enantiomeric forms such as metalaxyl-M, metconazole, metiram-zinc, oxadixyl, pefurazoate, penconazole, pencycuron, phosphorous acid and its derivatives such as fosetyl-Al, phthalide, probenazole, prochloraz, procymidone, propamocarb, propiconazole, pyrimethanil, pyroquilon, quinoxyfen, silthiofam, simeconazole, spiroxamine, tebuconazole, tetraconazole, thiabendazole, thifluzamide, thiophanate, for example thiophanate-methyl, thiram, triadimefon, triadimenol, triazolopyrimidines e.g. cloransulam-methyl, flumetsulam, florasulam, metosulam, tricyclazole, tridemorph, trifloxystrobin, triticonazole, derivatives of valinamide such as for example, iprovalicarb and benthiavalicarb, vinclozolin, zineb and zoxamide, as well as fungicide of the strobilurin family, for example azoxystrobin, kresoxym-methyl, metominostrobin, discostrobin, dimoxystrobin, picoxystrobin, pyraclostrobin, and trifloxystrobin.

Another subject of the invention relates to a process for curatively or preventively combating the phytopathogenic fungi of crops, characterised in that an effective (agronomically effective) and non-phytotoxic amount of an active material of formula (I), preferably in the form of a fungicidal composition according to the invention, is applied to the plant seeds or to the plant leaves and/or to the fruits of the plants or to the soil in which the plants are growing or in which it is desired to grow them.

The expression "effective and non-phytotoxic amount" means an amount of composition according to the invention which is sufficient to control or destroy the fungi present or liable to appear on the crops, and which does not entail any appreciable symptom of phytotoxicity for the said crops. Such an amount can vary within a wide range depending on the fungus to be combated, the type of crop, the climatic conditions and the compounds included in the fungicidal composition according to the invention. This amount can be determined by systematic field trials, which are within the capabilities of a person skilled in the art.

Lastly, the invention relates to a process for preventively or curatively protecting plant multiplication products, as well as the plants resulting therefrom, against fungal diseases, characterised in that the said products are coated with an effective and non-phytotoxic dose of a composition according to the invention.

The compositions according to the invention are useful for treating the seeds of cereals (wheat, rye, triticale and barley in particular), of potato, of cotton, of pea, of rape, of corn or of flax, or the seeds of woodland trees (in particular resiniferous trees) or genetically modified seeds of these plants.

It will be noted in this respect that, in the jargon of the person skilled in the art, the expression "seed treatment" in fact refers to treatment of the grains. The application techniques are well known to those skilled in the art and they may be used without a drawback in the context of the present invention. Mention may be made, for example, of film cladding or coating. Among the plant multiplication products concerned, mention may be made in particular of seeds or grains, and tubers.

As has been indicated above, the methods for coating the plant multiplication products, in particular the seeds, are well known in the art and in particular involve film-cladding or coating techniques.

The products and compositions according to the invention may also be applied as a foliar application to the plant crops, i.e. to the leaves, the flowers, the fruit and/or the trunks of the plants concerned.

Among the plants targeted by the method according to the invention, mention may be made of rice, corn, cotton, cereals, for instance wheat, barley, triticale, fruit trees, in particular apple trees, pear trees, peach trees, vines, banana trees, orange trees, lemon trees, etc., oil-yielding crops, for example rape and sunflower, market-garden crops and leguminous crops, tomatoes, lettuce, protein-yielding crops, peas, solanacea plants, for example potato, beetroot and flax, and woodland trees, as well as genetically modified homologues of these crops.

Among the plants targeted by the method according to the invention, mention may be made of
* wheat, as regards controlling the following seed diseases: fusaria (*Microdochium nivale* and *Fusarium roseum*), stinking smut (*Tilletia caries, Tilletia controversa* or *Tilletia indica*), septoria disease (*Septoria nodorum*) and loose smut;
* wheat, as regards controlling the following diseases of the aerial parts of the plant: cereal eyespot (*Tapesia yallundae*, *Tapesia acuiformis*), take-all (*Gaeumannomyces graminis*), foot blight (*F*. *culmorum*, *F*. *graminearum*), black speck (*Rhizoctonia cerealis*), powdery mildew (*Erysiphe graminis forma specie tritici*), rusts (*Puccinia striiformis* and *Puccinia recondita*) and septoria diseases (*Septoria tritici* and *Septoria nodorum*);
* wheat and barley, as regards controlling bacterial and viral diseases, for example barley yellow mosaic;
* barley, as regards controlling the following seed diseases: net blotch (*Pyrenophora graminea, Pyrenophora teres* and *Cochliobolus sativus*), loose smut (*Ustilago nuda*) and fusaria (*Microdochium nivale* and *Fusarium roseum);*
* barley, as regards controlling the following diseases of the aerial parts of the plant: cereal eyespot (*Tapesia yallundae*), net blotch (*Pyrenophora teres* and *Cochliobolus sativus*), powdery mildew (*Erysiphe graminis forma specie hordei*), dwarf leaf rust (*Puccinia hordei*) and leaf blotch (*Rhynchosporium secalis*);
* potato, as regards controlling tuber diseases (in particular *Helminthosporium solani*, *Phoma tuberosa*, *Rhizoctonia solani*, *Fusarium solani*), mildew (*Phytopthora infestans*) and certain viruses (virus Y);
* potato, as regards controlling the following foliage diseases: early blight (*Alternaria solani*), mildew (*Phytophthora infestans*);
* cotton, as regards controlling the following diseases of young plants grown from seeds: damping-off and collar rot (*Rhizoctonia solani*, *Fusarium oxysporum*) and black root rot (*Thielaviopsis basicola*);
* protein-yielding crops, for example peas, as regards controlling the following seed diseases: anthracnose (*Ascochyta pisi*, *Mycosphaerella pinodes*), fusaria (*Fusarium oxysporum*), grey mould (*Botrytis cinerea*) and mildew (*Peronospora pisi*);
* oil-bearing crops, for example rape, as regards controlling the following seed diseases: *Phoma lingam*, *Alternaria brassicae* and *Sclerotinia sclerotiorum*;
* corn, as regards controlling seed diseases: (*Rhizopus* sp., *Penicillium* sp., *Trichoderma* sp., *Aspergillus* sp., and *Gibberella fujikuroi*);
* flax, as regards controlling the seed disease: *Alternaria linicola*;
* forest trees, as regards controlling damping-off (*Fusarium oxysporum, Rhizoctonia solani*);
* rice, as regards controlling the following diseases of the aerial parts: blast disease (*Magnaporthe grisea*), bordered sheath spot (*Rhizoctonia solani*);
* leguminous crops, as regards controlling the following diseases of seeds or of young plants grown from seeds: damping-off and collar rot (*Fusarium oxysporum, Fusarium roseum*, *Rhizoctonia solani*, *Pythium sp*.);
* leguminous crops, as regards controlling the following diseases of the aerial parts: grey mould (Botrytis sp.), powdery mildews (in particular *Erysiphe cichoracearum*, *Sphaerotheca fuliginea* and *Leveillula taurica*), fusaria (*Fusarium oxysporum*, *Fusarium roseum*), leaf spot (*Cladosporium sp*.), alternaria leaf spot (*Alternaria sp.),* anthracnose (*Colletotrichum sp*.), septoria leaf spot (*Septoria sp*.), black speck (*Rhizoctonia solani*), mildews (for example *Bremia lactucae, Peronospora sp*., *Pseudoperonospora sp*., *Phytophthora sp.);*
* fruit trees, as regards diseases of the aerial parts: monilia disease (*Monilia fructigenae*, *M*. *laxa*), scab (*Venturia inaequalis*), powdery mildew (*Podosphaera leucotricha*);
* vine, as regards diseases of the foliage: in particular grey mould (*Botrytis cinerea*), powdery mildew (*Uncinula necator*), black rot (*Guignardia biwelli*) and mildew (*Plasmopara viticola*);
* beetroot, as regards the following diseases of the aerial parts: cercospora blight (*Cercospora beticola*), powdery mildew (*Erysiphe beticola*), leaf spot (*Ramularia beticola*).

Wheat and rice are the preferred plants for carrying out the method according to the invention, although all the crops, plants, plant multiplication products, flowers, wood and, as a general rule, all the plants which may suffer attack by phytopathogenic fungi, may be advantageously treated according to the method of the invention, by using one or more active materials, fungicidal compositions according to the present invention.

In the case of plant treatments, the dose of composition applied is generally and advantageously between 10 and 800 g/ha, preferably 50 to 300 g/ha of active material for applications in foliar treatment. The dose of composition applied is generally and advantageously such that the dose of active material is between 2 and 200 g of active material per 100 kg of seed, preferably between 3 and 150 g per 100 kg in the case of seed treatments. It is clearly understood that the doses indicated above are given as illustrative examples of the invention. A person skilled in the art will know how to tailor the application doses according to the nature of the crop to be treated.

The present invention also relates to a method for curatively or preventively treating, with the aid of one or more compounds according to the invention, or of a composition according to the present invention, against fungal diseases liable to grow on or inside lumber. The term "lumber" means all types of species of wood, and all types of working of this wood intended for construction, for example solid wood, high-density wood, laminated wood, plywood, etc.

Thus, the method for treating lumber according to the invention consists in placing one or more compounds of the present invention, or a composition according to the invention, in contact. This placing in contact may cover the most diverse of forms such as, for example, direct application, spraying, dipping, injection or any other suitable means.

The present invention also relates to the treatment of genetically modified plants with the compounds according to the invention or the agrochemical compositions according to the invention. Genetically modified plants are plants into whose genome a heterologous gene encoding a protein of interest has been stably integrated.

According to the invention, the expression "heterologous gene encoding a protein of interest" essentially means genes, which give the transformed plant new agronomic properties, or genes for improving the agronomic quality of the transformed plant.

Among the genes which give the transformed plants new agronomic properties, mention may be made of genes which impart a tolerance to certain herbicides, those which impart a resistance to certain insects, those which impart a tolerance to certain diseases, etc. Such genes are described in particular in patent applications WO 91/02071 and WO 95/06128.

Among the genes which impart a tolerance to certain herbicides, mention may be made of the *Bar* gene imparting tolerance to bialophos, the gene encoding a suitable EPSPS imparting a resistance to herbicides having EPSPS as target, such as glyphosate and its salts (US 4,535,060, US 4,769,061, US 5,094,945, US 4,940,835, US 5,188,642, US 4,971,908, US 5,145,783, US 5,310,667, US 5,312,910, US 5,627,061, US 5,633,435 and FR 2 736 926), the gene encoding glyphosate oxidoreductase (US 5 463 175) or a gene encoding an HPPD imparting a tolerance to herbicides having HPPD as target, such as isoxazoles, in particular isoxafutol (FR 95/06800 and FR 95/13570), diketonitriles (EP-A-0 496 630 and EP-A-0 496 631) or triketones, in particular sulcotrioine (EP-A-0 625 505, EP-A-0 625 508 and US 5 506 195). Such genes encoding an HPPD imparting a tolerance to herbicides having HPPD as target are disclosed in patent application WO 96/38567.

In the case of genes encoding EPSPS or HPPD, and more particularly for the above genes, the sequence encoding these enzymes is advantageously preceded by a sequence encoding a transit peptide, in particular for the transit peptide known as optimized transit peptide, disclosed in patent US 5 510 471.

Among the genes imparting novel insect-resistance properties, mention will be made more particularly of the genes encoding the *Bt* proteins, which are widely described in the literature, and well known to those skilled in the art. Mention will also be made of the genes encoding proteins extracted from bacteria such as *Photorabdus* (WO 97/17432 and WO 98/08932).

Among the genes imparting novel disease-resistance properties, mention will be made in particular of the genes encoding chitinases, glucanases and oxalate oxidase, all these proteins and their coding sequences being widely described in the literature, or genes encoding antibacterial and/or antifungal peptides, in particular cysteine-rich peptides containing less than 100 amino acids, such as plant thionines or defensines, and more particularly lytic peptides of all origins comprising one or more disulphide bridges between the cysteines and regions comprising basic amino acids, in particular the following lytic peptides: androctonine (WO 97/30082 and PCT/FR98/01814, filed on 18 August 1998) or drosomicin (PCT/FR98/01462, filed on 8 July 1998). Mention will also be made of the genes encoding fungal elicitor peptides, in particular the elicitins (Kamoun et al., 1993; Panabières et al., 1995).

Among the genes which modify the constitution of modified plants, mention may be made in particular of genes which modify the content and quality of certain essential fatty acids (EP-A-0 666 918) or the content and quality of proteins, in particular in the leaves and/or seeds of the said plants. Mention will be made in particular of the genes encoding proteins that are rich in sulphur-containing amino acids (WO 98/20133; WO 97/41239; WO 95/31554; WO 94/20828 and WO 92/14822).

The present invention relates more particularly to the treatment of genetically modified plants comprising a heterologous gene, which gives the plant disease-resistance properties. The heterologous gene preferentially gives the genetically modified plant a spectrum of activity that is complementary to the spectrum of activity of the compounds according to the invention. According to the invention, the expression "complementary spectrum" means a spectrum of activity for the heterologous gene which is different from the spectrum of activity of the compounds according to the invention, or a spectrum of activity relating to identical infectious agents but allowing an identical or improved control for lower application doses of compounds according to the invention.

Lastly, the invention relates to the use of the compounds according to the invention that are useful in human and animal therapy for the treatment of fungal diseases such as, for example, mycoses, dermatoses, trichophyton diseases and candidiases or diseases caused by *Aspergillus spp*., for example *Aspergillus fumigatus.*

The present invention also relates to the process of preparation of the compounds of general formula (I) and compounds useful as intermediates in the processes of preparation. The compounds can be prepared according to the general method of preparation described below. Although general, this method of preparation provides all of the operating conditions to be used for the synthesis of the compounds of formula (I) according to the present invention. It will nevertheless be understood that the skilled worker will be able to adapt this method according to the specifics of each of the compounds, which it is desired to synthesise.

The preparation of reagents used in one or other of the general methods of preparation is generally known and is generally described specifically in the prior art or in such a manner that the man skilled in the art can adapt it to the desired aim. The prior art usable by the normally skilled worker in order to establish conditions for the preparation of reagents can be found in numerous general chemistry text books such as "Advanced Organic Chemistry" by J. March, published by Wiley (1992), "Methoden der organischen Chemie" (Houben-Weyl), published by Georg Thieme Verlag or the "Chemical Abstracts" published by the American Chemical Society as well as in information data bases accessible to the public.

The following examples illustrate in a non-limiting manner several examples of fungicidal compounds according to the invention. In the following examples "MP" means "melting point" and is expressed in ° Celsius (°C); M+1 (or M-1) means the molecular ion peak, plus or minus 1 a.m.u. (atomic mass units) respectively, as observed in mass spectroscopy.

Compounds of the general formula (I) can be prepared as according to Scheme 1: 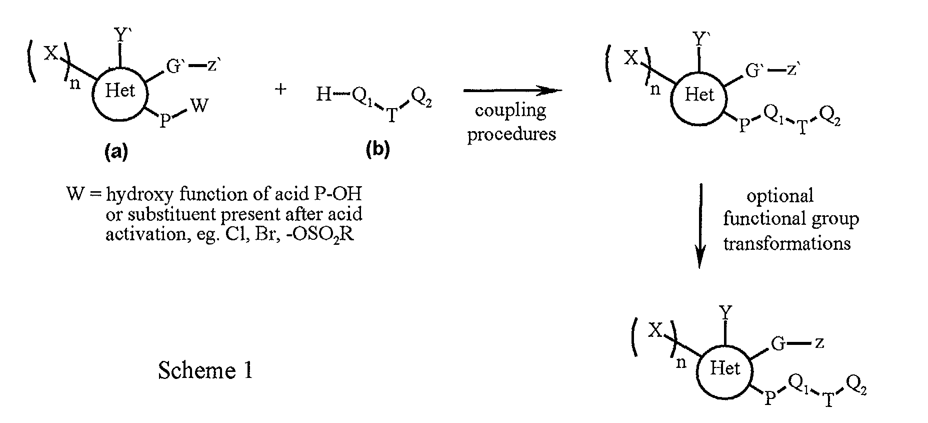 by effecting a reaction between a substituted heterocyclic acid or acid derivative (a) with a nucleophilic compound of formula (b), wherein W represents a hydroxy group (-OH) present in the free acid -P-OH or a group obtained after activation of the free acid -P-OH, like e.g. a halo radical, alkylcarbonyloxy or alkylsulphonyloxy.

The required starting materials are commercially available or readily available utilising standard procedures.

The amines utilised are either commercially available or prepared according to known procedures available to the skilled artisan. For example, certain amines are disclosed in the unpublished international patent application PCT/FR 01/00033 or in WO 01/143339.

If heterocyclic acids are obtained or purchased in the form of their alkylesters, they need be saponified according to standard deprotection procedures as described by or referenced in T. W. Greene, P. W. Greene, "*Protective Groups in Organic Synthesis",* Wiley & Sons, New York 1991. Subsequently, the heterocyclic acid or an acid derivative obtained from the acid is reacted with an amine according to known coupling procedures as published in Houben-Weyl, "Methoden der Organischen Chemie", Eugen Müller (Ed.), Thieme Publisher, Stuttgart. Subsequently, some substituants on the heterocylic carboxamide might optionally be subjected to functional group transformations as described or referenced in R. C. Larock, *"Comprehensive Organic Transformations*", VCH Publishers, Weinheim, 1989 or in computerised reaction databases like e.g. "Beilstein Commander 2000, Version 5.0", provided by MDL databases, to give another compound of the general formula (I).

### Example A : Preparation of pyrrole-2-carboxamides of Formula (I)

### Preparation of starting materials:

### 4-(4-Trifluormethylphenoxy)aniline:

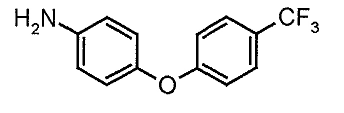

To a stirred solution of 4-trifluormethylbromobenzene (11.2 g, 50 mmol) in dimethylsulphoxide (50 mL) is added powdered potassium carbonate (3.4g, 60 mmol) and 4-aAminophenol (6.6 g, 55 mmol). The suspension is heated at 90°C for 4.5 h, then poured into water, extracted with di-isopropylether, filtered, decanted, the organic phase washed with water, dried (magnesium sulphate) and evaporated to give 9.6 g of a brown oil. To this triturated with pentane, the resulting precipitate filtered, washed with pentane and dried to give 3.3 g of the title compound (27%, mp. 72 °C).

### 1-Methyl-4-[(allyloxycarbonyl)amino]-3-methoxy-1H-pyrrole-2-carboxylic acid

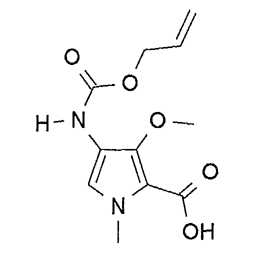

A mixture of 0.93g (3.3 mmol) of ethyl 1-methyl-4-[(allyloxycarbonyl)amino]-3-methoxy-1*H*-pyrrole-2-carboxylate (prepared according to the method described in K. Narkunan, M. A. Ciufolini, *Synthesis,* **2000,** 673-676), 3.3ml of a 2 molar sodium hydroxide aqueous solution and 3.3ml of ethanol is stirred at room temperature for 5 days. Water is added and the ethanol is evaporated. After extraction with diethyl ether, the aqueous phase is acidified to pH 2 with concentrated hydrochloric acid. Extraction with ethyl acetate, drying and concentration give 0.6g (71% yield) of a light brown solid. (M-1 = 253).

### Coupling Procedure According to Scheme 1:

### 1-Methyl-4-[(allyloxycarbonyl) amino]-3-methoxy-1H-N-para-[4-(trifluoromethyl)phenoxy]phenyl-pyrrole-2-carboxamide (19)

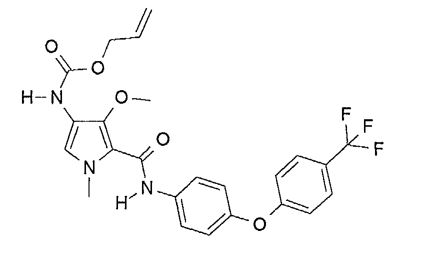

A mixture of 0.127g (0.5mmol) of 1-methyl-4-[(allyloxycarbonyl) amino]-3-methoxy-pyrrole-2-carboxylic acid, 0.127g (0.5mmol) of 4-[4-(trifluoromethyl) phenoxy]aniline, and 0.105g (0.55mmol) of 1-(3-dimethylaminopropyl)-3-ethyl-carbodiimide hydrochloride in 2.5ml of pyridine is heated at 90°C for 3 hours. After cooling, the pyridine is evaporated, the resulting dark oil is stirred at 0°C with 10ml of a 15% hydrochloric acid solution for 0.5 hour. The resulting precipitate is filtered off and washed with water and with diethyl ether. The solid is chromatographed (ethyl acetate/heptane) to give 0.08g (33%yield) of a white solid (MP = 144°C).

### Example B : Preparation of pyrrole-3-carboxamides of Formula (I)

### Coupling Procedure According to Scheme 1:

### 1-methyl-2-ethoxycarbonyl-3-hydroxy-1H-N-para-[4-(trifluoromethyl) phenoxy]phenyl-pyrrole-4-carboxamide (34)

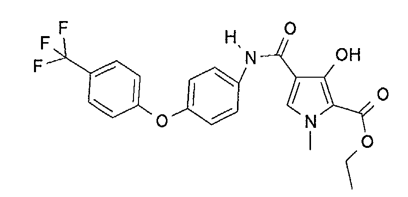

To a mixture of 1.0g (4.69 mmol) of 1-methyl-2-ethoxycarbonyl-3-hydroxy-1*H*-pyrrole-4-carboxylic acid (prepared according to the method described in K. Narkunan, M. A. Ciufolini, *Synthesis,* **2000,** 673-676), 1.42g (5.63 mmol) of 4-[4-(trifluoromethyl) phenoxy]aniline, and 0.644g (4.69 mmol) of 1-hydroxy benzotriazol in 10ml of tetrahydrofuran cooled to 0°C is added 1.08g (5.63mmol) of 1-(3-dimethylaminopropyl)-3-ethylcarbodiimide hydrochloride in 10ml of tetrahydrofuran. The resulting mixture is stirred at 0°C for 2 hours and then heated at 45°C for 16 hours. After cooling, the tetrahydrofuran is evaporated. The residue is chromatographed (ethyl acetate/heptane 9/1 to 6/4) to give 1.37g (65%yield) of a yellow solid (M+1 = 449)

### Functional group transformation according to Scheme 1:

### 1-methyl-2-ethoxycarbonyl-3-methoxy-1H-N-para-[4-(trifluoromethyl) phenoxy]phenyl- pyrrole-4-carboxamide (31)

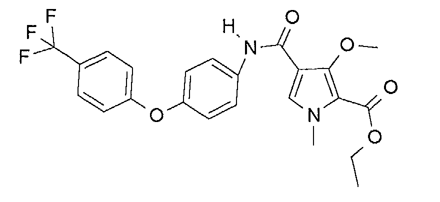

To a mixture of 0.80g (1.78 mmol) of 1-methyl-2-ethoxycarbonyl-3-hydroxy-1*H*-N*-para-*[4*-* (trifluoromethyl)phenoxy]phenyl-pyrrole-4-carboxamide, 0.49g ( 3.57 mmol) of potassium carbonate in 8ml of acetone under nitrogen, is added 0.20 mL (2.14 mmol) of dimethylsulphate. The resulting mixture is stirred at 50°C for 16 hours. After concentration, the residue is taken up in water. Extraction with diethyl ether and ethyl acetate, drying, and concentration give 0.745g (90% yield) of an orange oil (M +1 = 463)

### 1-methyl-2-hydroxycarbonyl-3-methoxy-1H-N-para-[4- (trifluoromethyl) phenoxy] phenyl- pyrrole-4-carboxamide (32)

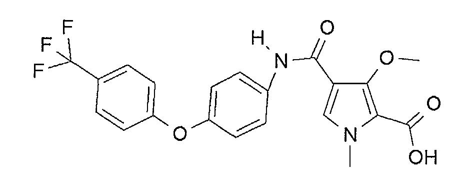

To a solution of 0.64g (1.39 mmol) of 1-methyl-2-ethoxycarbonyl-3-methoxy-1*H*-N-*para*-[4-(trifluoromethyl)phenoxy]phenyl-pyrrole-4-carboxamide in 4ml of ethanol is added 1.4ml (2.8 mmol) of a 2 molar sodium hydroxide solution. The resulting solution is stirred at room temperature for 5 days. Water is added and the ethanol is evaporated. After extraction with diethyl ether, the aqueous phase is acidified to pH 2 with concentrated hydrochloric acid. Extraction with ethyl acetate, drying and concentration give 0.5g (85% yield) of a yellowish solid. (M +1 = 435).

### 1-methyl-2-amino-3-methoxy-1H-N-para-[4-(trifluoromethyl)phenoxy]phenyl-pyrrole-4-carboxamide (33)

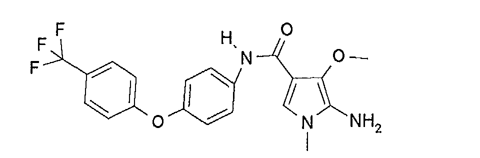

0.46g (1.05 mmol) of 1-methyl-2-hydroxycarbonyl-3-methoxy-1H-N-para-[4-(trifluoromethyl)phenoxy]phenyl-pyrrole-4-carboxamide is stirred at room temperature for 18 hours in 3.5ml ofthionyl chloride. After concentration, the residue is taken up in di-isopropylether, and the suspension is filtered off. The filtrate is concentrated to give 0.4g (85% yield) of an orange solid. A solution containing 0.370g (0.82 mmol) of the obtained acid chloride in 12 ml of acetone is added to a solution of 0.06g (1 mmol) of sodium azide in 0.2ml of water. After 0.5 hours of stirring, 12ml of water is added, and the suspension is filtered off. The solid is washed with water and heptane. The filtrate's phases are separated and the aqueous phase is made basic. Extraction with ethyl acetate, drying and concentration give 0.13mg (39% yield) of a yellow powder

### Example C: Preparation of pyrazoles of formula (I)

### Coupling procedure according to Scheme 1:

### 4-Bromo-1-methyl-5-nitro-N-para-[3-(trifluoromethyl)phenoxy]phenyl pyrazole-3-carboxamide (70)

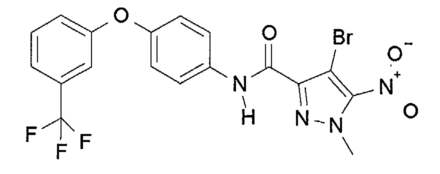

To a mixture of 0.99g (3.96 mmol) of 1-methyl-4-bromo-5-nitropyrazole-3 -carboxylic acid (prepared according to the method described in Y. Manaev, M. Andrea, V. Perevalov, B. Stepanov, V. Dubrovskaya, V. Seraya, *J*. *Gen*. *Chem. USSR* (*Engl*. *Trans*.*)* **1982,** 2291-2296), 1.00g (3.96 mmol) of 4-[3-(trifluoromethyl) phenoxy]aniline, and 0.05g (0.39 mmol) of 1-hydroxybenzotriazole in 50 ml of dichloromethane with ice cooling, is added 0.83g (4.35 mmol) of 1-(3-dimethyl-aminopropyl)-3-ethylcarbodiimide hydrochloride in portions. The resulting mixture is stirred at room temperature for 2 hours, and diluted with 150 mL dichloromethane and 100ml of a 1 molar hydrochloric acid solution. The two phases solution is well shaken, and the phases are separated. The dichloromethylated solution is dried and concentrated to give a yellow solid, which is washed with di-isopropyl ether to give 1.56g (81% yield) of compound **70** as a solid (M+1 = 485).

### Functional group transformation according to Scheme 1:

### 1-Methyl-4-hydroxy-5-amino-N-para-[3-(trifluoromethyl) phenoxy] phenyl pyrazole-3-carboxamide (71)

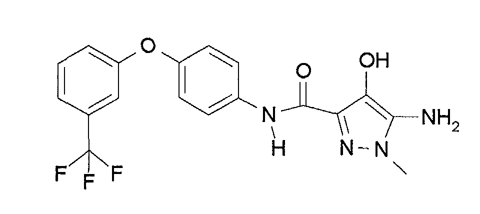

A mixture of 1.5g (3.1mmol) of 1-methyl-4-bromo-5-nitro-N*-para*-[3-(trifluoromethyl)phenoxy]phenyl pyrazole-3-carboxamide, 2.5g (0.065 mmol) of sodium hydroxide, 25ml of water, 25ml of ethanol and 200mg (0.8 mmol) of copper bromide is stirred at 90°C for 1 hour, 25ml of water is added and the mixture is stirred at 100°C for 3 hours. After cooling, the mixture is diluted with 200ml of water and made acidic with concentrated hydrochloric acid. After extraction with ethyl acetate, drying and concentration to 5ml, diethyl ether is added and after 5 minutes of stirring, the resulting solid is filtered off and washed with more diethyl ether to provide 0.54g of a solid. Of this solid, 0.47g (1.11mmol) were mixed with 0.10g of 5% palladium on charcoal in 50ml of methanol and stirred at room temperature under hydrogen at 60 psi for 3 hours. Filtration through silica and washing with acetic acid, concentration give a green solid which is washed with diethyl ether to give 0.15g (34% yield) of **71** as a grey solid (M+1 = 393).

### Example D: Preparation of Isoxazoles of Formula (I)

### Preparation of starting material:

### 3-(methoxycarbonyl)amino-4-methoxy-isoxazole-5-carboxylic acid

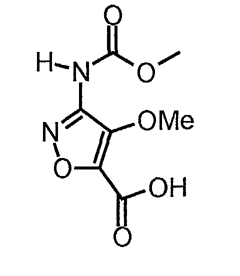

A mixture of 2.50g (0.011mmol) of methyl-3-(methoxycarbonyl)amino-4-methoxy-isoxazole-5-carboxylate (prepared according to the method described in W. Kloetzer, J. Schantz, *Monatsh*. *Chem.,* **1965,**102-115) and 1.30g of powdered sodium hydroxide dissolved in 9 ml of water is heated at 90°C for 3 hours. After cooling, the mixture is acidified to pH = 2 with concentrated hydrochloric acid, controlling the temperature so as not to exceed 5°C. After 1 hour of stirring at 0°C, the precipitate obtained is filtered off and washed with cold water. There is obtained 2.01g (86% yield) of a white solid (M+1 = 217)

### Coupling procedure according to Scheme 1:

### 3-[(Methoxycarbonyl)amino]-N-para-[4-(trifluoromethyl)phenoxy]phenyl-isoxazole-5-carboxamide (44)

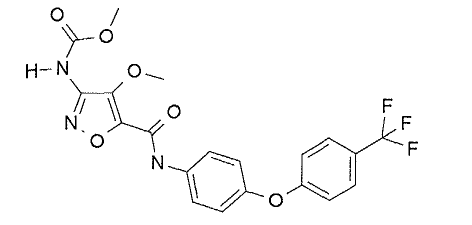

A mixture of 0.162g (0.75mmol) of 3-(methoxycarbonyl)amino-4-methoxy-isoxazole-5-carboxylic acid, 0.172g (0.68mmol) of 4-[4-(trifluoromethyl) phenoxy]aniline and 0.212g (0.75mmol) of 4-(4,6-dimethoxy-1,3,5-triazin-2-yl)-4-methylmorpholinium chloride in 5ml of ethanol is heated at 70°C for 6 hours, and then stirred at room temperature for 16 hours. After evaporation of the ethanol, the residue is taken up in ethyl acetate and the resulting solution is washed successively with a saturated solution of sodium hydrogenocarbonate, water, 2% solution of hydrochloric acid, and water. After drying and concentrating, the residue is chromatographed (ethyl acetate/heptane, 2:8) to give 0,122g (37% yield) of a cream solid (M+1 = 452).

### Functional group transformation according to Scheme 1:

### 3-Amino-4-hydroxy-N-para-[4-(trifluoromethyl) phenoxy] phenylisoxazole-5-carboxamide (51)

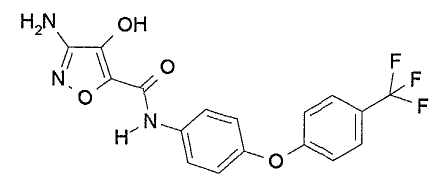

To 0.122g (0.27mmol) of 3-[(methoxycarbonyl)amino]-N-*para*-[4-(trifluoromethyl) phenoxy]phenylisoxazole-5-carboxamide in 2ml of dichloromethane at 0°C, 0.62ml (0.62mmol) of a 1 molar solution of boron tribromide in dichloromethane is added drop-wise. The mixture is stirred for 4 hours with the temperature being kept between 0 and 10°C, then for 20 hours at room temperature. The reaction mixture is poured into a saturated solution of sodium hydrogenocarbonate, and the phases are separated. The aqueous phase is further extracted with ethyl acetate. The combined organic phases are dried and concentrated. The residue is stirred in acetonitrile and the resulting suspension is filtered off to give a cream solid.

### Example D: Preparation of Isoxazole-3-carboxamides of Formula (I)

### Coupling procedure according to Scheme 1:

### 2-methoxycarbonyl-3-methoxy-N-para-[4-(trifluoromethyl)phenoxy]phenyl isoxazole-4-carboxamide (52)

To a suspension of 1g (4.4 mmol) of 2-azidocarbonyl-3-methoxy-isoxazole-4-carboxylic acid (prepared according to the method described in W. Kloetzer, J. Schantz, *Monatsh*. *Chem.,* **1965**, 102-115) in 10ml of water cooled at 0°C, is added drop-wise a solution of 3.36g (13.2 mmol) of 4-[4-(trifluoromethyl)phenoxy]aniline in 15ml of tetrahydrofuran. The resulting mixture is stirred at 0°C for 5 hours and left to stand at room temperature for 16 hours. After concentration, the residue is taken up in ethyl acetate and the organic phase is washed successively with a 1% hydrochloric acid solution, and with brine. Drying and concentration give a solid which is purified on silica to give 0.72g (38% yield) of a white solid (M+1 = 436).

### Functional group transformation according to Scheme 1:

### 2-hydroxycarbonyl-3-hydroxy-N-para-[4- (trifluoromethyl) phenoxy] phenyl-isoxazole-4-carboxamide (53)

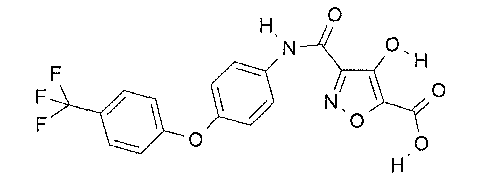

A mixture of 0.64g ( 1.47mmol) of 2-methoxycarbonyl-3-methoxy-N-para-[4-(trifluoromethyl) phenoxy] phenyl-isoxazole-4-carboxamide, 0.98g ( 7.4 mmol) of lithium iodide, and 0.12g (1.47 mmol) of sodium acetate in 5ml of dimethylformamide is heated at 90°C for 8 hours, left to stand at room temperature and heated at 90°C for 6 hours. After cooling, 15ml of water is added, the solution is made basic with a 30% sodium hydroxide solution and washed 3 times with diethyl ether. The aqueous phase is acidified with a concentrated hydrochloric acid solution. After extraction with ethyl acetate, the organic phase is washed with a saturated lithium chloride solution, dried, and concentrated to give 0.33g (56% yield) of a solid.

### Example C : Preparation of Pyridine-4-carboxamides of Formula (I):

### Preparation of starting material:

### [(2-Diethylamino-oxazolo [4,5-b3] pyridine]-4-carboxylic acid

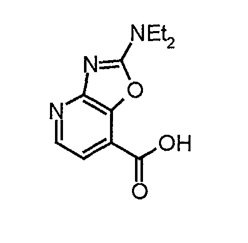

A solution of *t*-butyl-[(2-diethylamino-oxazolo[4,5-b3]pyridine]-4-carboxylate (0.76g, 2.61 mmol) was prepared as described in the literature (R. K. Russel et al., *Tetrahedron Lett.* **1993,** *34,* 203-206) and the crude material refluxed for 3 hours in formic acid (15 mL). Then all volatiles were removed, the residue taken up in trichloromethane out of which the pure material precipitated upon cooling (577 mg, 94%). (M+1 = 236).

### Coupling procedure and functional group transformation according to Scheme 1:

### [4-(Trifluoromethyl)phenoxy]anilino-(2-amino-3-hydroxy)pyridyl-4 -carboxamide (38)

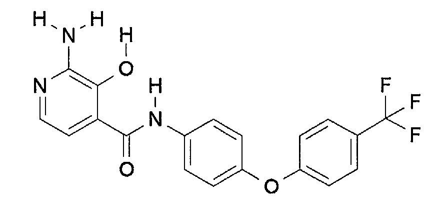

To a mixture of [(2-diethylamino-oxazolo[4,5-b3]pyridine]-4-carboxylic acid (0.30g, 1.28 mmol), 4-dimethylaminopyridine (16 mg, 0.13 mmol) and 4-(trifluoromethyl) phenoxy]anilin (0.39 g, 1.53 mmol) in pyridine (15 mL) was added dimethylaminopropylethylcarbodi-imide (0.28 g, 1.79 mmol). The reaction mixture was stirred for 3d, then all volatiles removed, the crude taken up in ethylacetate, extracted with pH 4 buffer solution, the organic phase separated, dried (magnesium sulphate), evaporated and the residue purified by chromatography (silica, heptane/ethyl acetate) to give 256 mg of a yellow solid. 100 mg of this solid were dissolved in dimethylsulphoxide (2 mL) to which was added 50% aqueous potassium hydroxide (3 mL), the solution was stirred for 2 h at 80°C, then cooled to room temperature, poured into pH4 buffer solution and extracted with ethyl acetate. After drying the organic phase (magnesium sulphate),evaporation of all volatiles and chromatography, 31 mg of the desired product was obtained as yellow solid (38%). (M-1 = 388).

### Example D: Preparation of isothiazoles of formula (I):

### Coupling procedure according to Scheme 1:

### 5-Nitro-N-para-[4-(trifluoromethyl)phenoxy]phenyl-isothiazole-3-carboxamide (58)

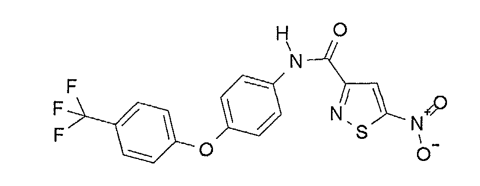

To 0.8g (4.6mmol) of 5-nitro-isothiazole-3-carboxylic acid (prepared according to the method described in R. J. A. Walsh, K. R. H. Woolbridge, *J*. *Chem*. *Soc*. *Perkin Trans 1*, **1972**,1247-1249) is added 14 ml of thionyl chloride under nitrogen, and the resulting mixture is stirred at reflux for 3 hours. After cooling, the solvent is evaporated to give a solid. This solid is suspended in a mixture of 20ml of diethyl ether and 20ml of dichloromethane. The suspension is added to a mixture of 2.74g (10.8mmol) of 4-[4-(trifluoromethyl) phenoxy] aniline and 2.5ml (18.3mmol) of triethylamine in 40ml of diethyl ether. The resulting mixture is stirred at room temperature for 4 hours and left to stand at room temperature overnight. The resulting suspension is filtered, the filtrate is washed with a saturated sodium chloride solution, then dried and concentrated to give a brown solid which is chromatographed (ethyl acetate/heptane) to give 1.2g (63%yield) of an orange solid (M+1 =410).

### Functional group transformation according to Scheme 1:

### 5-Amino-N-para-[4-(trifluoromethyl)phenoxy]phenyl-isothiazole-3-carboxamide (57)

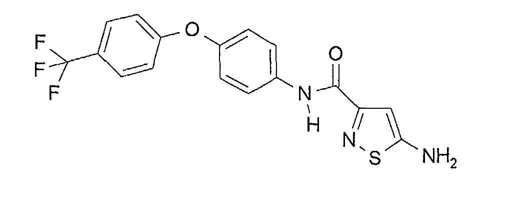

To a solution of 1.05g (2.57mmol) of 5-nitro-N-para-[4-(trifluoromethyl)phenoxy] phenyl-isothiazole-3-carboxamide in 5ml of concentrated hydrochloric acid is added a solution of 2.5g (12.8mmol) of tin chloride in 5ml of concentrated hydrochloric acid and the resulting mixture is heated at 70°C for 16 hours. After cooling, the solvent is evaporated, and the residue is taken up in water. The aqueous phase is made basic with a 30% sodium hydroxide solution. After extraction with ethyl acetate, drying, and concentration, the residue is chromatographed (ethyl acetate/heptane) to give 0.580g (60%mmol) of a light brown solid (M+1 = 380).

### Example E: Preparation of Thiophene-2-carboxamides of Formula (I):

### Coupling procedure according to Scheme 1:

### 4-Bromo-3-methoxy-N-4-[4(trifluoromethyl)phenoxy]phenyl-thiophene-2-carboxamide (3)

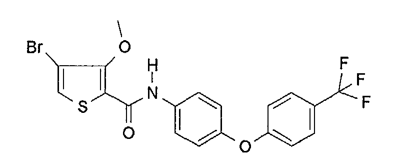

To 2.39g (10mmol) of 4-bromo-3-methoxy thiophene-2-carboxylic acid ( prepared according to the method of C.Corral, M.B.El-Ashmawy, J.Lissavetzky, A.Basilio, A.Giraldez, *Eur*.*J*.*Med*.*Chem*. **1987,** *22,* 251-254) was added 10ml ofthionyl chloride, the mixture was refluxed for 2 hours, cooled to room temperature and the excess thionyl chloride removed by evaporation. The crude acid chloride in 10ml of dry tetrahydrofuran was added drop wise to a stirred mixture of 0.53g (10mmol) of 4-(4-trifluorophenoxy)aniline and 1.01g (10mmol) of triethylamine in 20ml of dry tetrahydrofuran at 0°C, the mixture stirred at room temperature for 18 hours, treated with 150mL of water and extracted with diethyl ether, dried and evaporated to yield a solid, which was washed with pentane to yield 4.43g (94% yield) of a buff solid (M+1 = 473).

### Functional group transformation according to Scheme 1:

### 2-N-4-[4(Trifluoromethyl)phenoxy]phenylcarboxamide-3-methoxy thiophene-4-carboxylic acid (6)

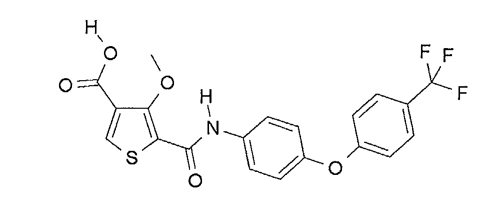

To 2.00g (4.24mmol) of 4-bromo-3-methoxy-N-4-[4-(trifluoromethyl) phenoxy]phenyl-thiophene-2-carboxamide in 40ml of dry tetrahydrofuran at -78°C, was added drop-wise 6ml (8.9mmol) of n-butyllithium (1.6M in hexane) the reaction stirred at -78°C for 1 hour before he mixture was poured onto an excess of dry ice and allowed to warm to room temperature. After treatment with 50mL of water and extraction with diethyl ether, the aqueous fraction was acidified to pH3 with aqueous hydrogen chloride and extracted with dichloromethane, dried and evaporated to yield a solid, washed with 5% diethyl ether in pentane and filtered to yield 1.11g (54% yield) of a buff solid (M+1= 438).

### 4-[(Allyloxycarbonyl)amino]-3-methoxy-N-4-[4-(trifluoromethyl)phenoxy] phenyl-thiophene-2-carboxamide (8)

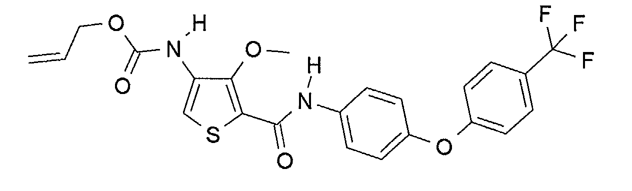

To 0.5g (1.14mmol) of 2-N-4-[4(trifluoromethyl)phenoxy]phenyl carboxamide-3-methoxy thiophene-4-carboxylic acid in 5ml of acetonitrile under argon was added 0.116g (1.14mmol) of triethylamine and 0.315g (1.14mmol) of diphenylphosphorylazide, the mixture was heated at 85°C for 2 hours, 2.5ml of allyl alcohol was added and the heating continued for 18 hours. The mixture was cooled to room temperature and evaporated, the residue was chromatographed (dichloromethane/heptane) to yield 0.15g (27% yield) of a pale yellow solid (M+1 = 493).

### 4-Methylthio-3-methoxy-N-4-[3-(trifluoromethyl)phenoxy]phenyl-thiophene-2-c arboxamide (2)

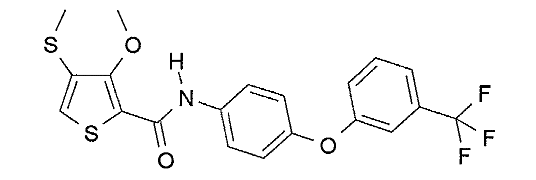

To 0.1g (0.21mmol) of 4-bromo-3-methoxy-N-4-[3(trifluoromethyl) phenoxy]phenyl-thiophene-2-carboxamide in 2ml of dry tetrahydrofuran, at -78°C, was added drop-wise 0.3mL (0.45mmol) of n-butyl lithium (1.6M in hexane). The mixture was stirred at -78°C for 1 hour, treated with 0.02g ((0.21mmol) of dimethyl disulphide in 0.5ml of dry tetrahydrofuran, stirred at -78°C for 3 hours and 18 hours at room temperature. The mixture was poured into 25 ml of water and extracted with dichloromethane, dried and evaporated to yield an oil, chromatographed (dichloromethane/heptane) to yield 0.035g (38% yield) of a colourless oil (M+1 = 440).

### 4-Methylsulphonyl-3-methoxy-N-4-[3-(trifluoromethyl) phenoxy] phenyl-thiophene-2-carboxamide (5)

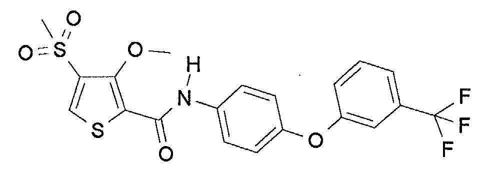

To 0.124g (0.282mmol) of 4-methylthio-3-methoxy-N-4-[3-(trifluoromethyl) phenoxy]phenyl-thiophene-2- carboxamide in 2ml of dichloromethane, at 0°C, was added 0.14g (0.565mmol) of 70% m-chloroperoxybenzoic acid, the mixture was stirred at room temperature for 18 hours, treated with 20mL of a saturated solution of sodium metabisulphite, extracted with dichloromethane, dried and evaporated to yield a buff solid, chromatographed (dichloromethane/heptane) to yield 0.104g (78% yield) of a buff solid (M+1 = 472).

### 4-Trimethylsilyl-3-methoxy-N-4-[3-(trifluoromethyl)phenoxy]phenyl-thiophene-2-carboxamide (4)

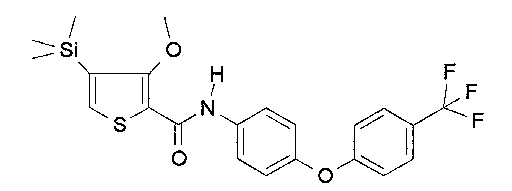

To 0.2g (0.42mmol) of 4-bromo-3-methoxy-N-4-[3(trifluoromethyl)phenoxy] phenyl-thiophene-2-carboxamide in 5ml of dry tetrahydrofuran, at -78°C, was added drop-wise 0.6ml (0.9mmol) of n-butyl lithium (1.6M in hexane). The mixture was stirred at -78°C for 1 hour, treated with 0.097g (0.89mmol) of trimethylsilyl chloride in 1ml of dry tetrahydrofuran, stirred at -78°C for 2 hours and 18 hours at room temperature. The mixture was poured into 50 ml of water and extracted with dichloromethane, dried and evaporated to yield an oil, chromatographed (dichloromethane/heptane) to yield 0.094g (48% yield) of a colourless oil (M+1 = 466).

### 5-Methylthio-4-bromo-3-methoxy-N-4-[3-(trifluoromethyl phenoxy] phenyl-thiophene-2- carboxamide (7)

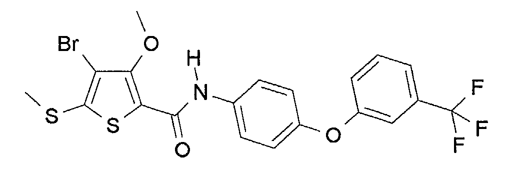

To 1.3g (2.75mmol) of 4-bromo-3-methoxy-N-4-[3(trifluoromethyl)phenoxy]phenyl thiophene-2-carboxamide in 25ml of dry tetrahydrofuran, at -78°C, was added drop-wise 3.8ml (0.45mmol) of n-butyl lithium (1.6M in hexane). The mixture was stirred at -78°C for 3 hours, treated with 0.26g (2.75mmol) of dimethyl disulphide in 5ml of dry tetrahydrofuran, stirred for 18 hours at room temperature. The mixture was poured into 100 ml of water and extracted with dichloromethane, dried and evaporated to yield an oil, chromatographed (dichloromethane/heptane) to yield 0.164g (12% yield) of a colourless oil (M+1 = 520).

All compounds of the present invention may be prepared by using identical and/or similar techniques as the ones described here above.

Tables I to XIII, which follow, present, as illustrative examples only, some of the compounds that are encompassed by the present invention. The invention shall therefore not be understood as limited to these examples.

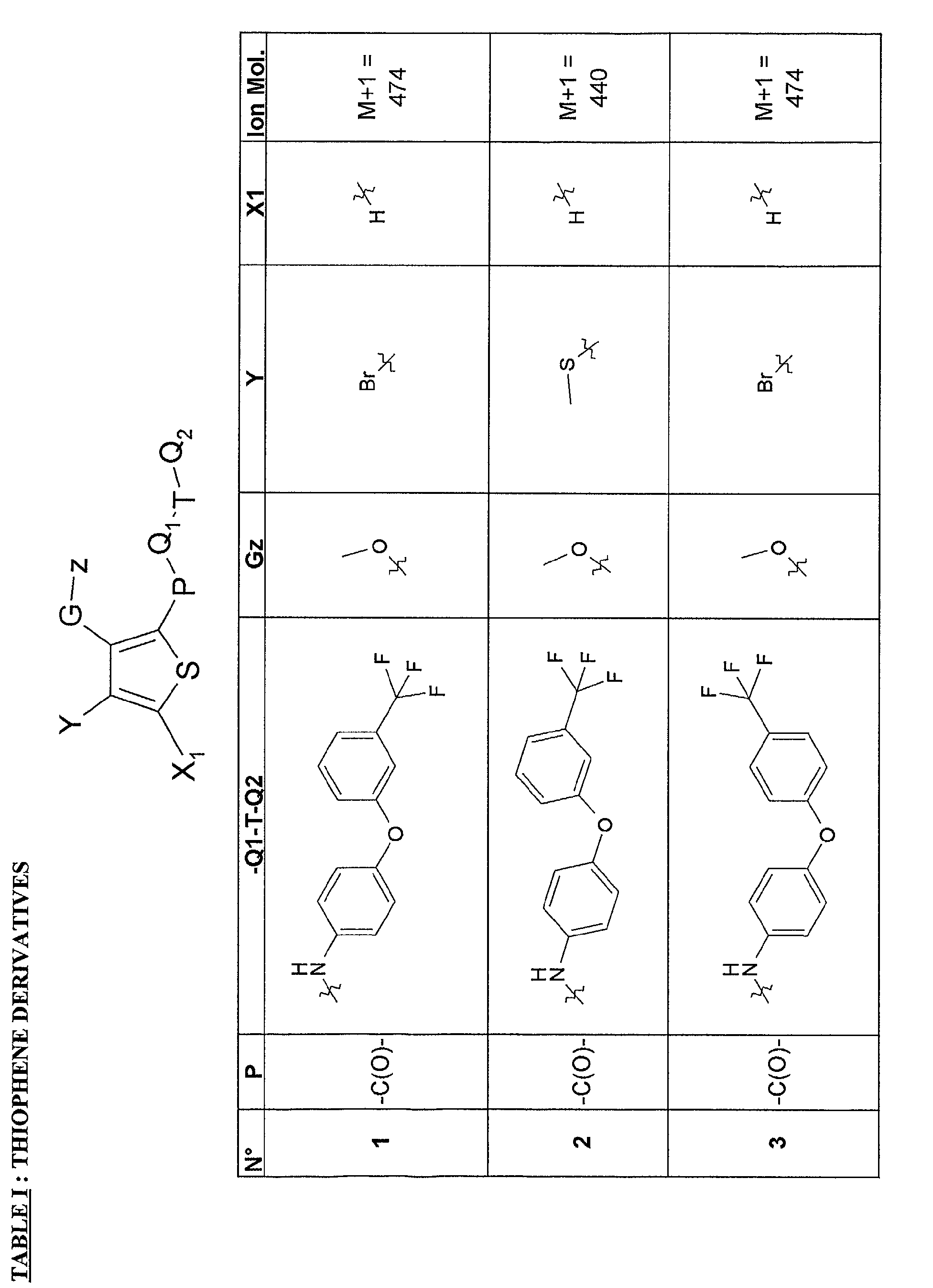

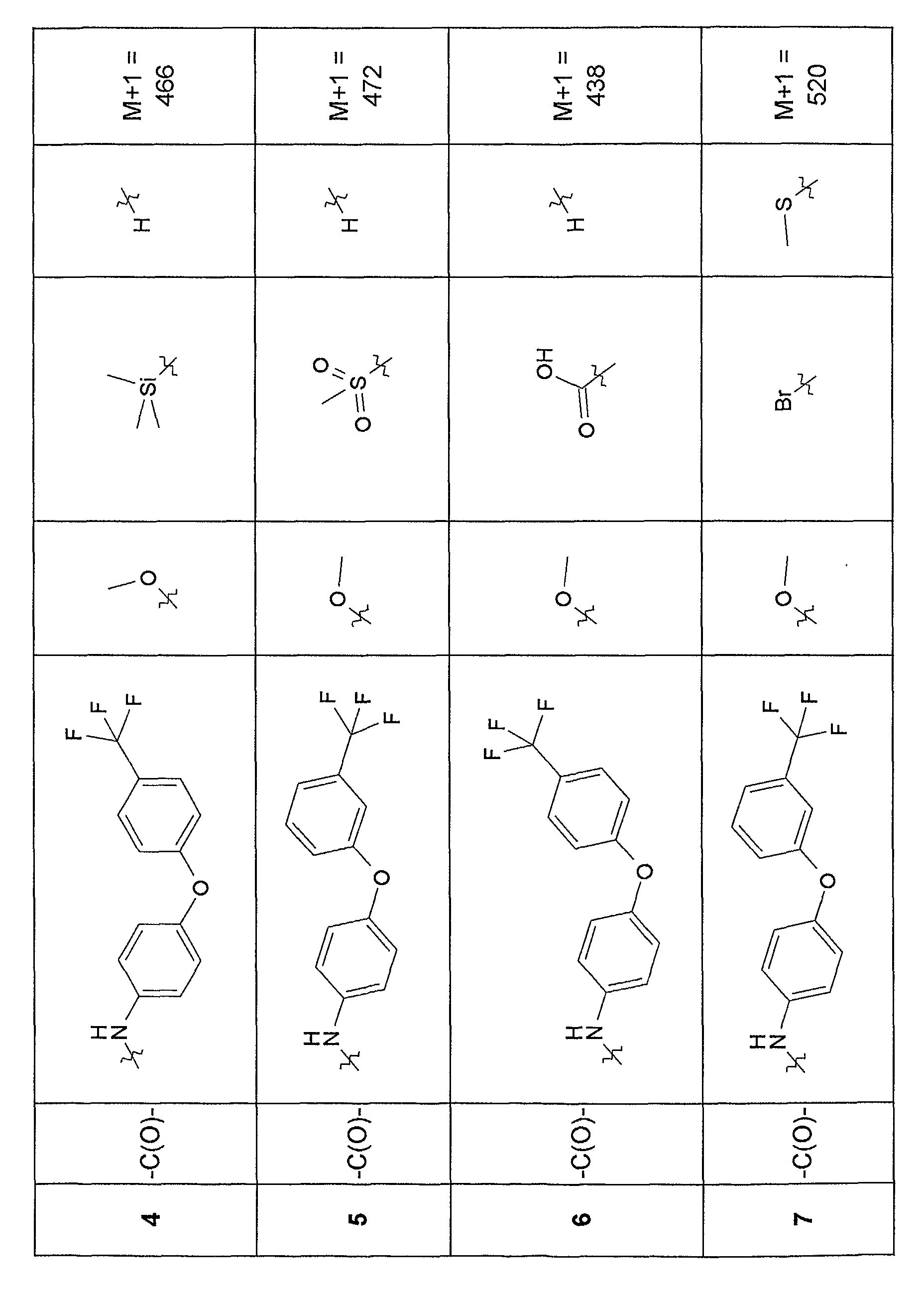

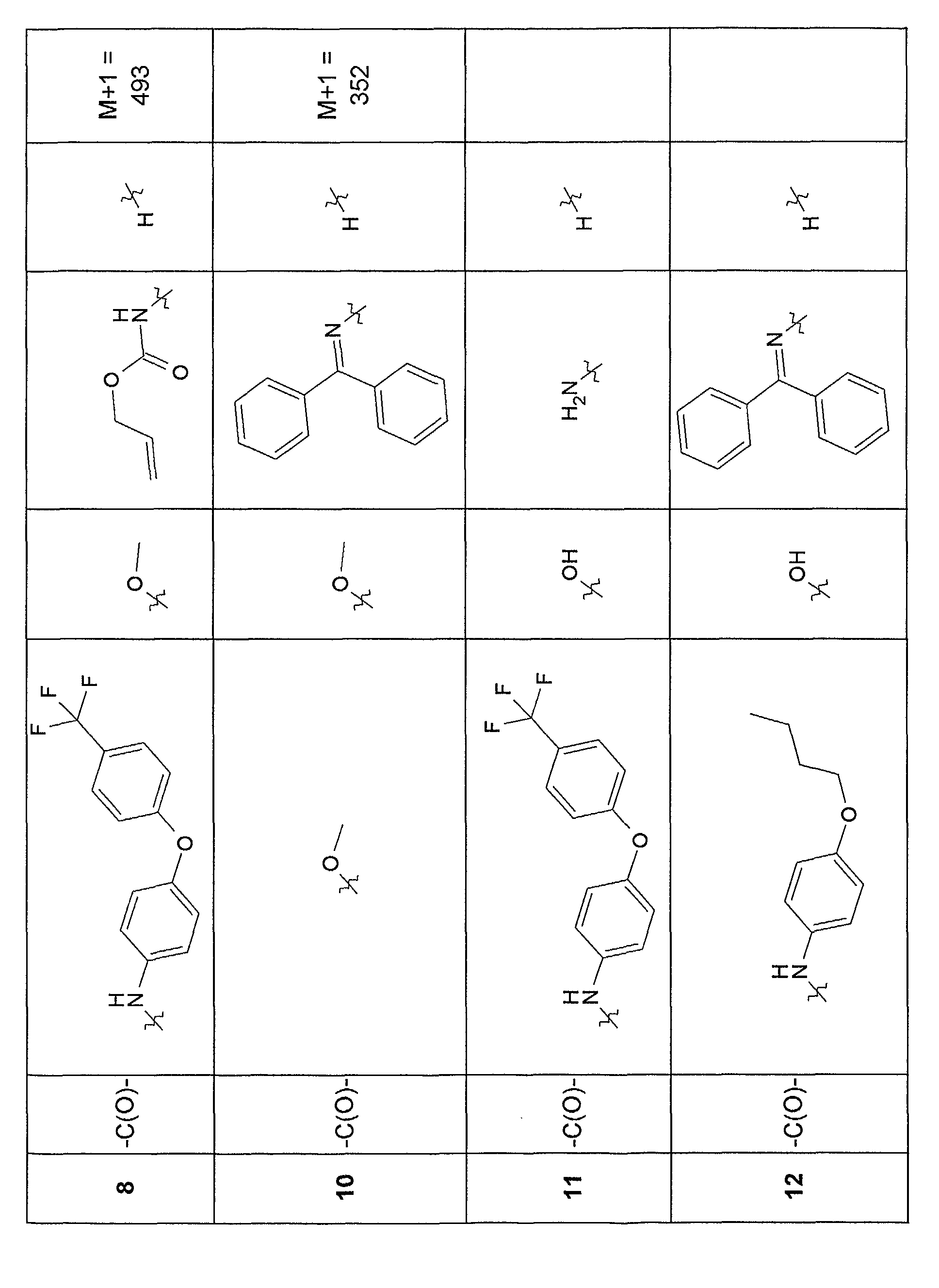

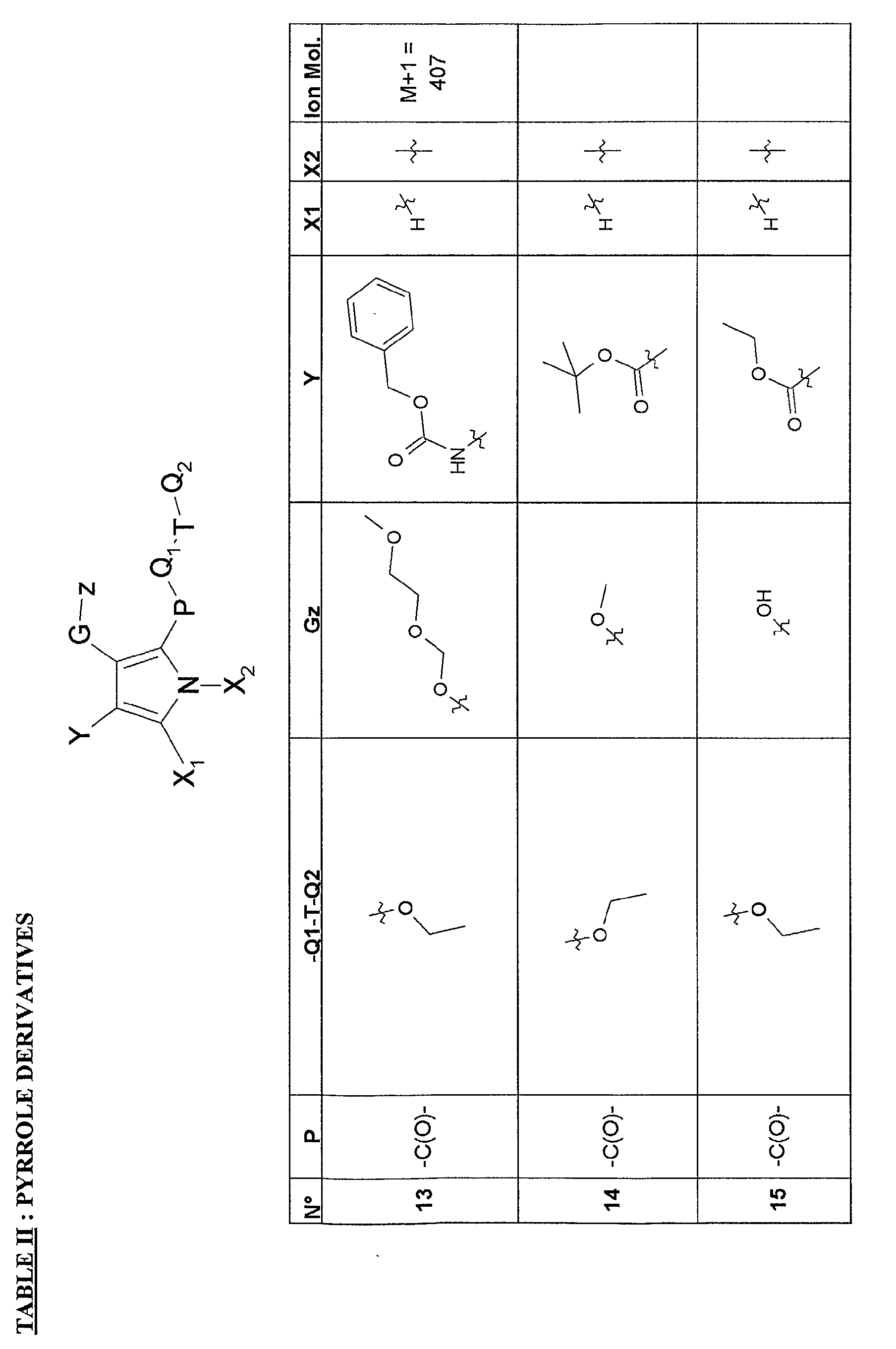

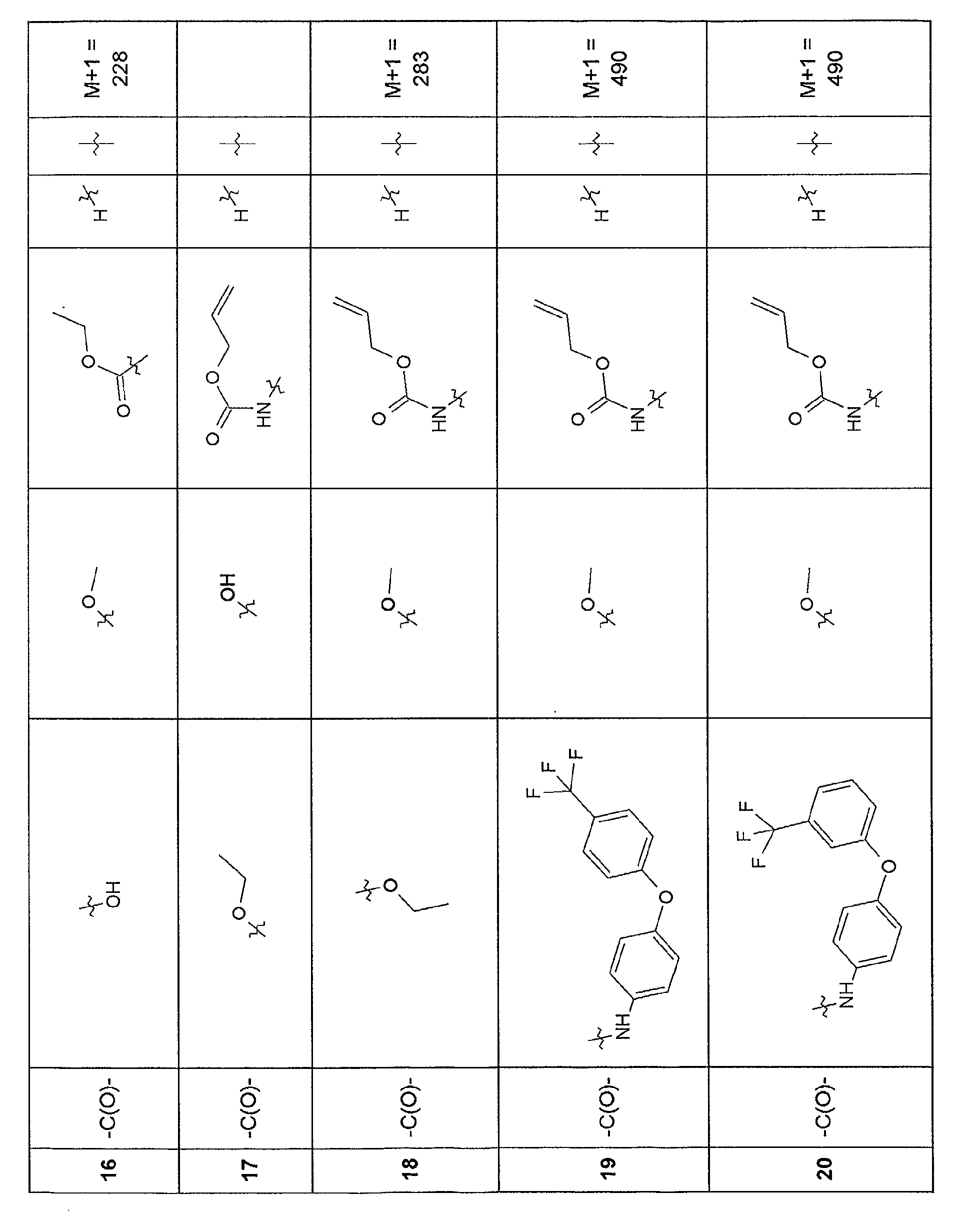

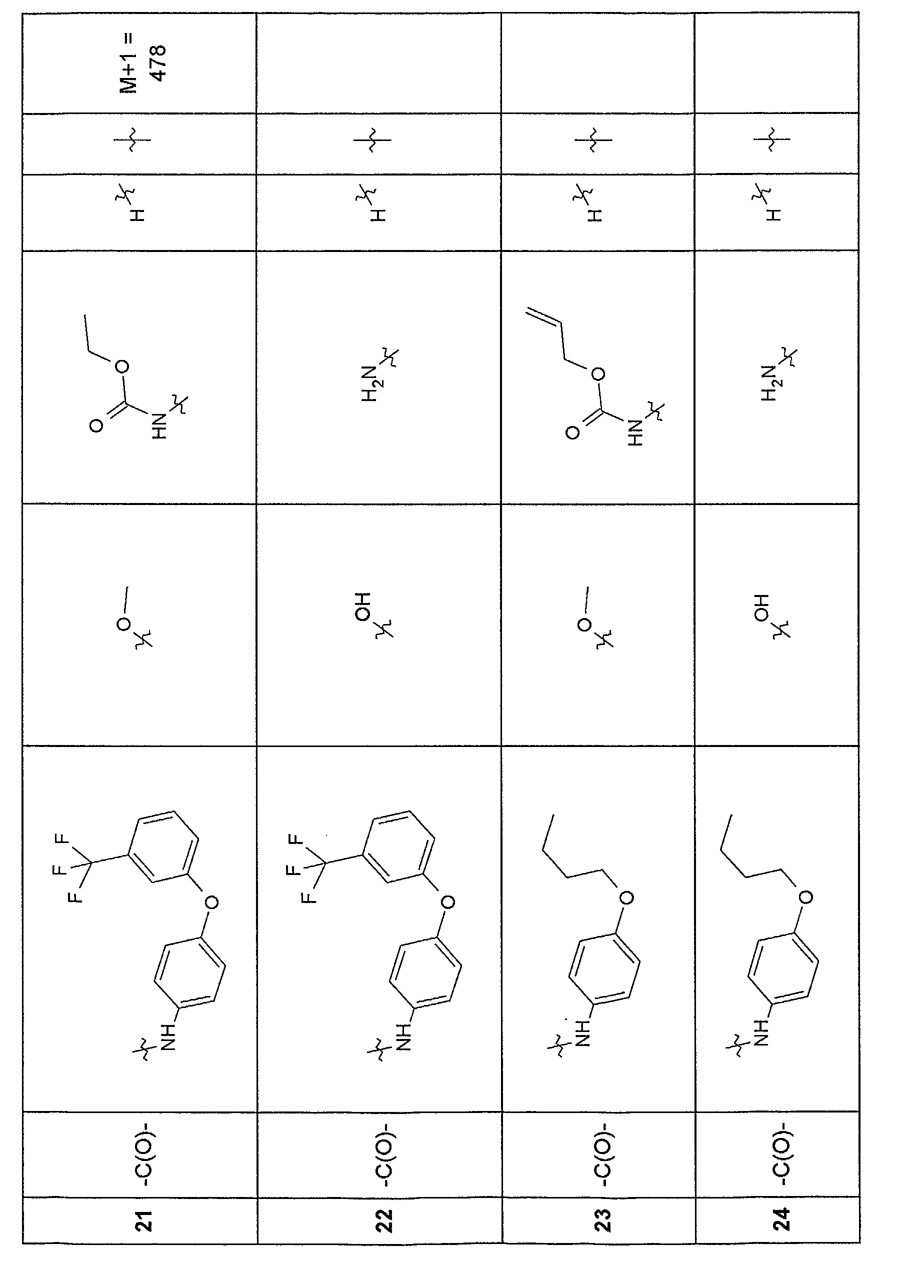

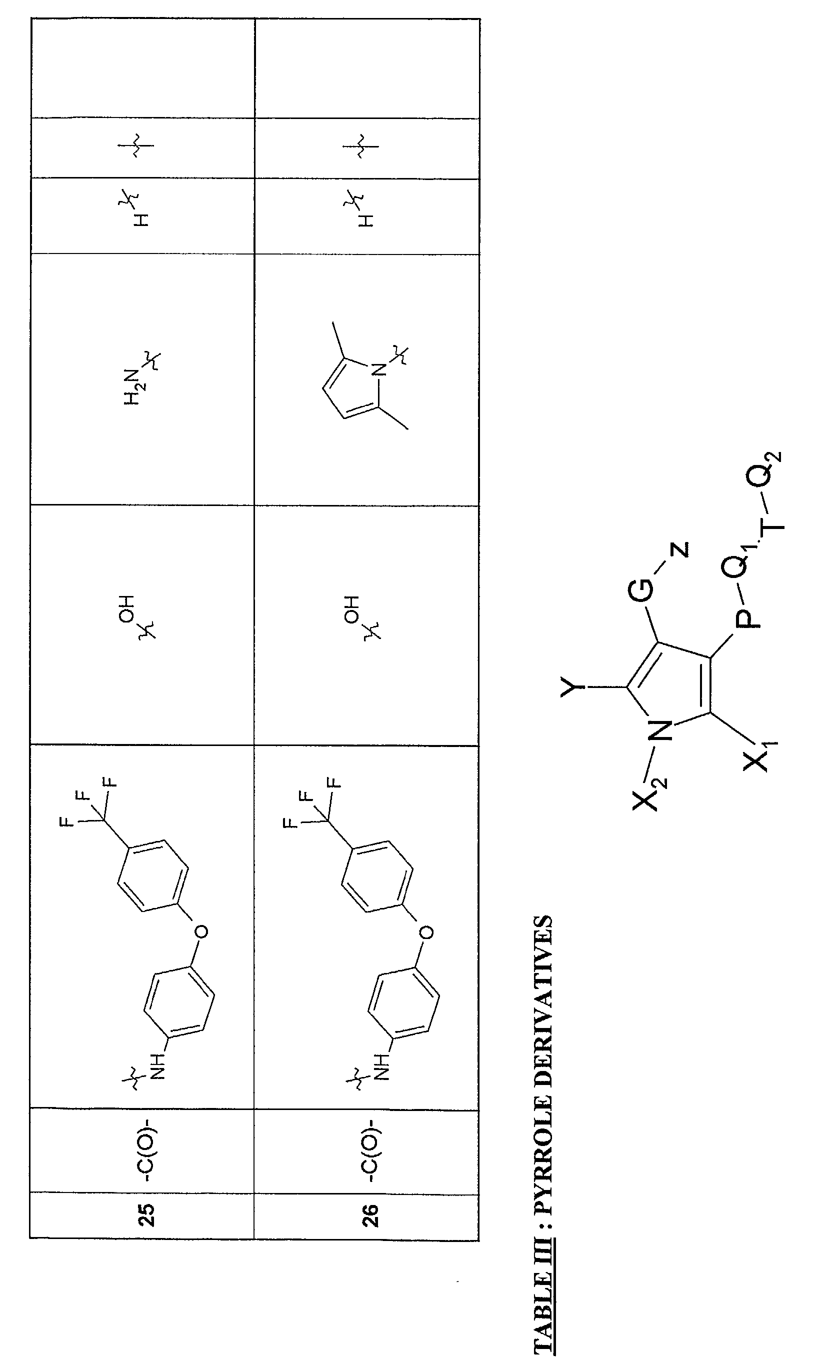

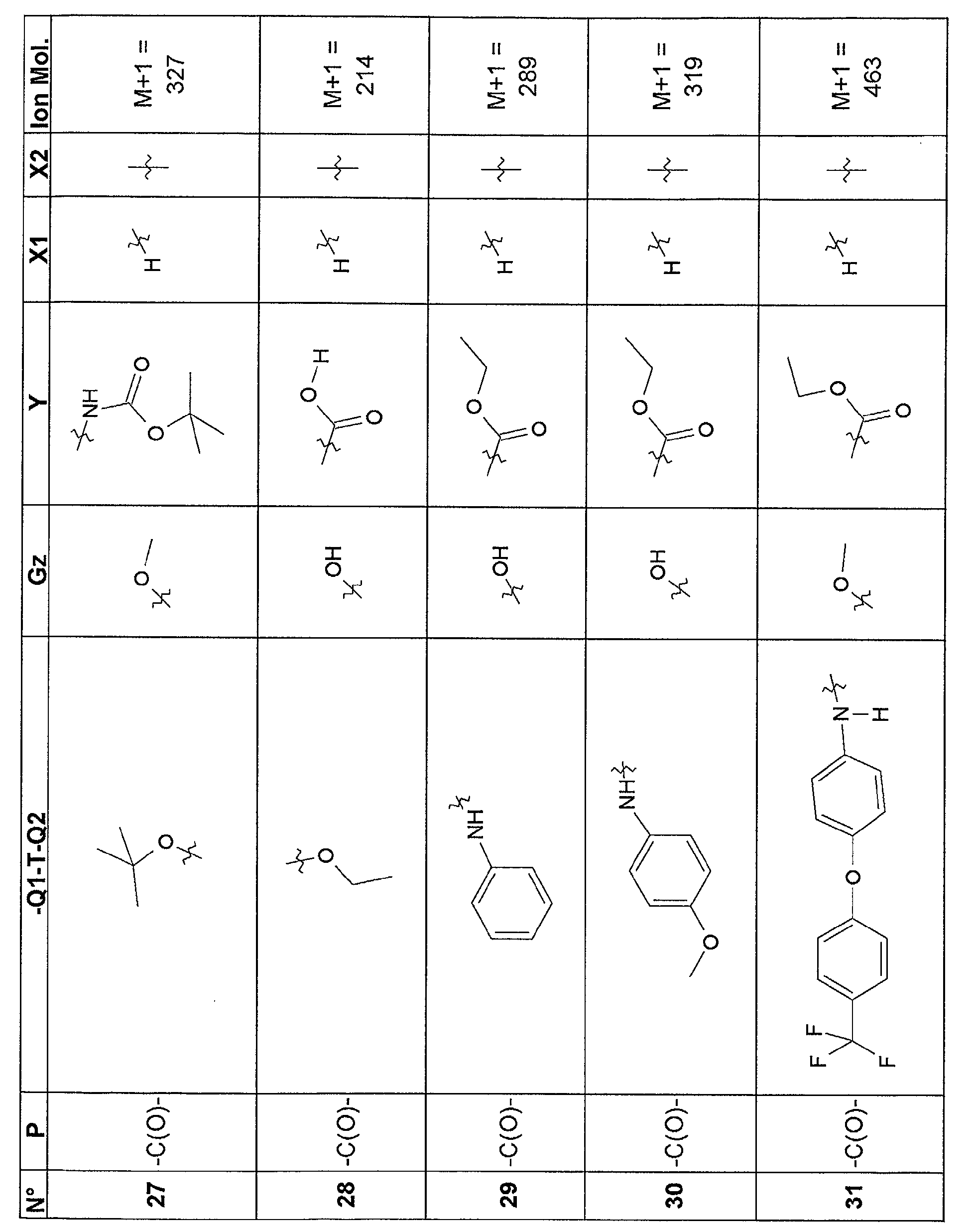

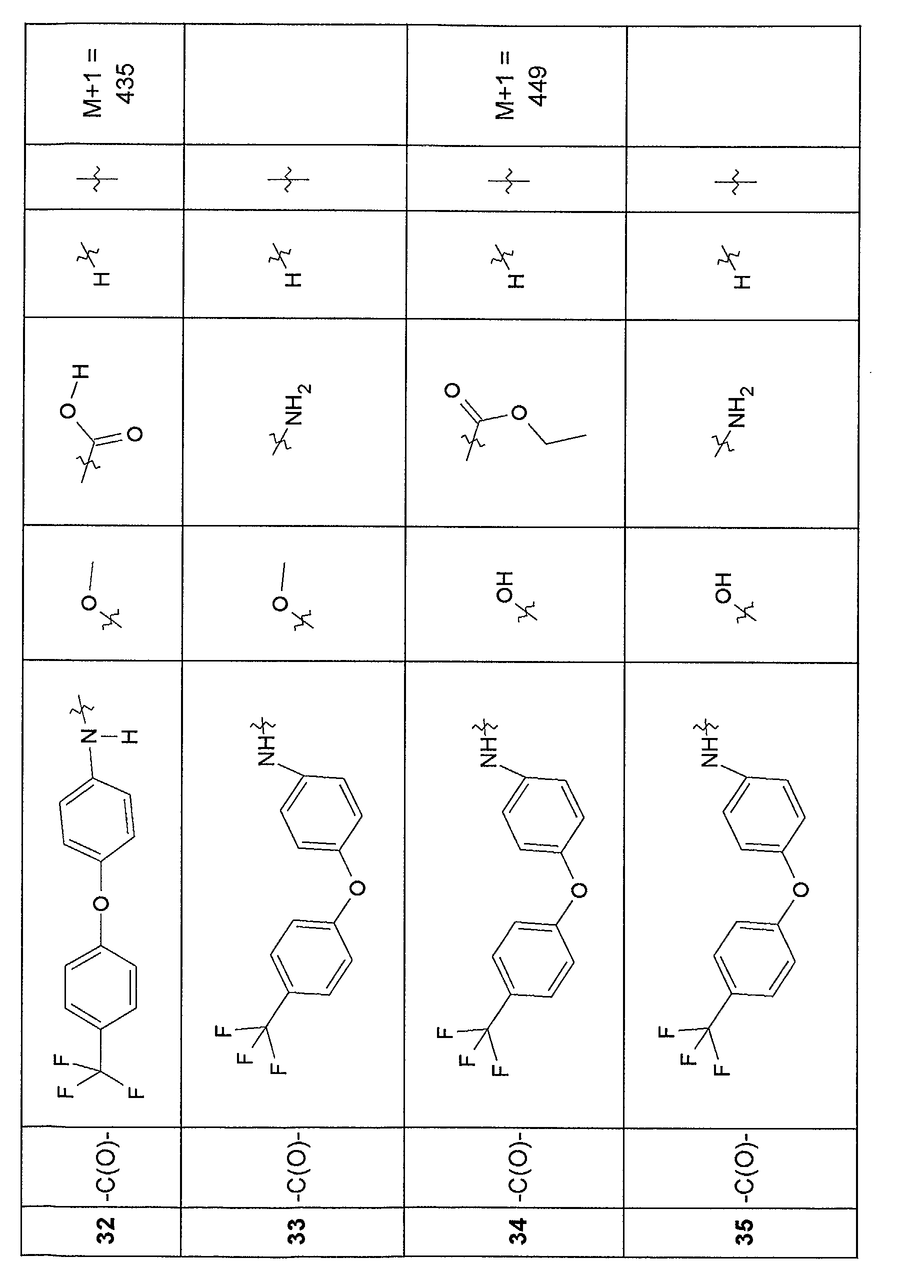

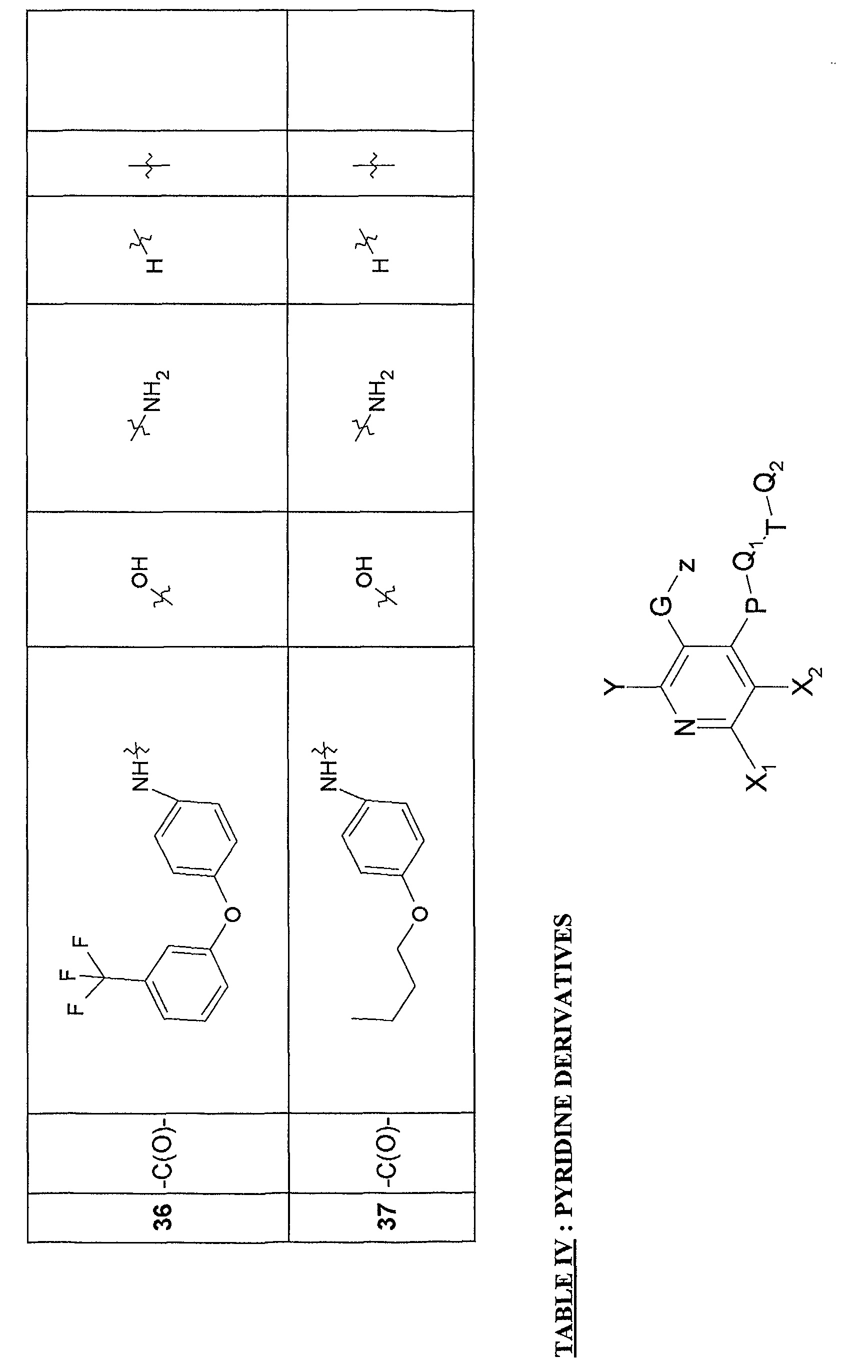

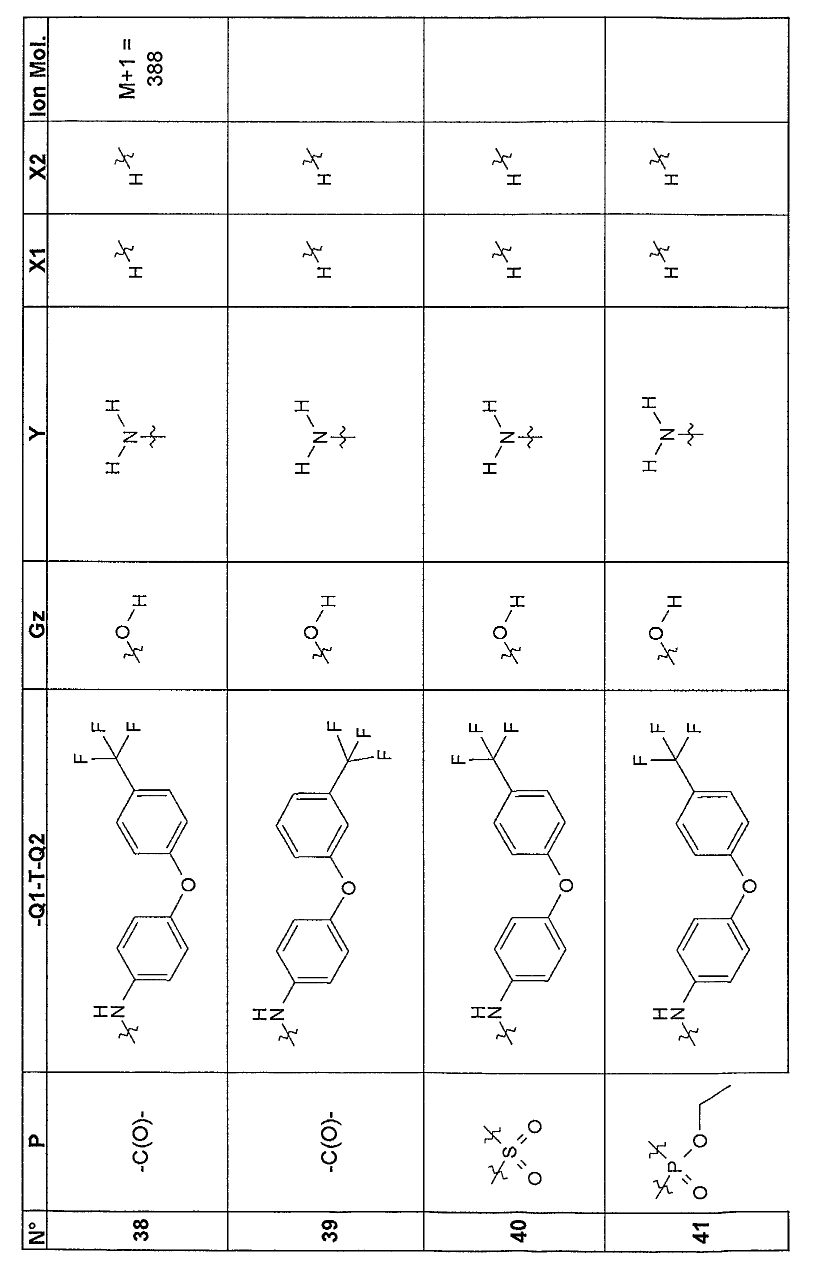

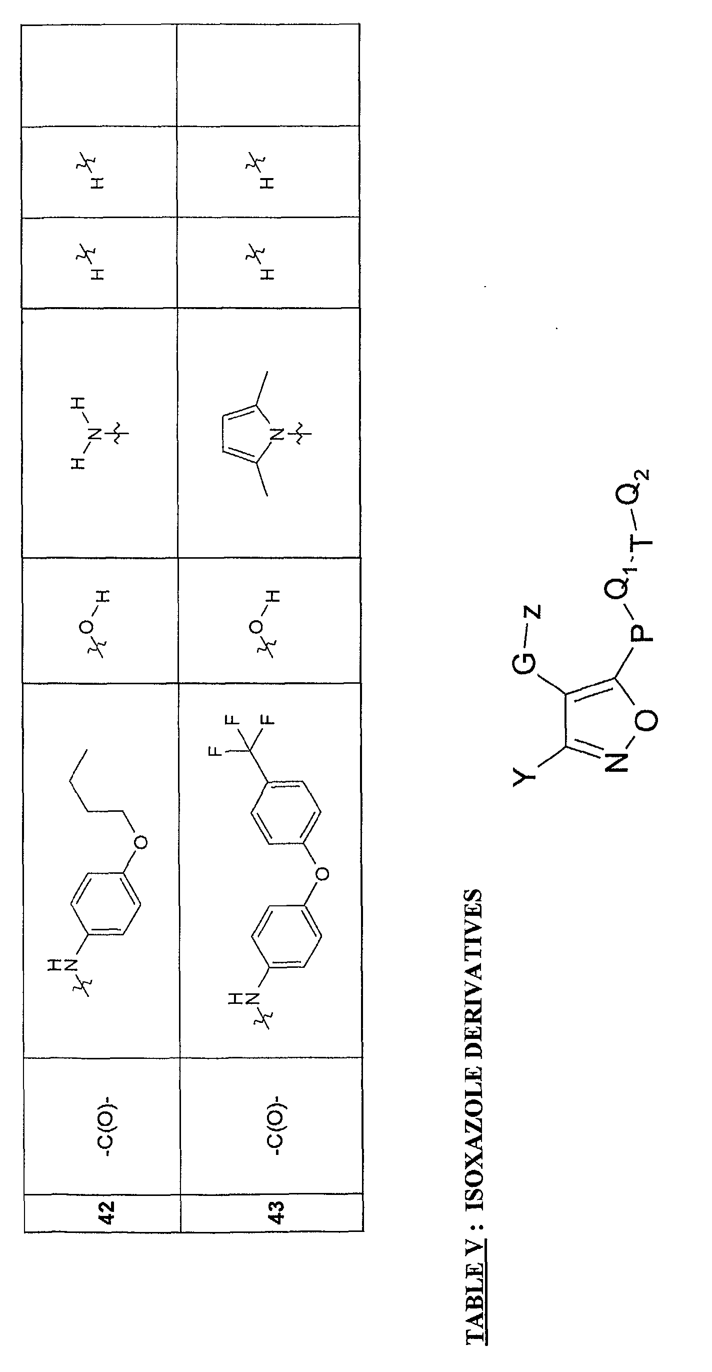

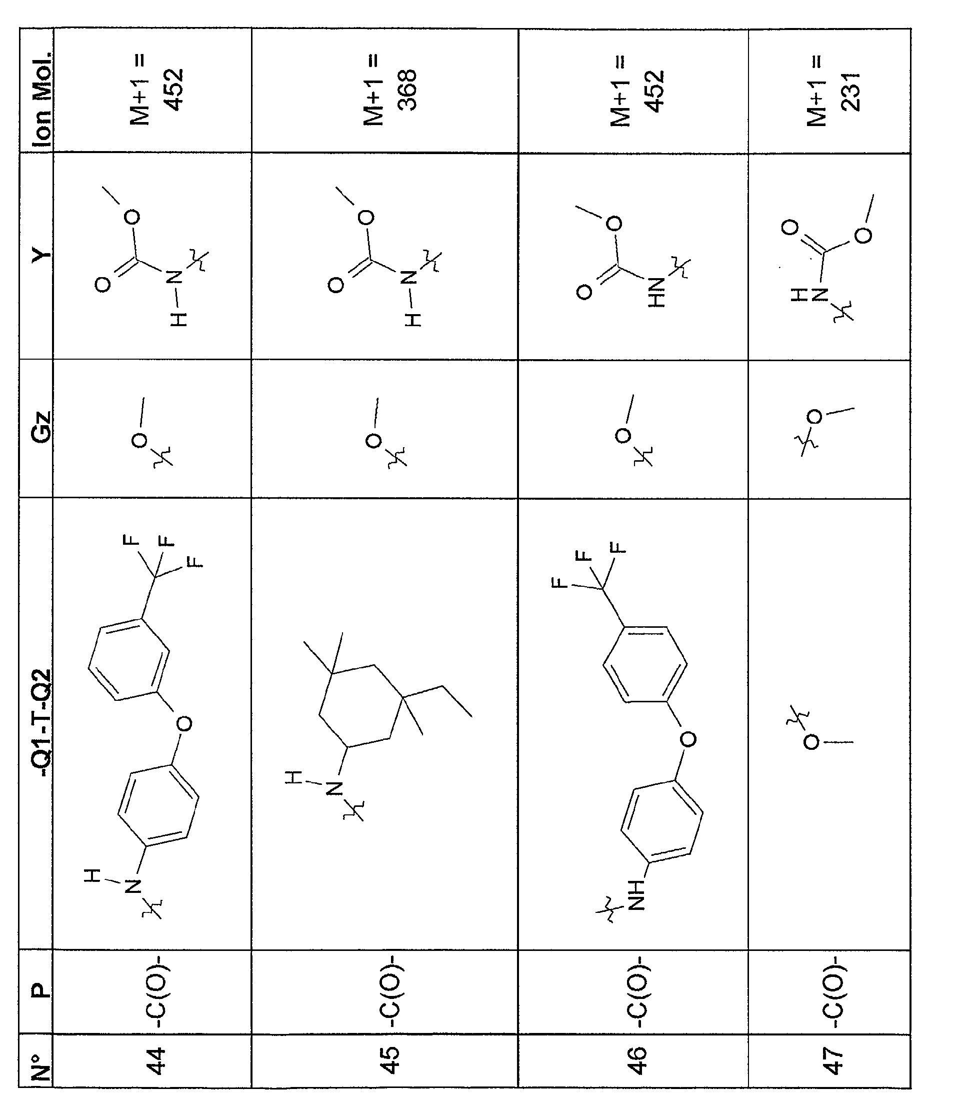

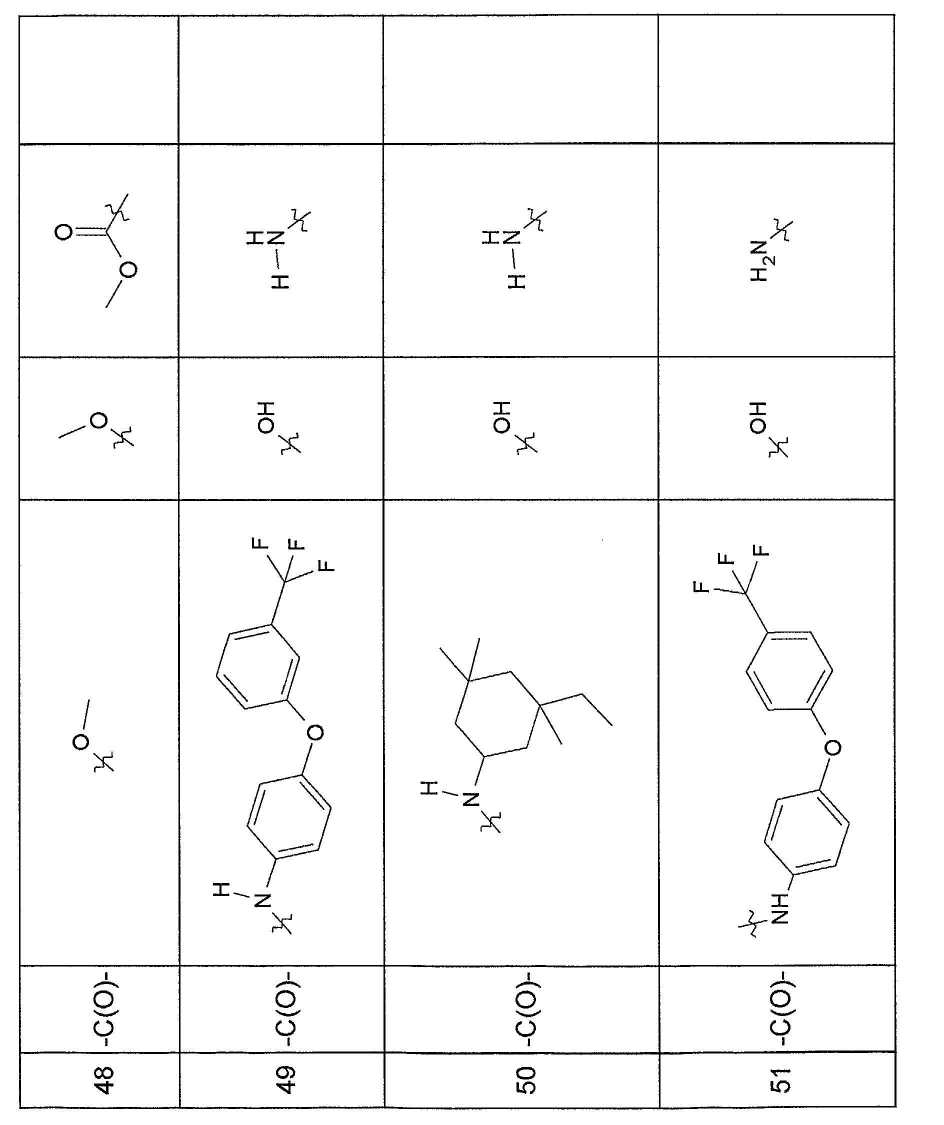

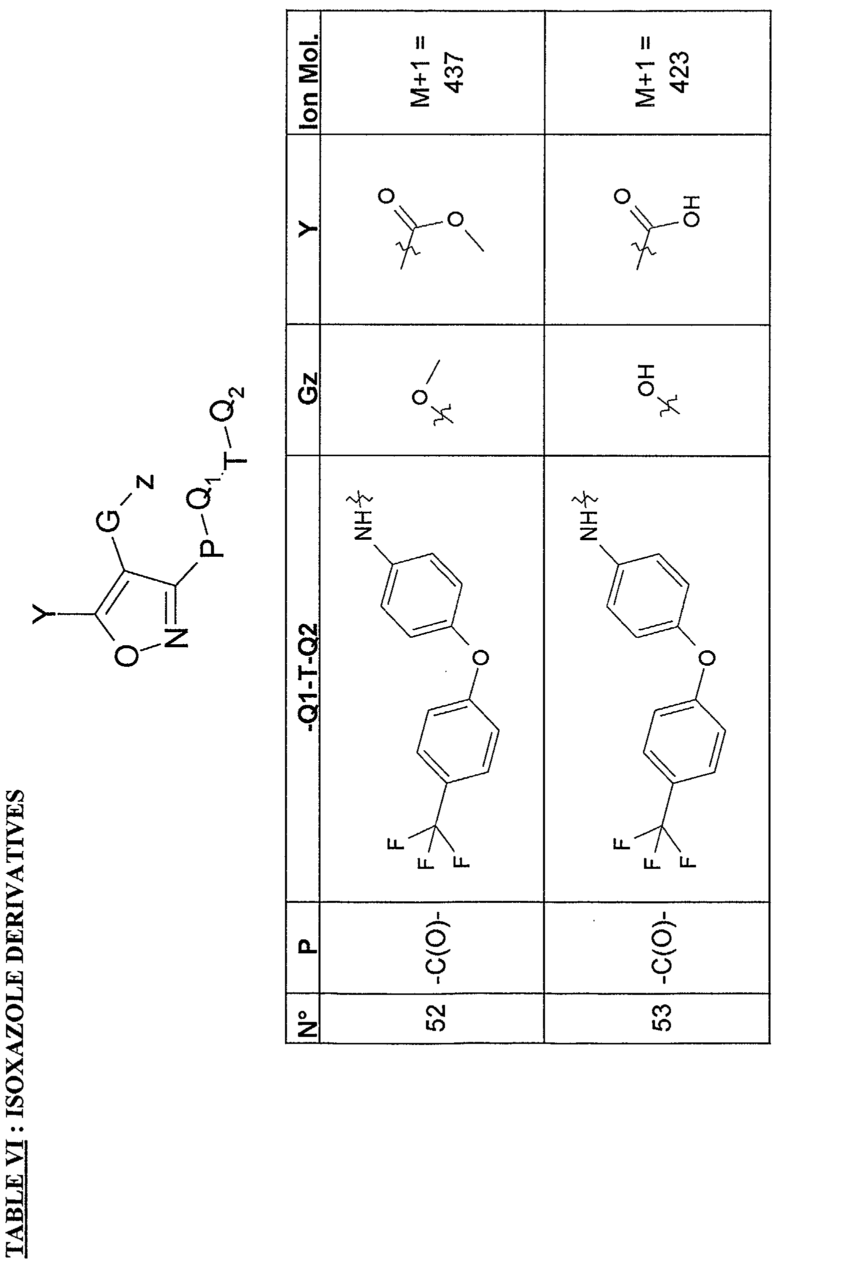

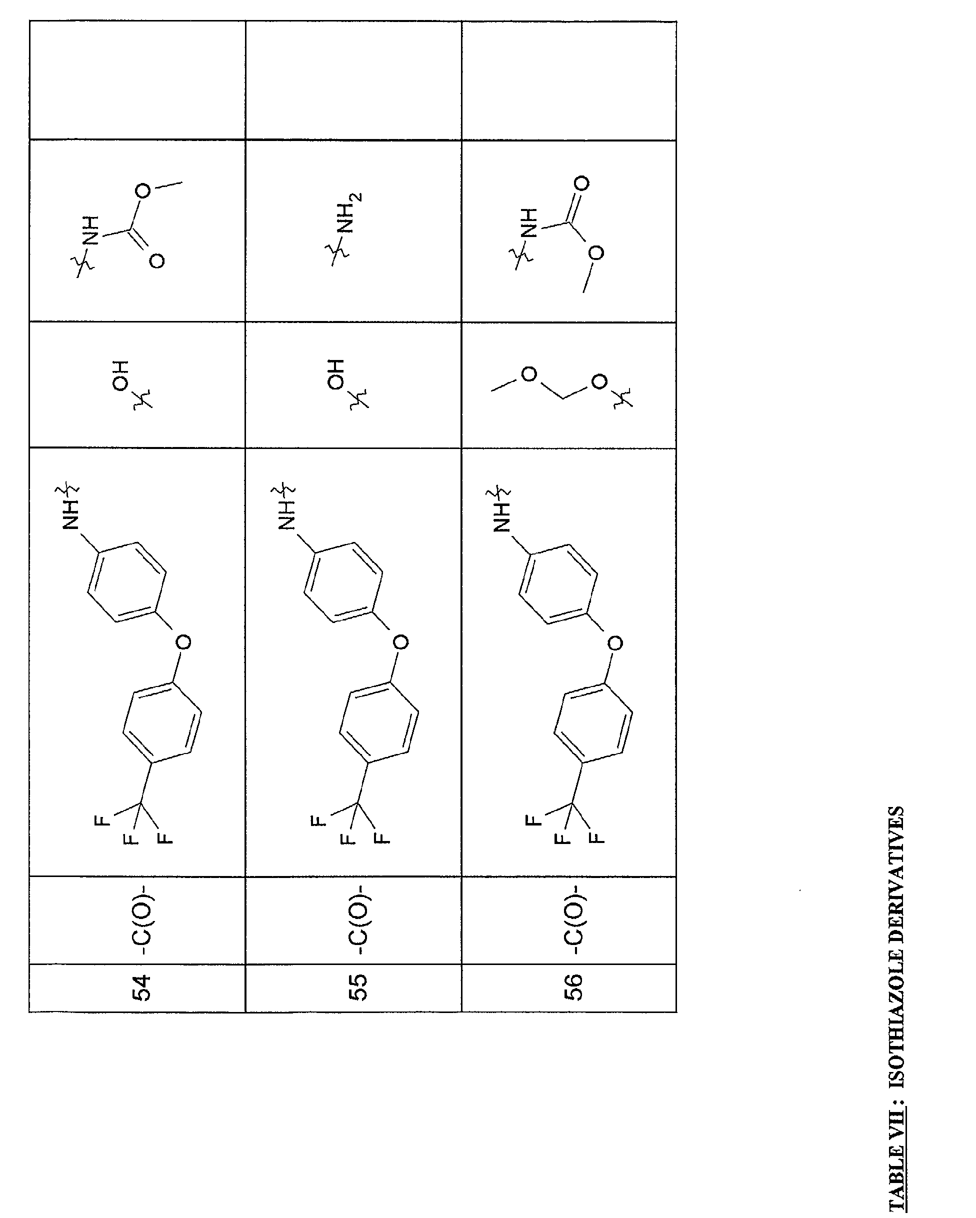

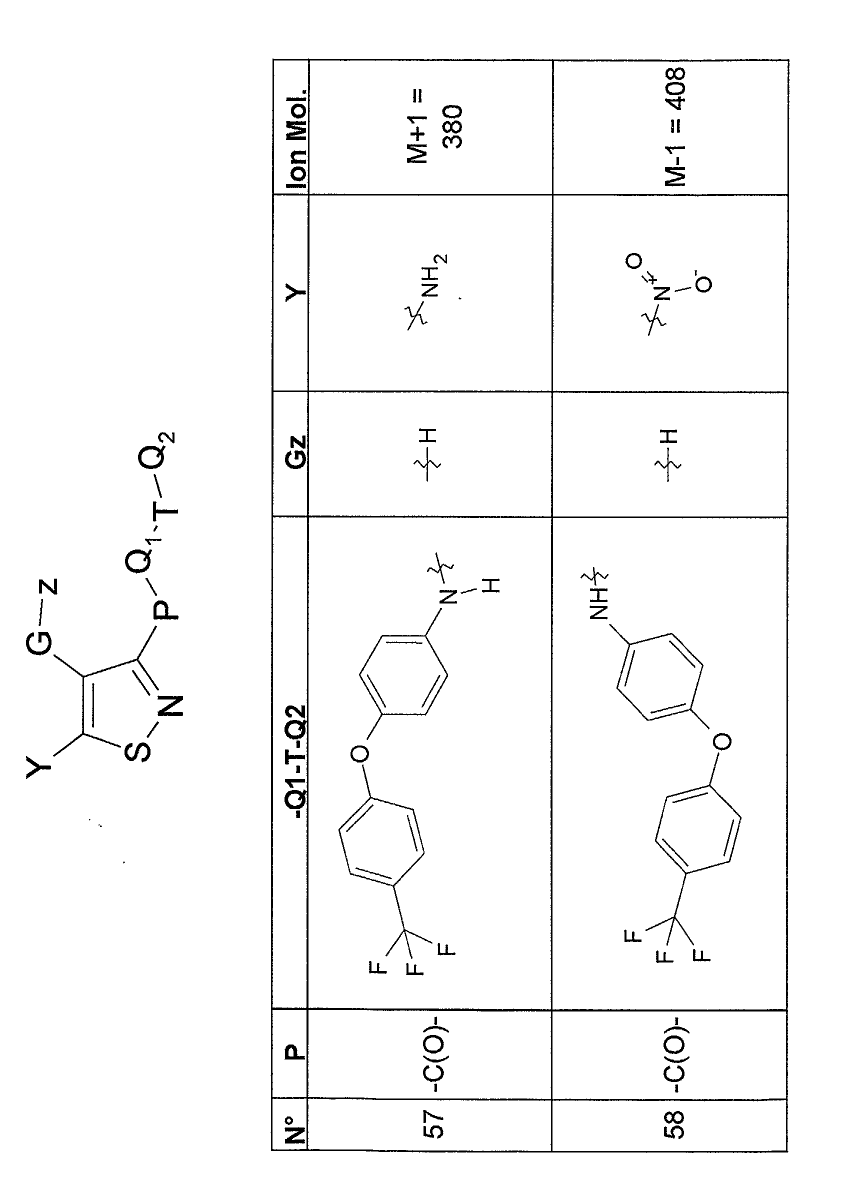

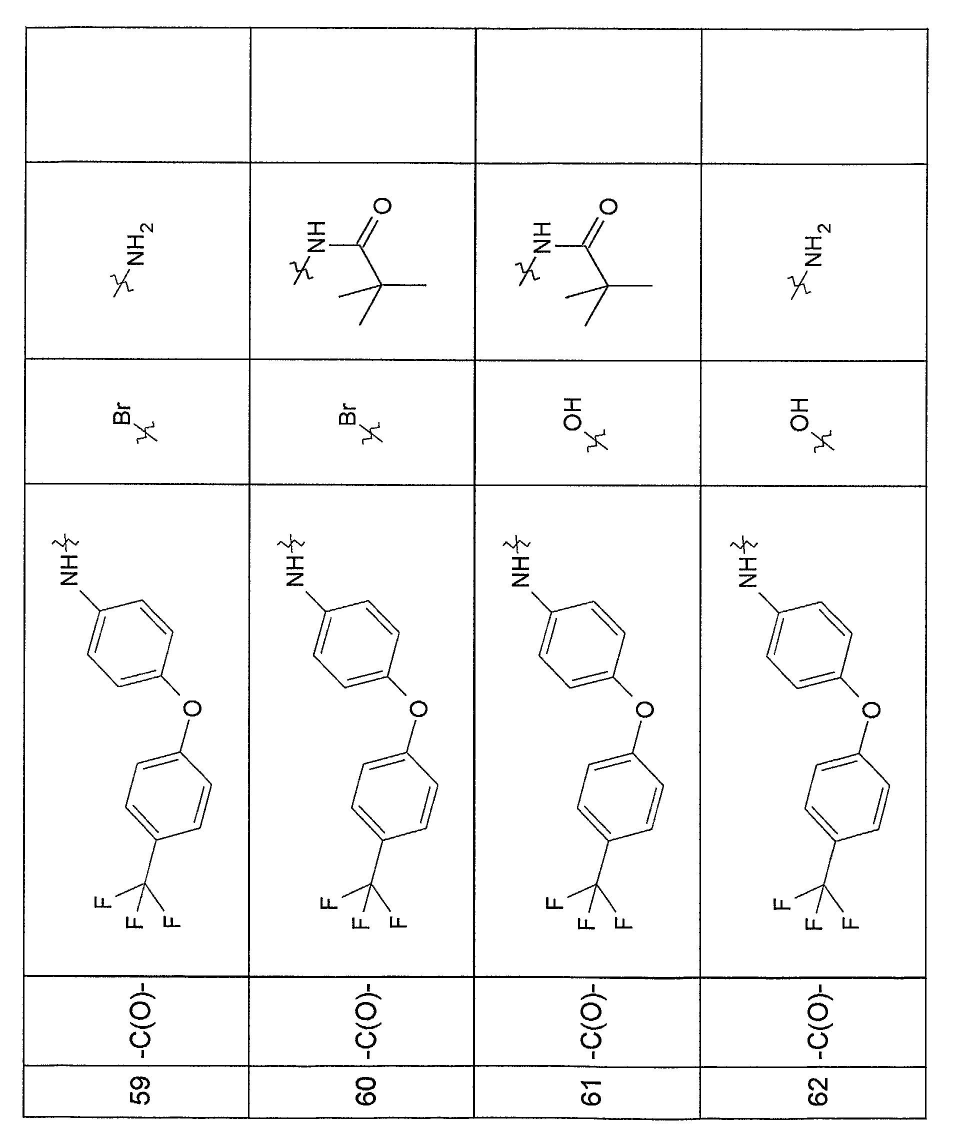

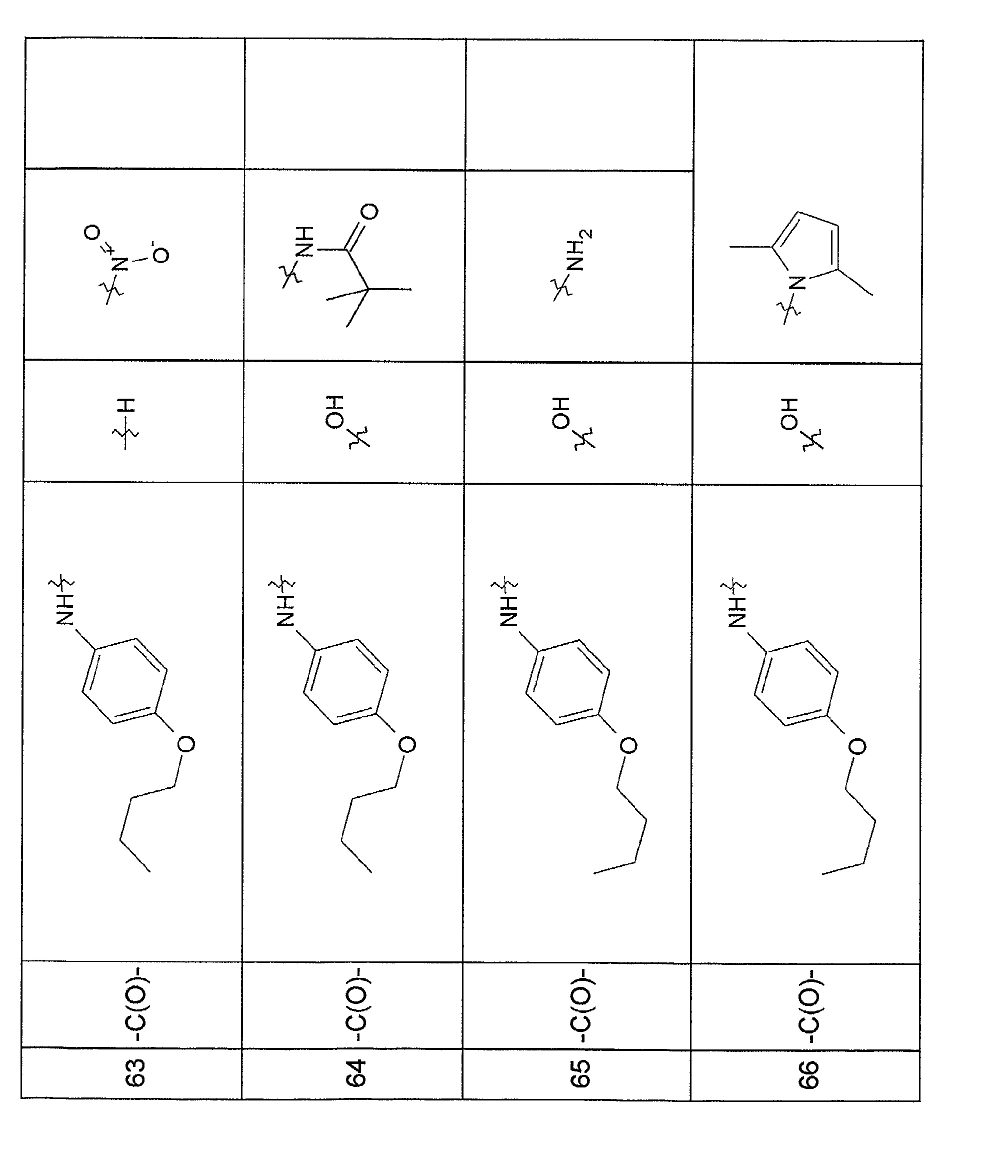

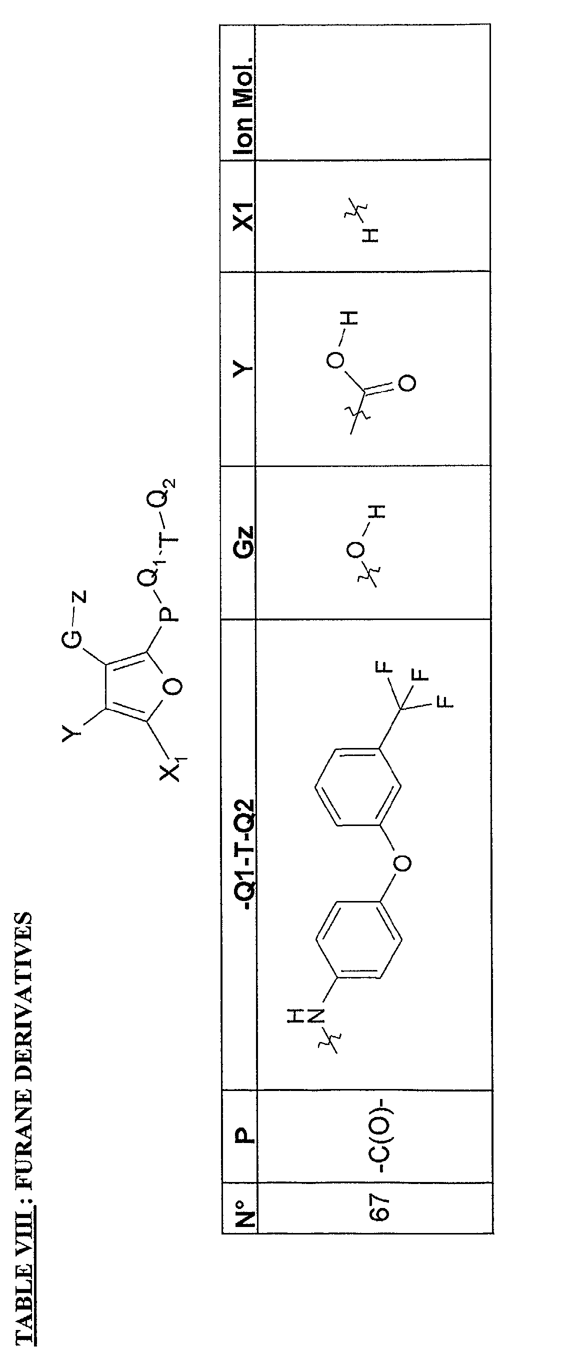

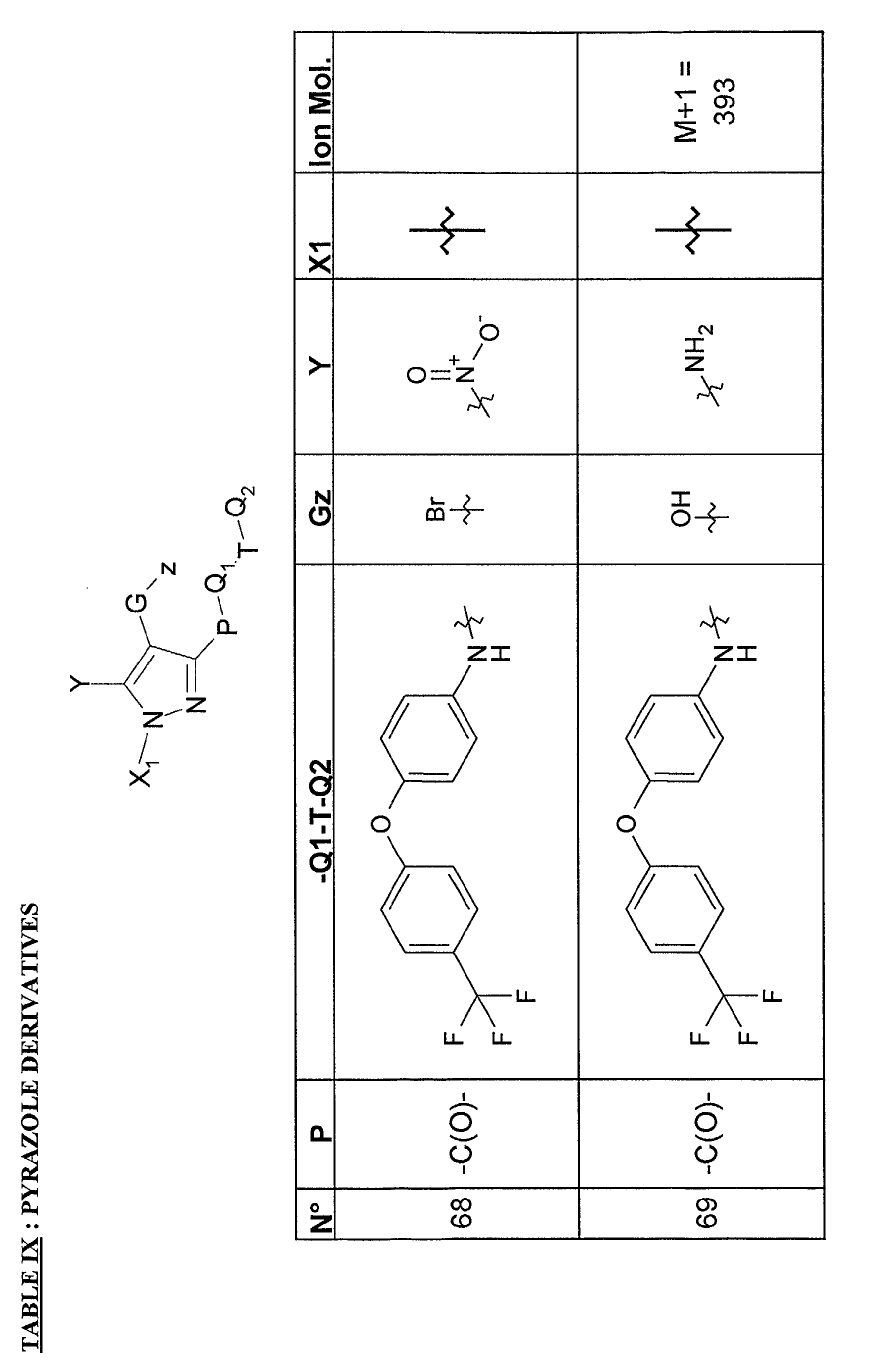

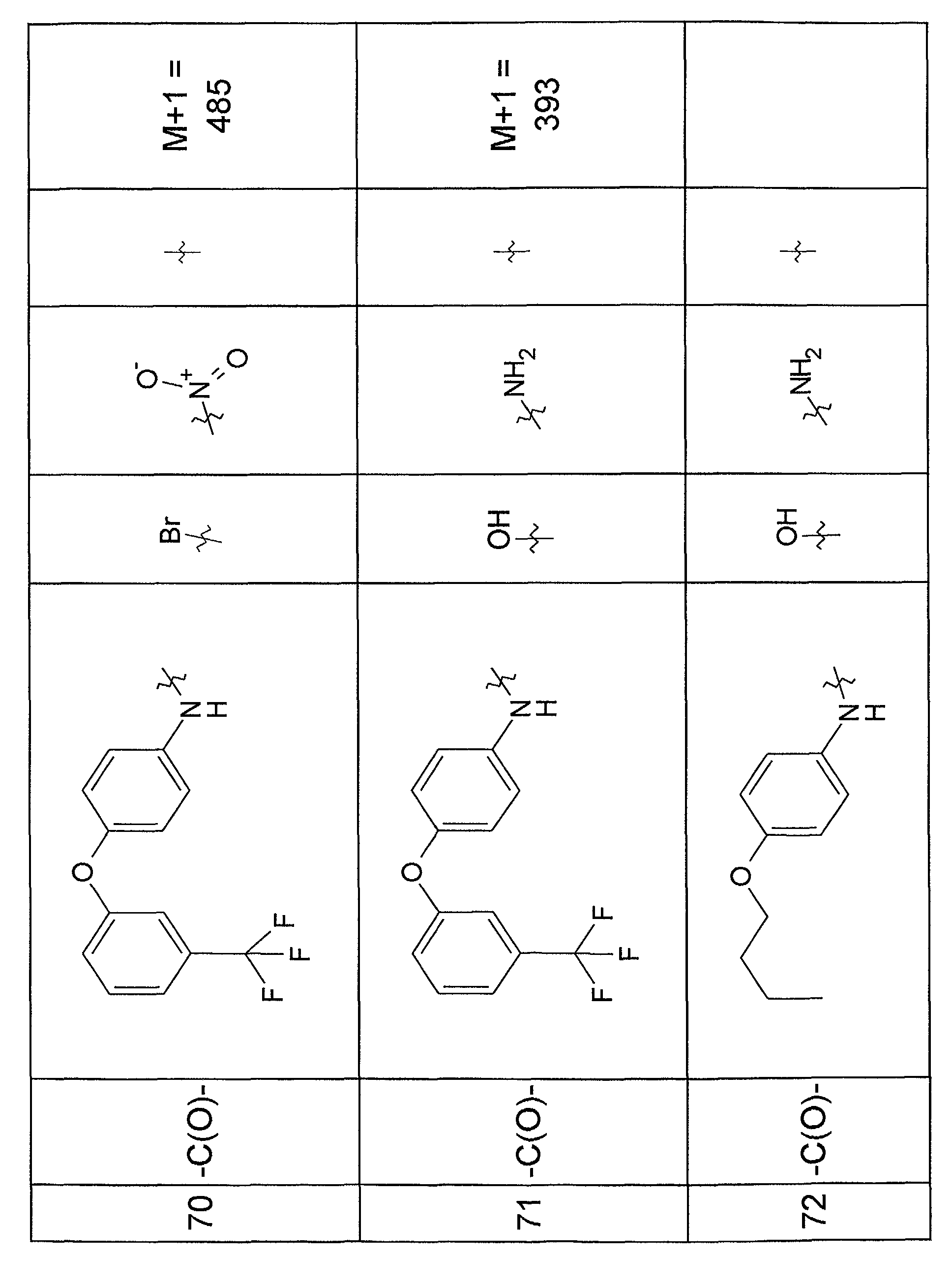

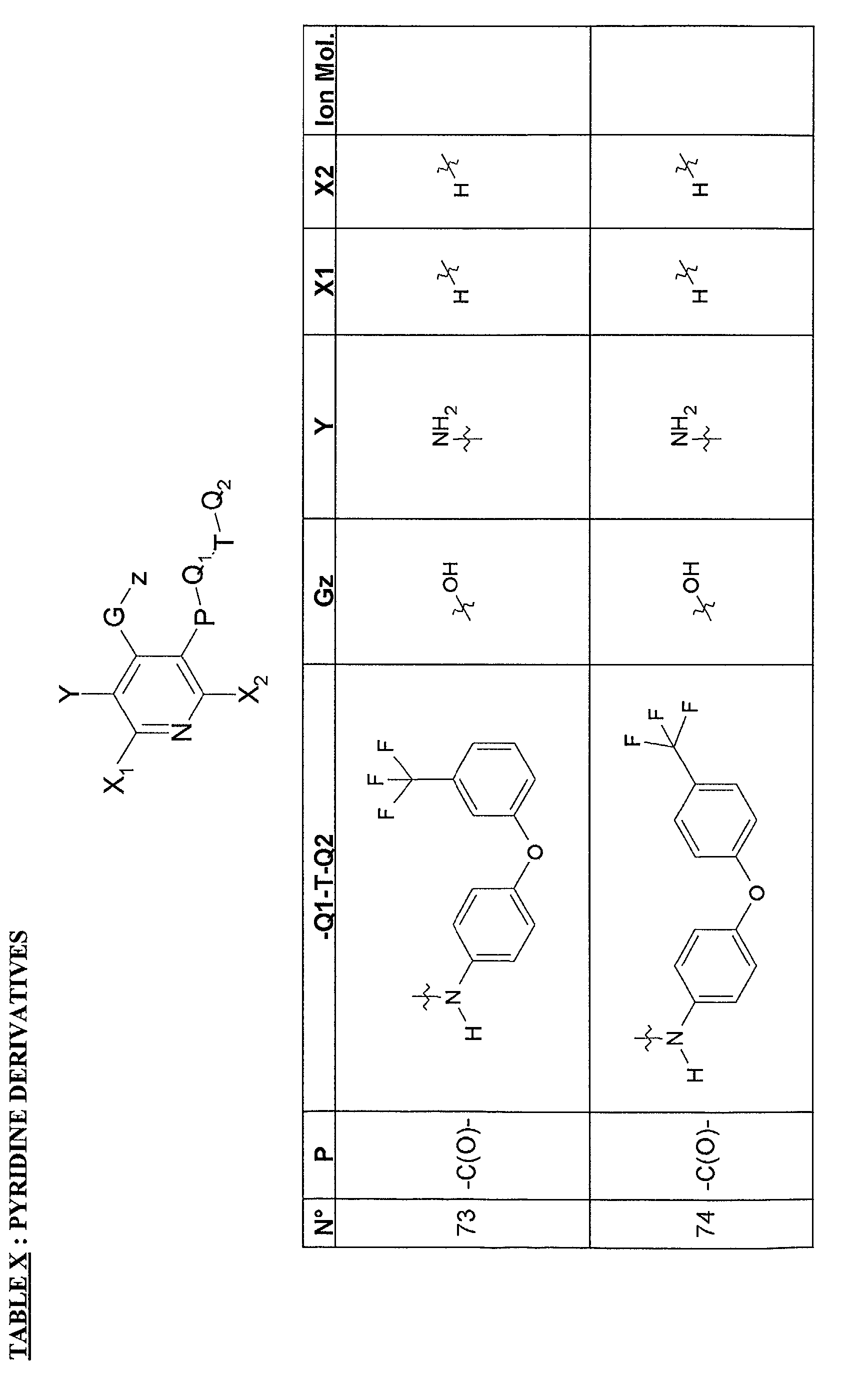

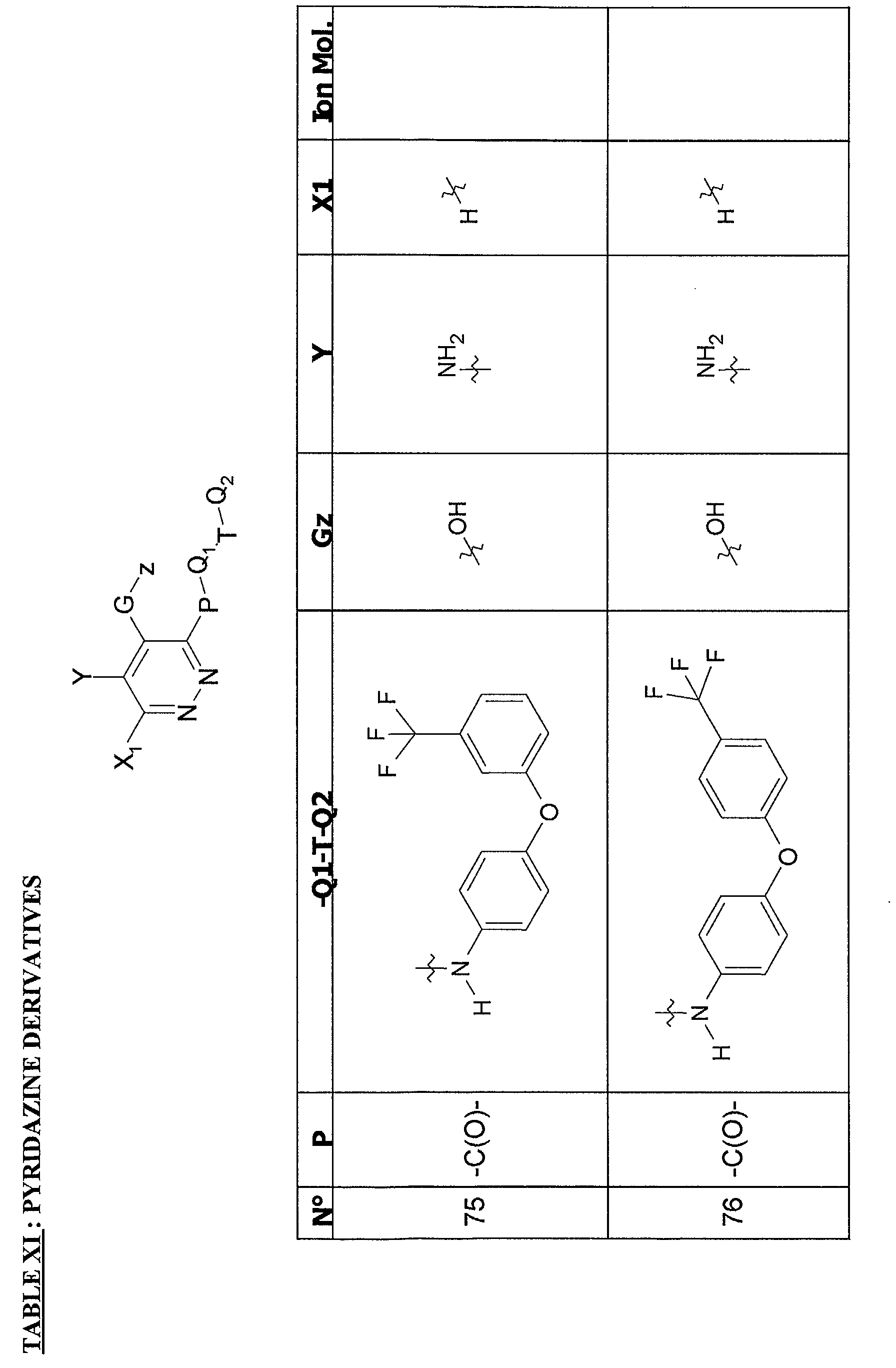

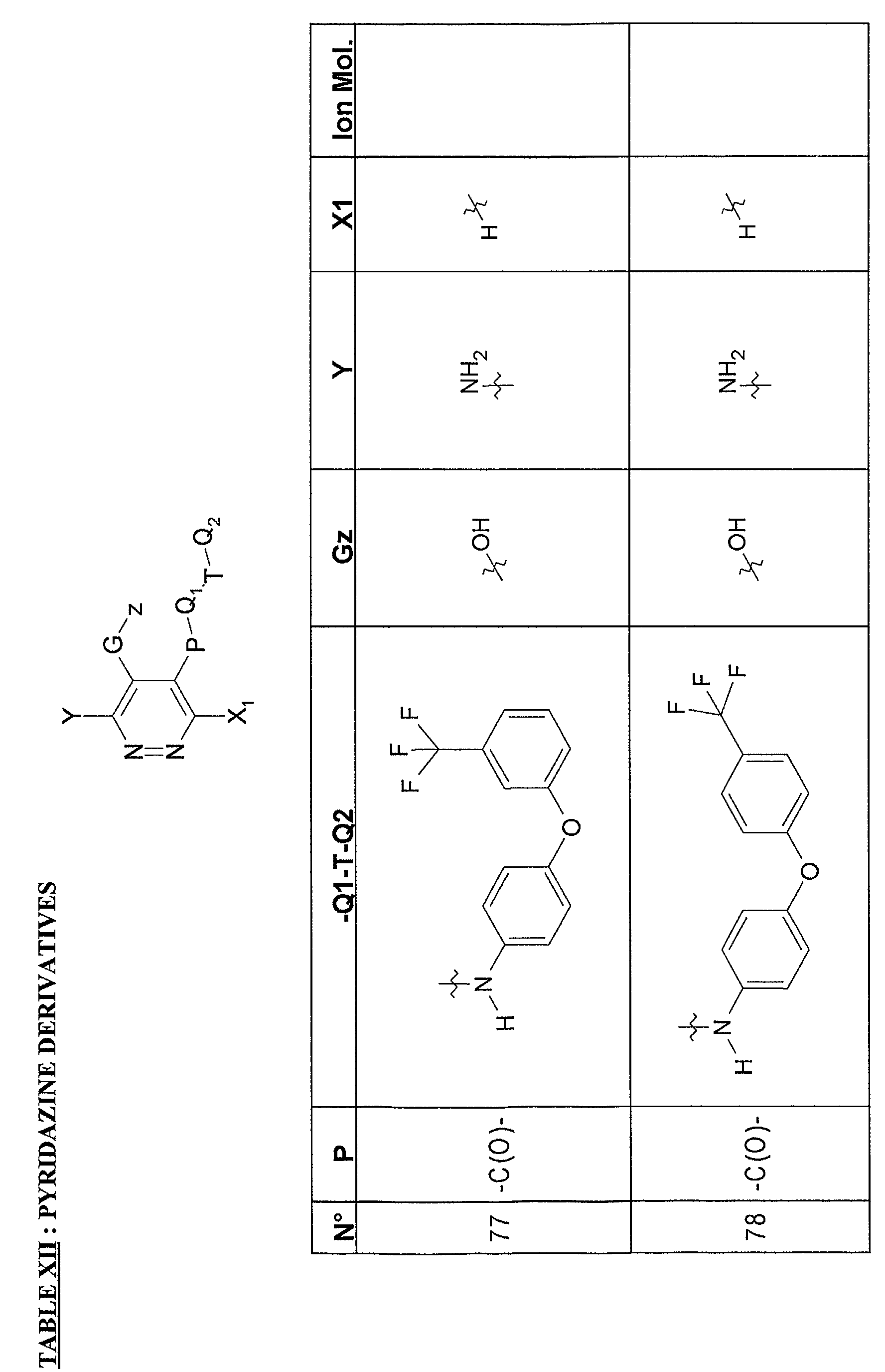

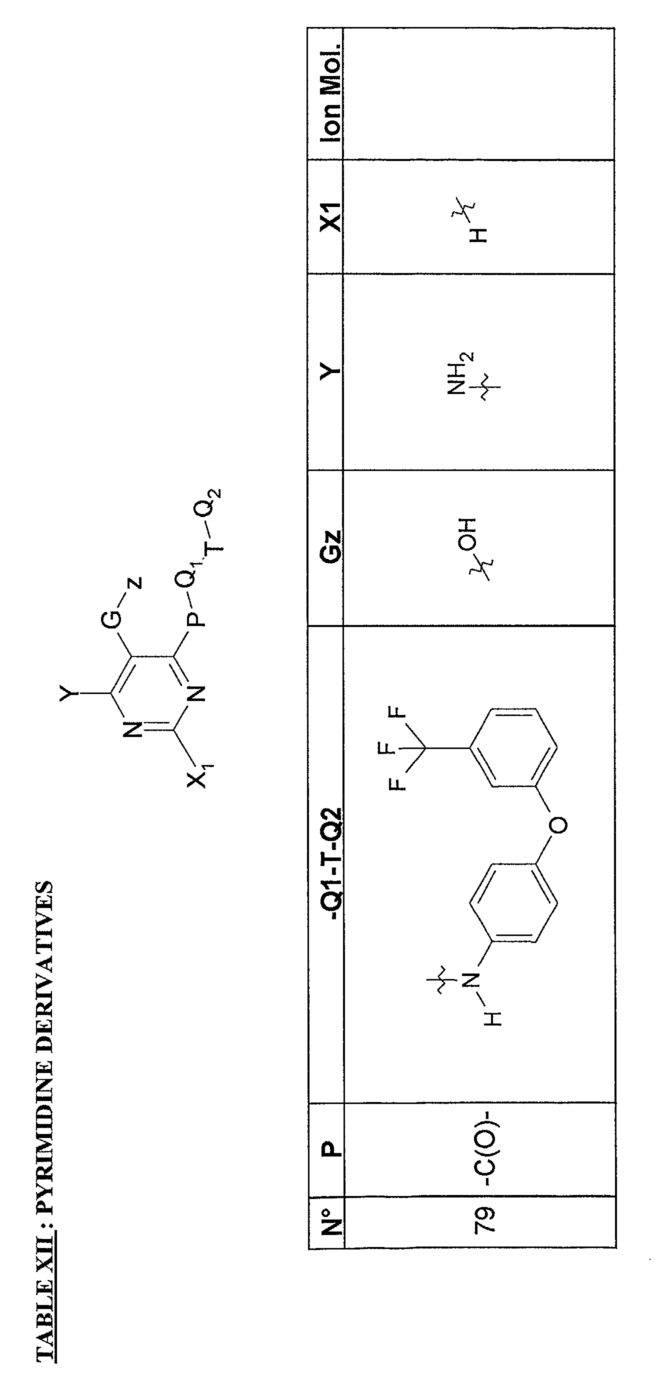

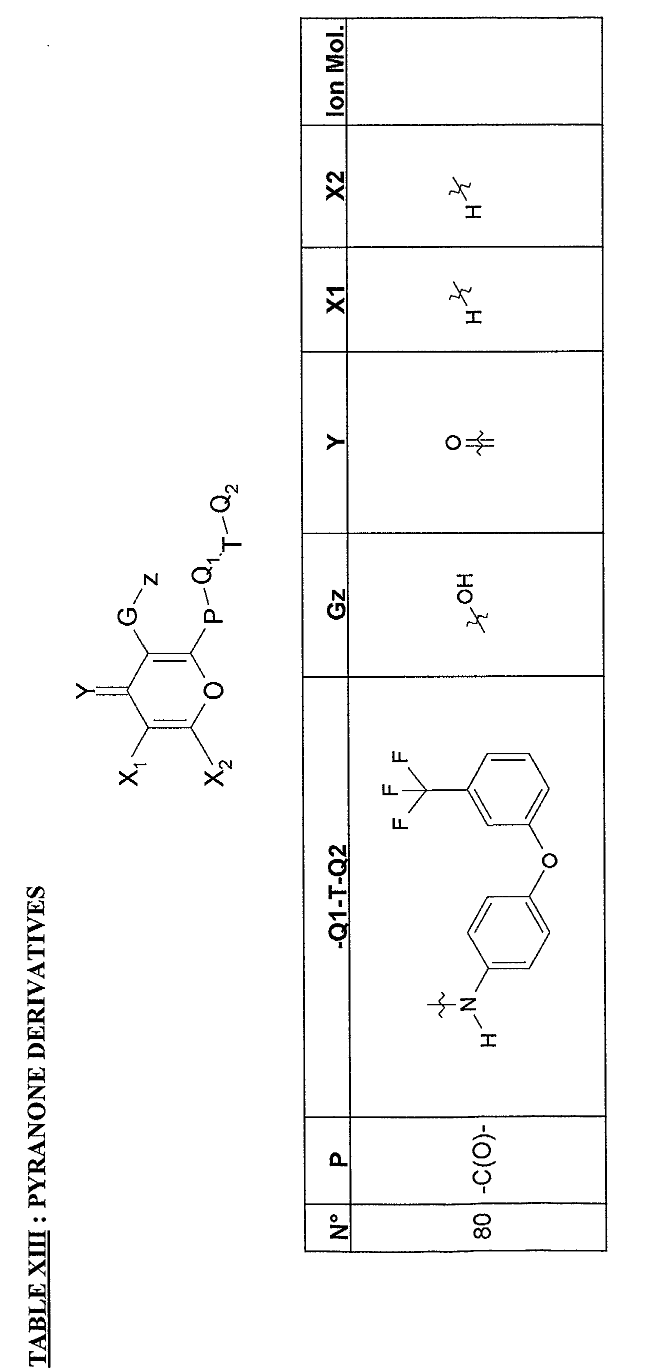

### Examples of biological activity of the compounds of the invention

### Example A: in vivo test on Alternaria brassicae (Leaf spot of crucifers):

An aqueous suspension, at a concentration of 1.5%, of the active material tested is obtained by grinding it finely in a formulation of concentrated suspension type. This aqueous suspension is then diluted in water so as to obtain the desired active material concentration. Radish plants (Pernot variety) in starter cups, sown on a 50/50 peat soil-pozzolana substrate and grown at 18-20°C, are treated at the cotyledon stage by spraying with the aqueous suspension described above. Plants, used as controls, are treated with an aqueous solution not containing the active material.
After 24 hours, the plants are contaminated by spraying them with an aqueous suspension of *Alternaria brassicae* spores (40,000 spores per cm³). The spores are collected from a 12-13 day-old culture. The contaminated radish plants are incubated for 6-7 days at about 18°C, under a humid atmosphere. Grading is carried out 6 to 7 days after the contamination, in comparison with the control plants. Under these conditions, good (at least 50%) or total protection is observed at a dose of 500 g/ha with the following compounds: 10, 29, 30, 31, 44.

### Example B: in vivo test on Septoria nodorum (wheat Glume blotch):

An aqueous suspension, at a concentration of 1.5%, of the active material tested is obtained by grinding it finely in a formulation of concentrated suspension type. This aqueous suspension is then diluted in water so as to obtain the desired active material concentration.
Wheat plants (Scipion variety) in starter cups, sown on a 50/50 peat soil-pozzolana substrate and grown at 12°C, are treated at the 1-leaf stage (10 cm tall) by spraying them with the aqueous suspension described above. Plants, used as controls, are treated with an aqueous solution not containing the active material. After 24 hours, the plants are contaminated by spraying with an aqueous suspension of *Septoria nodorum* spores (500,000 spores per cm³). The spores are collected from a seven-day-old culture. The contaminated wheat plants are incubated for 72 hours at about 18°C, under a humid atmosphere, and then for 14 days at 90% relative humidity.
Grading is carried out 15 to 20 days after contamination, in comparison with the control plants. Under these conditions, good (at least 50%) or total protection is observed, at a dose of 500 g/ha, with the following compounds: 10, 29, 31.

### Example C: in vivo test on Erisyphe graminis f. sp. tritici (powdery mildew of wheat):

An aqueous suspension, at a concentration of 1.5%, of the active material tested is obtained by grinding it finely in a formulation of concentrated suspension type. This aqueous suspension is then diluted in water so as to obtain the desired active material concentration. Wheat plants (Audace variety) in starter cups, sown on 50/50 peat soil-pozzulana substrate and grown at 12°C, are treated at the 1-leaf stage (10 cm tall) by spraying with the aqueous suspension described above. Plants, used as controls, are treated with an aqueous solution not containing the active material. After 24 hours, the plants are contaminated by dusting them with *Erisyphe graminis* f. sp. *tritici* spores, the dusting being carried out using diseased plants.
Grading is carried out 7 to 14 days after the contamination, in comparison with the control plants. Under these conditions, good (at least 50%) or total protection is observed, at a dose of 500 g/ha, with the following compounds: 10, 18, 19, 21, 29, 30, 31, 32, 44, 52, 53, 68.

### Example D: in vivo test on Septoria tritici (Leaf spot of wheat):

An aqueous suspension, at a concentration of 1.5%, of the active material tested is obtained by grinding it finely in a formulation of concentrated suspension type. This aqueous suspension is then diluted in water so as to obtain the desired active material concentration. Wheat plants (Scipion variety) in starter cups, sown on a 50/50 peat soil-pozzolana substrate and grown at 12°C, are treated at the 1-leaf stage (10 cm tall) by spraying with the aqueous suspension described above. Plants, used as controls, are treated with an aqueous solution not containing the active material.
After 24 hours, the plants are contaminated by spraying them with an aqueous suspension of *Septoria tritici* spores (500,000 spores per mL). The spores are collected from a 15-day-old culture and are suspended in a nutrient solution composed of:
- 1.5 g/L of gelatine
- 0.5 g/L of sodium oleate
- 24 g/L of PDB

The contaminated wheat plants are incubated for 72 hours at about 20°C and at 100% relative humidity, and then for 15 days at 80% relative humidity.
Grading is carried out 15 to 20 days after the contamination, in comparison with the control plants. Under these conditions, good (at least 50%) protection is observed, at a dose of 500 g/ha, with the following compounds: 10, 29.

### Example E: in vivo test on Drechslera teres (Barley Net blotch):

An aqueous suspension, at a concentration of 1.5%, of the active material tested is obtained by grinding it finely in a formulation of concentrated suspension type. This aqueous suspension is then diluted in water so as to obtain the desired active material concentration. Barley plants (Express variety) in starter cups, sown on a 50/50 peat soil-pozzolana substrate and grown at 12°C, are treated at the 1-leaf stage (10 cm tall) by spraying with the aqueous suspension described above. Plants, used as controls, are treated with an aqueous solution not containing the active material.
After 24 hours, the plants are contaminated by spraying them with an aqueous suspension of *Dreschslera teres* spores (12,000 spores per mL). The spores are collected from a 12-day-old culture. The contaminated wheat plants are incubated for 24 hours at about 20°C and at 100% relative humidity, and then for 12 days at 80% relative humidity.
Grading is carried out 12 days after the contamination, in comparison with the control plants. Under these conditions, good (at least 50%) protection is observed, at a dose of 500 g/ha, with the compounds described as example: 21, 29.

### Example F: in vivo test on Rhynchosporium secalis (Barley leaf scald):

An aqueous suspension, at a concentration of 1.5%, of the active material tested is obtained by grinding it finely in a formulation of concentrated suspension type. This aqueous suspension is then diluted in water so as to obtain the desired active material concentration. Barley plants (Express or Barrack variety) in starter cups, sown on a 50/50 peat soil-pozzolana substrate and grown at 12°C, are treated at the 1-leaf stage (10 cm tall) by spraying with the aqueous suspension described above. Plants, used as controls, are treated with an aqueous solution not containing the active material.
After 24 hours, the plants are contaminated by spraying them with an aqueous suspension of *Rhynchosporium secalis* spores (800,000 spores per mL). The contaminated wheat plants are incubated for 48 hours at about 20°C and at 100% relative humidity, and then for 12/14 days at 80% relative humidity.
Grading is carried out 12/14 days after the contamination, in comparison with the control plants. Under these conditions, good (at least 50%) protection is observed, at a dose of 500 g/ha, with the following compound described as example: 32

### Example G: in vivo test on Puccinia recondita (Wheat brown rust):

An aqueous suspension, at a concentration of 1.5%, of the active material tested is obtained by grinding it finely in a formulation of concentrated suspension type, This aqueous suspension is then diluted in water so as to obtain the desired active material concentration. Wheat plants (Scipion variety) in starter cups, sown on a 50/50 peat soil-pozzolana substrate and grown at 12°C, are treated at the 1-leaf stage (10 cm tall) by spraying with the aqueous suspension described above. Plants, used as controls, are treated with an aqueous solution not containing the active material.
After 24 hours, the plants are contaminated by spraying them with an aqueous suspension of *Puccinia recondita* spores (150,000 spores per mL). The contaminated wheat plants are incubated for 24 hours at about 20°C and at 100% relative humidity, and then for 10 days at 70 % relative humidity.
Grading is carried out 10 days after the contamination, in comparison with the control plants. Under these conditions, good (at least 50%) protection is observed, at a dose of 500 g/ha, with the following compounds described as example: 10, 13, 21, 29, 30, 31, 32, 44, 52, 68.

### Example H: in vivo test on Botiytis cinerea (cucumber Grey mould):

An aqueous suspension, at a concentration of 1.5%, of the active material tested is obtained by grinding it finely in a formulation of concentrated suspension type. This aqueous suspension is then diluted in water so as to obtain the desired active material concentration. Cucumber plants (Marketer variety) in starter cups, sown on a 50/50 peat soil-pozzolana substrate and grown at 18- 20°C, are treated at the cotyledon Z11 stage by spraying with the aqueous suspension described above. Plants, used as controls, are treated with an aqueous solution not containing the active material.
After 24 hours, the plants are contaminated by depositing drops of an aqueous suspension of *Botrytis cinerea* spores (150,000 spores per ml) on upper surface of the leaves. The spores are collected from a 15-day-old culture and are suspended in a nutrient solution composed of:
- 20 g/L of gelatine
- 50 g/L of cane sugar
- 2 g/L of NH4NO3
- 1 g/L of KH2PO4

The contaminated cucumber plants are settled for 5/7 days in a climatic room at 15-11 °c (day/night) and at 80% relative humidity.
Grading is carried out 5/7 days after the contamination, in comparison with the control plants. Under these conditions, good (at least 50%) protection is observed, at a dose of 500 g/ha, with the following compounds described as example: 21, 58

## Claims

1. Compounds of general formula (I): 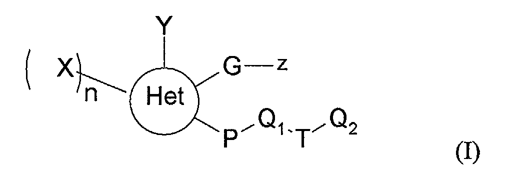 in which:
**Het** represents a five or six membered saturated, partially unsaturated or aromatic ring containing between one and six heteroatoms of the group N, O, S, in which the heterocycle is substituted in an adjacent manner with -P-Q¹-T-Q², -GZ and Y, such that the substituant -GZ is adjacent to both, each further ring members possibly being substituted by Xₙ;
**n** represents the integer 0, 1, 2 or 3;
**P** is chosen from among -C(O)-, -C(S)-, -SO₂-, -C(NR¹)-, -C(N-NR¹R²)-, -P(O)(OR¹)-, -P(O)(NR¹R²)-, and -P(O)R¹-;
**R**^{**1**} **and R**^{**2**} are the same or different and are each independently chosen from hydrogen, alkyl, cycloalkyl, aryl, heteroaryl, alkenyl, alkynyl, and sulphonyl, all optionally and independently from another substituted by one or several R⁵ and/or R⁶;
**G** represents oxy (-O-), thio (-S-), sulphinyl (-SO-) or a direct bond;
**Q**^{**1**} is chosen from -OR³, -SR³ and -NR³R⁴
**P and Q**^{**1**} may furthermore together form a ring containing from 5 to 7 atoms, 2 to 3 of which being chosen from nitrogen, oxygen and sulphur, and in which ring members may optionally be substituted with one or several R⁵;
**Q**^{**2**} represents R³, which is optionally and independently from another substituted by one or several R⁵ and/or R⁶;
**R**^{**3**} **and R**^{**4**}, which are the same or different, are each chosen, independently from one another, from:
* hydrogen;
* aryl, alkylaryl, cycloalkylaryl, bisaryl, heteroarylaryl, cycloalkylalkylaryl, arylalkylaryl, heteroarylalkylaryl, alkanoylaryl, cycloalkylcarbonylaryl, aroylaryl, hetaroylaryl, alkoxyaryl, cycloalkoxyaryl, aryloxyaryl, heteroaryloxyaryl, alkylaminoaryl, cycloalkylaminoaryl, arylaminoaryl, heteroarylaminoaryl, alkylthioaryl, cycloalkylthioaryl, arylthioaryl, heteroarylthioaryl, where all aryl, alkyl, cycloalkyl, heteroaryl may optionally and independently from another be substituted by one or several R⁵ and/or R⁶;
* heteroaryl, alkylheteroaryl, cycloalkylheteroaryl, arylheteroaryl, bisheteroaryl, cycloalkylalkylheteroaryl, arylalkylheteroaryl, heteroarylalkylheteroaryl, alkanoylheteroaryl, cycloalkylcarbonylheteroaryl, aroylheteroaryl, hetaroylheteroaryl, alkoxyheteroaryl, cycloalkoxyheteroaryl, aryloxyheteroaryl, heteroaryloxyheteroaryl, alkoxyalkylheteroaryl, cycloalkoxyalkylheteroaryl, aryloxyalkylheteroaryl, heteroaryloxyalkylheteroaryl, alkylaminoalkylheteroaryl, cycloalkylaminoalkylheteroaryl, arylaminoalkylheteroaryl, heteroarylaminoalkylheteroaryl, alkylaminoalkylheteroaryl, cycloalkylaminoalkylheteroaryl, arylaminoalkylheteroaryl, heteroarylaminoalkylheteroaryl, alkylthioheteroaryl, cycloalkylthioheteroaryl, arylthioheteroaryl, heteroarylthioheteroaryl, alkylthioalkylheteroaryl, cycloalkylthioalkylheteroaryl, arylthioalkylheteroaryl, heteroarylthioalkylheteroaryl, where all aryl, alkyl, cycloalkyl, heteroaryl may optionally and independently from another be substituted by one or several R⁵ and/or R⁶;
* alkyl, cycloalkylalkyl, arylalkyl, heteroarylalkyl, alkanoylalkyl, cycloalkylcarbonylalkyl, aroylalkyl, hetaroylalkyl, alkoxyalkyl, cycloalkoxyalkyl, aryloxyalkyl, heteroaryloxyalkyl, alkylaminoalkyl, cycloalkylaminoalkyl, arylaminoalkyl, heteroarylaminoalkyl, alkylthioalkyl, cycloalkylthioalkyl, arylthioalkyl, heteroarylthioalkyl, where all aryl, alkyl, cycloalkyl, heteroaryl may optionally and independently from another be substituted by one or several R⁵ and/or R⁶;
* cycloalkyl, alkylcycloalkyl, spirocycloalkyl, biscycloalkyl, arylcycloalkyl, heteroarylcycloalkyl, cycloalkylalkylcycloalkyl, arylalkylcycloalkyl, heteroarylalkylcycloalkyl, alkanoylcycloalkyl, cycloalkylcarbonylcycloalkyl, arylalkylcycloalkyl, heteroarylalkylcycloalkyl, alkoxycycloalkyl, cycloalkoxycycloalkyl, aryloxycycloalkyl, heteroaryloxycycloalkyl, alkoxyalkylcycloalkyl, cycloalkoxyalkylcycloalkyl, aryloxyalkylcycloalkyl, aryloxyalkylcycloalkyl, alkylaminoalkylcycloalkyl, cycloalkylaminoalkylcycloalkyl, arylaminoalkylcycloalkyl, heteroarylaminoalkylcycloalkyl, alkylaminoalkylcycloalkyl, cycloalkylaminoalkylcycloalkyl, arylaminoalkylcycloalkyl, heteroarylaminoalkylcycloalkyl, alkylthiocycloalkyl, cycloalkylthiocycloalkyl, arylthiocycloalkyl, heteroarylthiocycloalkyl, alkylthioalkylcycloalkyl, cycloalkylthioalkylcycloalkyl, arylthioalkylcycloalkyl, and heteroarylthioalkylcycloalkyl, where all aryl, alkyl, cycloalkyl, heteroaryl may optionally and independently from another be substituted by one or several R⁵ and/or R⁶;
**Z** is chosen from hydrogen, halogen, cyano, formyl, alkyl, cycloalkyl, allyl, aryl, heteroaryl, arylalkyl, propargyl, alkenyl, alkynyl, alkoxyalkyl, alkylthioalkyl, N-alkylaminoalkyl, N,N-dialkylaminoalkyl, alkanoylaminoalkyl, acyl, thioacyl, C₃-C₁₀ alkanoyl, aroyl, hetaroyl, alkoxycarbonyl, alkenoxycarbonyl, alkynyloxycarbonyl, aryloxycarbonyl, heteroaryloxycarbonyl, N-alkylaminocarbonyl, N,N-dialkylaminocarbonyl, alkylthiocarbonyl, N-alkylamidinyl, alkoxythiocarbonyl, C₁-C₆ alkylaminothiocarbonyl, alkylcarbonyl, alkoxycarbonyl, N-alkylaminocarbonyl, N,N-dialkylaminocarbonyl, N,N-dialkylaminothiocarbonyl, arylcarbonyl, heteroarylcarbonyl, aryloxycarbonyl, heteroaryloxycarbonyl, alkenylcarbonyl, alkenyloxycarbonyl, alkynylcarbonyl, alkynyloxycarbonyl, dialkylphosphatyl, diarylphosphatyl, alkylarylphosphatyl, trialkylsilyl, alkylarylsilyl, triarylsilyl, alkylsulphonyl, alkoxysulphonyl, aryloxysulphonyl, aminosulphonyl, N-alkylaminosulphonyl, N,N-dialkylaminosulphonyl, N-arylaminosulphonyl, N,N-diarylaminosulphonyl, arylsulphonyl, alkoxysulphinyl, alkylaminosulphinyl, alkylsulphinyl, and arylsulphinyl, where all alkyl, alkoxy, alkenyl, alkynyl, cycloalkyl, heteroaryl and aryl group may optionally be substituted by one or several R⁵ and/or R⁶;
**R**^{**5**} is chosen from cyano, cyanoalkyl, nitroalkyl, halogen, haloalkyl, hydroxy, hydroxyalkyl, mercapto, mercaptoalkyl, amino, aminoalkyl, hydrazino, nitrido, hydroxylamino, alkylhydroxylamino, alkoxamino, cycloalkoxamino, formyl, trialkylsilyl, triarylsilyl, thiocyanato, azido, dihydroxyphosphanyl, dialkoxyphosphanyl, alkoxy, haloalkoxy, cycloalkoxy, alkylthio, haloalkylthio, N-alkylamino, N,N-dialkylamino, N-haloalkylamino, N,N-halodialkylamino, alkoxylamino, haloalkoxamino, oxo, imino, alkoxyimino, haloalkoximino, hydrazono, alkylsulphonyl, arylsulphonyl, mono- or disubstituted aminosulphonyl, aroyl, alkanoyl, and mono- or disubstituted aminocarbonyl;
**R**^{**6**} is chosen form alkyl, cycloalkyl, alkoxyalkyl, alkylthioalkyl, N-alkylaminoalkyl, N,N-dialkylaminoalkyl, alkenyl, cycloalkenyl, alkoxyalkenyl, alkylthioalkenyl, N-alkylaminoalkenyl, N,N-dialkylaminoalkenyl, alkynyl, cycloalkynyl, alkoxyalkynyl, alkylthioalkynyl, N-alkylaminoalkynyl, N,N-dialkylaminoalkynyl, oxo, alkylimino, cycloalkylimino, alkoxyimino, alkoxyalkylimino, N-alkylaminoalkylimino, N,N-dialkylaminoalkylimino, hydrazono, alkylhydrazono, cycloalkylhydrazono, arylhydrazono, heteroarylhydrazono, alkanoyl, cycloalkanoyl, alkoxyalkanoyl, alkylthioalkanoyl, N-alkylaminoalkanoyl, N,N-dialkylaminoalkanoyl, aroyl, cycloaroyl, alkoxyaroyl, alkylthioaroyl, N-alkylaminoaroyl, N,N-dialkylaminoaroyl, aryl, cycloalkylaryl, alkoxyaryl, alkylthioalkyl, diaminoalkylaryl, alkylthio, cycloalkylthio, haloalkylthio, hydroxyalkylthio, aminoalkylthio, thioalkylthio, alkoxyalkylthio, alkylthioalkylthio, N-alkylaminoalkylthio, N,N-dialkylaminoalkylthio, alkylamino, cycloalkylamino, haloalkylamino, hydroxyalkylamino, thioalkylamino, aminoalkylamino, alkoxyalkylamino, alkoxamino, N-alkylaminoalkylamino, N,N-dialkylaminoalkylamino, alkoxylamino, alkylhydrazino, alkylsulphonylamino, cycloalkylsulphonylamino, haloalkylsulphonylamino, arylsulphonylamino, heteroaryloxy, heteroarylthio, heteroarylamino, N-alkylaminocarbonyl, N-alkylaminocarbonyl, N,N-dialkylaminocarbonyl, alkoxycarbonyl, cycloalkoxycarbonyl, haloalkoxycarbonyl, hydroxyalkoxycarbonyl, N-alkylaminoalkoxycarbonyl, N,N-diaminoalkoxycarbonyl, alkylsulphonyl, cycloalkylsulphonyl, haloalkylsulphonyl, pentafluoroalkylsulphonyl, arylsulphonyl, N-alkylaminosulphonyl, N,N-dialkyaminosulphonyl, alkysulphoxidyl, cycloalkylsulphoxidyl, arylsulphinyl, and heteroarylsulphinyl, where all aryl, alkyl, cycloalkyl, heteroaryl may optionally and independently from another be substituted by one or several R⁵;
**Y** is chosen from
* azido, formyl, -C(O)R⁷, -SH, thiocyanato, -SiR⁷R⁸R⁹;
* -NH₂, -NR⁷R⁸, -NR⁷-NR⁸R⁹, -ONR⁷R⁸, -ON=CR⁷R⁸, -NR⁷NR⁸(CO)R⁹, -NR⁷N=CR⁸R⁹;
* -NR⁷C(O)OR⁸, -NR⁷C(O)SR⁸, -NR⁷C(O)NR⁸R⁹, -NR⁷C(S)OR⁸, -NR⁷C(S)SR⁸;
* -NR⁷C(S)NR⁸R⁹, -NR⁷C(NR⁸)OR⁹, -NR⁷C(NR⁸)SR⁹, -NR⁷C(NR⁸)NR⁹R¹⁰, -NR⁷SOₙR⁷, -NR⁷SOₙOR⁷, -NR⁷SOₙNR⁷R⁸, -NR⁷SOₙSR⁷, wherein n represents 1 or 2;
* -P(O)(OR⁷)₂, -P(O)(NR⁷R⁸)₂, -P(O)R⁷R⁸;
* cyclobutyl, cyclopropyl, cyclohexyl;
* -(CR⁷R⁸)ₙ-R⁵, -(CR⁷R⁸)ₙ-R⁶ wherein represents an integer from 1 to 10, except when R⁵ represents halogen and R⁶ represents alkenyl or alkynyl;
* alkoxycarbonylaminoalkyl, haloalkoxycarbonylaminoalkyl, cycloalkoxycarbonylaminoalkyl;
* cycloalkyloxy, alkenyloxy, alkenylamino, alkynyloxy, alkenylthio, alkynylthio;
* R⁷OC(O)O-, R⁷SC(O)O-, R⁷R⁸NC(O)O-, R⁷OC(S)O-, R⁷SC(S)O-, R⁷R⁸NC(S)O-, R⁷OC(NR⁹)O-, R⁷SC(NR⁹)O-, R⁷R⁸NC(NR⁹)O-, alkanoyloxy, alkenoyloxy, alkynoyloxy, aroyloxy, hetaroyloxy;
* alkoxycarbonylaminoalkyl;
* -C(O)OH, -C(O)OR⁷, -C(O)SR⁷, -C(O)NR⁷R⁸, -C(S)OR⁷, -C(S)SR⁷, -C(S)NR⁷R⁸;
* -C(=NR⁷R⁸)H, -C(=NR⁷)OR⁸, -C(=NR⁷)SR⁸, -C(=NR⁷)NR⁸R⁹
* -S(O)R⁷, -SO₂R⁷, -SO₃R⁷, -SO₂NR⁷R⁸, -SO₂SR⁷, pentafluorophenylsulphonyl, alkoxysulphinyl;
* N-alkylaminosulphinyl, and N,N-dialkylaminosulphinyl; and
* when **Het** represents an unsaturated or partially saturated ring, Y may also be selected from =O, =NR⁶ and =S;
* when G represents oxygen and Z represents alkyl, or when G is a direct bond and Z represents hydrogen, Y may also be chosen from halogen, nitro, cyano and alkylthio, cycloalkyl, alkenyl, alkynyl, aryl and heteroaryl;
all alkyl, cycloalkyl, alkenyl, alkynyl, aryl and heteroaryl may optionally be substituted with one or several R⁵ and/or R⁶;
**R**^{**7**}**, R**^{**8**}**, R**^{**9**} **and R**^{**10**}, which may be the same or different, are each chosen from hydrogen, linear or branched C₁-C₁₀ alkyl, cycloalkyl, alkenyl, alkynyl, aryl, heteroaryl, heterocyclyl, each being optionally substituted independently from another with one or several R⁵ and/or R⁶;
R⁷ and R⁸ on the one hand and R⁹ and R¹⁰ on the other hand, may further together form a saturated, partially saturated or unsaturated 3-8 membered ring containing optionally 0 to 3 heteroatoms chosen from among O, N, and S;
**X**_{**n**} is chosen from
* hydrogen, halogen, -OH, -OR⁷, -ONR⁷R⁸, -SH, -SR⁷, -S(O)R⁷, -SO₂R⁷, -SO₂NR⁷R⁸, -C(O)R⁷, -C(O)OR⁷, -C(O)NR⁷R⁸, -NR⁷R⁸, nitro, thiocyanato, azido, cyano, pentafluorosulphonyl, -P(O)(OR⁷)₂, -P(O)(NR⁷R⁸)₂, -P(O)R⁷R⁸, oxo (=O) and imino (=R⁷),
* linear or branched C₁-C₁₀ alkyl, cycloalkyl, alkenyl, alkynyl, aryl, heteroaryl, and heterocyclyl, each of them being optionally substituted independently from another with one or several R⁵ and/or R⁶;
* Xₙ and Xₙ₊₁ may also be joined together, thus forming a saturated, partially unsaturated or totally unsaturated 4- to 8-membered ring optionally comprising one or more heteroatoms chosen from sulphur, oxygen, nitrogen and phosphorus; all alkyl, cycloalkyl, alkenyl, alkynyl, aryl and heteroaryl may optionally be substituted with one or several R⁵ and/or R⁶;
**T** is chosen from
* a direct bond,
* a divalent radical chosen from -(C[R₃]₂)ₘ-, -(C[R⁵]₂)ₘ-or -(CH₂)ₘ, where m can take a value between 1 and 12, limits included, and where the said radical can optionally be interrupted or ending with one or two heteroatoms chosen from nitrogen, oxygen and/or sulphur,
* oxyalkylene, alkoxyalkylene, carbonyl (-CO-), oxycarbonyl (-O-CO-), carbonyloxy (-CO-O-), sulphinyl (-SO-), sulphonyl (-SO₂-), oxysulphonyl (-O-SO₂-), sulphonyloxy (-SO₂-O-), oxysulphinyl (-O-SO-), sulphinyloxy (-SO-O-), thio (-S-), oxy (-O-), amino (-NR¹-), vinyl (-C=C-), ethynyl (-C≡C-), -NR¹-, -NR¹O-, -ONR¹-, -N=N-, -NR¹-NR²-, -NR¹-S-, -NR¹-SO-, -NR¹-SO₂-, -S-NR¹-, -SO-NR¹-, -SO₂-NR¹-, -CO-NR¹-O- or -O-NR¹-CO-;
as well as all eventual N-oxides, geometrical and/or optical isomers, enantiomers and/or diastereoisomers, tautomeric forms, salts and metal and metalloid complexes thereof.

2. Compounds according to Claim 1 for which **Het** (representing "heterocycle") represents saturated, partially unsaturated or aromatic monocycle or bicycles containing from 4 to 10 ring units, comprising at least one heteroatom chosen from nitrogen, oxygen, sulphur, silicon and phosphorus.

3. Compounds according to Claim 1or 2, for which the sub-unit P-Q¹-T-Q² is more precisely defined such that:
* P is chosen from -C(O)-, -C(S)-, -SO₂-, and -P(O)(OR¹)-,
* Q¹ represents -NR³R⁴, where R³ represents hydrogen and where R⁴ is chosen from alkyl, cycloalkyl, aryl, heteroaryl optionally substituted with one or several identical or different R⁵ and/or R⁶,
* T is chosen from a direct bond, -(CH₂)ₘ-, where m can take a value between 1 and 6, limits included, and where the said radical can optionally be interrupted or ending with one or two heteroatoms chosen from nitrogen, oxygen and/or sulphur, an oxyalkylene, alkoxyalkylene, carbonyl (-CO-), oxycarbonyl (-O-CO-), carbonyloxy (-CO-O-), sulphinyl (-SO-), sulphonyl (-SO₂-), oxysulphonyl (-O-SO₂-), sulphonyloxy (-SO₂-O-), oxysulphinyl (-O-SO-), sulphinyloxy (-SO-O-), thio (-S-), oxy (-O-), amino (-NR¹-), vinyl (-C=C-), ethynyl (-C≡C-), -NR¹-, -NR¹O-, -ONR¹-, -N=N-, -NR¹-NR²-, -NR¹-S-, -NR¹-SO-, -NR¹-SO₂-, -S-NR¹-, -SO-NR¹-, -SO₂-NR¹-, -CO-NR¹-O- and -O-NR¹-CO- radical,
* Q² is chosen from hydrogen, alkyl, cycloalkyl, aryl, and heteroaryl optionally substituted with one or several R⁵ and/or R⁶ independently chosen from another, the other substituants being as defined in Claim 1, as well as the eventual N-oxides, geometrical and/or optical isomers and enantiomers, tautomeric forms, salts and metal and metalloid complexes thereof.

4. Compounds according to any of Claims 1 to 3, wherein Y is chosen from -SiR⁷R⁸R⁹, -NH₂, -NR⁷R⁸, -NR⁷-NR⁸R⁹, -ONR⁷R⁸, -ON=CR⁷R⁸, -NR⁷NR⁸(CO)R⁹, -NR⁷N=CR⁸R⁹, -NR⁷C(O)OR⁸, -NR⁷C(O)SR⁸, -NR⁷C(O)NR⁸R⁹, -NR⁷C(S)OR⁸, -NR⁷C(S)SR⁸, -NR⁷C(S)NR⁸R⁹, -NR⁷C(NR⁸)OR⁹, -NR⁷C(NR⁸)SR⁹, -NR⁷C(NR⁸)NR⁹R¹⁰, -NR⁷SOₙR⁷, -NR⁷SOₙOR⁷, -NR⁷SOₙNR⁷R⁸, -NR⁷SOₙSR⁷, where n represents 0, 1 or 2, cycloalkyloxy, alkenyloxy, alkenylamino, alkynyloxy, alkenylthio, alkynylthio, R⁷OC(O)O-, R⁷SC(O)O-, R⁷R⁸NC(O)O-, R⁷OC(S)O-, R⁷SC(S)O-, R⁷R⁸NC(S)O-, R⁷OC(NR⁹)O-, R⁷SC(NR⁹)O-, R⁷R⁸NC(NR⁹)O-, alkanoyloxy, alkenoyloxy, alkynoyloxy, aroyloxy, hetaroyloxy, -C(O)OH, -C(O)OR⁷, -C(O)SR⁷, -C(O)NR⁷R⁸, -C(S)OR⁷, -C(S)SR⁷, -C(S)NR⁷R⁸, -C(=NR⁷R⁸)H, -C(=NR⁷)OR⁸, -C(=NR⁷)SR⁸, -C(=NR⁷)NR⁸R⁹, -S(O)R⁷, -SO₂R⁷, -SO₃R⁷, -SO₂NR⁷R⁸, -SO₂SR⁷, pentafluorophenylsulphonyl, alkoxysulphinyl, N-alkylaminosulphinyl, N,N-dialkylaminosulphinyl, arylsulphinyl, and alkoxycarbonyloxy,
when Het represents an unsaturated ring, Y may also be selected from =O, =NR⁶ and =S;
* when G represents oxygen and Z represents alkyl, or when G is a direct bond and Z represents hydrogen, Y may also be chosen from halogen, nitro, cyano, alkylthio, cycloalkyl, alkenyl, alkynyl, aryl and heteroaryl;
the other substituants being as defined in Claim 1,
as well as the eventual N-oxides, geometrical and/or optical isomers and enantiomers, tautomeric forms, salts and metal and metalloid complexes thereof.

5. Compounds according to any of the preceding Claims, for which Y is chosen from
-SiR⁷R⁸R⁹,
-NH₂, -NR⁷R⁸, -NR⁷-NR⁸R⁹, -ONR⁷R⁸, -ON=CR⁷R⁸, -NR⁷NR⁸(CO)R⁹,
-NR⁷N=CR⁸R⁹,
-NR⁷C(O)OR⁸, -NR⁷C(O)SR⁸, -NR⁷C(O)NR⁸R⁹, -NR⁷C(S)OR⁸, -NR⁷C(S)SR⁸,
-NR⁷C(S)NR⁸R⁹, -NR⁷C(NR⁸)OR⁹, -NR⁷C(NR⁸)SR⁹, -NR⁷C(NR⁸)NR⁹R¹⁰,
-C(O)OH, -C(O)OR⁷, -C(O)SR⁷, -C(O)NR⁷R⁸, -C(S)OR⁷, -C(S)SR⁷, -C(S)NR⁷R⁸,
-C(=NR⁷R⁸)H, -C(=NR⁷)OR⁸, -C(=NR⁷)SR⁸, -C(=NR⁷)NR⁸R⁹,
-S(O)R⁷, -SO₂R⁷, -SO₃R⁷, -SO₂NR⁷R⁸, -SO₂SR⁷, pentafluorophenylsulphonyl, alkoxysulphinyl, N-alkylaminosulphinyl, N,N-dialkylaminosulphinyl, arylsulphinyl, alkoxycarbonyloxy, substituants R⁷, R⁸, R⁹ as being as previously defined,
the other substituants being as defined in Claim 1,
as well as the eventual N-oxides, geometrical and/or optical isomers and enantiomers, tautomeric forms, salts and metal and metalloid complexes thereof.

6. Compounds according to Claim 1, having the following characteristics, taken separately or in combination:
Xₙ is chosen from hydrogen, alkyl, -SO₂R⁷, -C(O)OR⁷, and -P(O)(OR⁷)₂,
G represents oxygen, sulphur or a direct bond,
Z is chosen from hydrogen, halogen, alkyl, allyl, propargyl, alkanoyl, aroyl, hetaroyl, alkoxycarbonyl, alkenoxycarbonyl, aryloxycarbonyl, heteroaryloxycarbonyl, N-alkylaminocarbonyl, N,N-dialkylaminocarbonyl, alkylthiocarbonyl, alkoxythiocarbonyl, N-alkylaminothiocarbonyl, alkylcarbonyl, alkoxycarbonyl, N-alkylaminocarbonyl, N,N-dialkylaminocarbonyl, N,N-dialkylaminothiocarbonyl, dialkylphosphatyl, diarylphosphatyl, alkylarylphosphatyl, trialkylsilyl, alkylarylsilyl, triarylsilyl, alkylsulphonyl, arylsulphonyl, alkoxysulphonyl, aryloxysulphonyl, aminosulphonyl, N-alkylaminosulphonyl, N,N-dialkylaminosulphonyl, N-arylaminosulphonyl,, N,N-diarylaminosulphonyl, alkoxysulphinyl, alkylaminosulphinyl, alkylsulphinyl and arylsulphinyl, where all alkyl, alkoxy, alkenyl, alkynyl, cycloalkyl, heteroaryl and aryl group may optionally be substituted by one or several R⁵ and/or R⁶,
Y is chosen from trialkylsilyl, amino, -NR⁷C(O)OR⁸, -NR⁷C(O)NR⁸R⁹, -NR⁷C(O)OR⁸, -NR⁷SOₙR⁷ with n = 1 or 2, S(O)R⁷, -SO₂R⁷, -SO₃R⁷, -CO₂H, C(O)OR⁷, -C(=NR⁷)H, -C(O)NR⁷R⁸; Y can also be =O, =NR⁶, =S, where Het signifies an unsaturated ring;
Y can also be halogen, nitro, alkylthio and alkoxy when G = oxygen and Z = alkyl, cycloalkyl, alkenyl, alkynyl, aryl, heteroaryl or when G = bond and Z = H,
P represents -C(O)- or -SO₂-,
T represents a direct bond, or is chosen from oxy (-O-), thio (-S-), carbonyl -C(O)-and -(CH₂)ₘ, m being an integer from 1 to 6,
Q¹ represents -NR³R⁴, where R³ represents hydrogen and R⁴ is chosen from alkyl, cycloalkyl, aryl, heteroaryl optionally substituted with one or several R⁵ and/or R⁶ independently chosen from another,
Q² is chosen from hydrogen, alkyl, cycloalkyl, aryl, heteroaryl optionally substituted with one or several R⁵ and/or R⁶ independently chosen from another,
the other substituants being as defined in Claim 1, as well as the optional N-oxides, geometrical and/or optical isomers, enantiomers and/or diastereoisomers, tautomeric forms, salts and metal and metalloid complexes thereof.

7. Compounds according to Claim 1 having the following characteristics, taken individually or in combination:
Xₙ is chosen from hydrogen, alkyl, -SO₂R⁷, -C(O)OR⁷, and -P(O)(OR⁷)₂,
G represents a direct bond or oxygen,
Z is chosen from hydrogen, halogen, alkyl, alkoxycarbonyl, and alkanoyl,
Y is chosen from trialkylsilyl, amino, -NR⁷C(O)OR⁸, -SR⁷, -SO₂R⁷, -CO₂H, C(O)OR⁷, -C(=NR⁷)H, -C(O)NR⁷R⁸,
Y can also be =O, =NR⁶, =S, where Het signifies an unsaturated ring,
Y can also be halo, nitro, alkylthio and alkoxy when G = oxygen and Z = alkyl, cycloalkyl, alkenyl, alkynyl, aryl or heteroaryl or when G = bond and Z = H,
P represents -C(O)- or -SO₂-,
Q¹ represents -NR³R⁴, where R³ represents hydrogen and R⁴ is chosen from alkyl, cycloalkyl, aryl, and heteroaryl optionally substituted with one or several R⁵ and/or R⁶ independently chosen from another,
T represents a direct bond, or is chosen from oxygen, sulphur, -C(O)-, and -(CH₂)ₘ, m having a value of between 1 and 6,
Q² is chosen from hydrogen, alkyl, cycloalkyl, aryl, and heteroaryl optionally substituted with one or several R⁵ and/or R⁶ independently chosen from another,
the other substituants being as defined in Claim 1, as well as the optional N-oxides, geometrical and/or optical isomers, enantiomers and/or diastereoisomers, tautomeric forms, salts and metal and metalloid complexes thereof.

8. Compounds according to Claim 1, having the following characteristics:
Het represents thiophene,
Y is chosen from bromine, alkylthio, -SiR⁷R⁸R⁹, -SO₂R⁷, NH₂, -N=CR⁷R⁸, -NC(O)OR⁷, CO₂H, -C(=NR⁷)H, and -SO₂R⁷,
n is equal to 1,
X¹ represents hydrogen or alkylthio,
G represents oxygen,
Z is chosen from H, alkyl, alkanoyl, alkylsulphonyl, and arylsulphonyl,
P represents -C(O)-,
Q¹ represents -NR³R⁴,
T is a direct bond or represents -O-,
Q² is chosen from hydrogen and R³, R³ being alkyl or aryl,
R⁴ represents hydrogen,
R⁷ represents alkyl or aryl,
the other substituants being as defined in Claim 1, as well as the optional N-oxides, geometrical and/or optical isomers, enantiomers and/or diastereoisomers, tautomeric forms, salts and metal and metalloid complexes thereof.

9. Compounds according to Claim 1, having the following characteristics:
Het represents pyrrole,
Y is chosen from -NH₂, -NR⁷R⁸, -NC(O)OR⁷, CO₂H, -C(O)OR⁷, -C(O)NR⁷R⁸, -SO₂R⁷, and -CO₂H,
n represents 1 or 2,
X¹ represents hydrogen,
X² is chosen from alkyl, -SO₂R⁷, -C(O)OR⁷, and -P(O)(OR⁷)₂,
G represents -O-,
Z is chosen from hydrogen, alkyl, alkanoyl, alkylsulphonyl, and arylsulphonyl,
P represents -C(O)-,
Q¹ represents -NR³R⁴, or -OR³,
T represents a direct bond, or -O-,
Q² is chosen from hydrogen and R³,
R³ is chosen from alkyl, and aryl,
R⁴ represents hydrogen,
R⁷ and R⁸ which are the same or different are independently chosen from alkyl, alkenyl, and aryl, R⁷ and R⁸ optionally forming an unsaturated or saturated cycle,
the other substituants being as defined in Claim 1, as well as the optional N-oxides, geometrical and/or optical isomers, enantiomers and/or diastereoisomers, tautomeric forms, salts and metal and metalloid complexes thereof.

10. Compounds according to Claim 1, having the following characteristics:
Het represents pyrazole,
n represents 1,
X¹ is chosen from alkyl, -SO₂R⁷, -C(O)OR⁷, and -P(O)(OR⁷)₂,
Y is chosen from -NH₂, -NO₂, -NHC(O)OR⁷, -NHC(O) R⁷, -NR⁷R⁸, -SO₂R⁷, and -CO₂H,
G represents a direct bond or -O-,
Z is chosen from hydrogen, bromine, alkyl, alkanoyl, alkylsulphonyl, and arylsulphonyl,
P represents -C(O)-,
Q¹ is chosen from -NR³R⁴, and -OR³,
T represents a direct bond or -O-,
Q² is chosen from hydrogen and R³,
R³ is chosen from hydrogen, alkyl, and aryl,
R⁴ represents hydrogen,
R⁷ and R⁸ which are the same or different are independently chosen from hydrogen, alkyl, alkenyl, and aryl,
the other substituants being as defined in Claim 1, as well as the optional N-oxides, geometrical and/or optical isomers, enantiomers and/or diastereoisomers, tautomeric forms, salts and metal and metalloid complexes thereof.

11. Compounds according to Claim 1, having the following characteristics:
Het represents isothiazole,
n represents 0,
Y is chosen from -NH₂, -NO₂, -NHC(O)R⁷, -NR⁷R⁸, -SO₂R⁷, -NHC(O)OR⁷, and -CO₂H,
G represents a direct bond or -O-,
Z is chosen from hydrogen, bromine, alkyl, alkanoyl, alkylsulphonyl, and arylsulphonyl,
P represents -C(O)-,
Q¹ is chosen from -NR³R⁴, and -OR³,
T represents a direct bond or -O-,
Q² is chosen from hydrogen and R³,
R³ is chosen from hydrogen, alkyl, and aryl,
R⁴ represents hydrogen,
R⁷ and R⁸ which are the same or different are independently chosen from hydrogen, alkyl, alkenyl, and aryl,
the other substituants being as defined in Claim 1, as well as the optional N-oxides, geometrical and/or optical isomers, enantiomers and/or diastereoisomers, tautomeric forms, salts and metal and metalloid complexes thereof.

12. Compounds according to Claim 1, having the following characteristics:
Het represents isoxazole,
n is 0,
Y is chosen from -NH₂, -CO₂H, -NHC(O)OR⁷, -CO₂R⁷, -NR⁷R⁸, -SO₂R⁷, and -CO₂H,
G represents -O-,
Z is chosen from hydrogen, alkyl, alkanoyl, alkylsulphonyl, and arylsulphonyl,
P represents -C(O)-
Q1 is chosen from -NR³R⁴, and OR³,
T is a direct bond or is -O-,
Q² is chosen from hydrogen and R³,
R³ is chosen from alkyl and aryl,
R⁴ represents hydrogen,
R⁷ and R⁸, which are identical or different, are independently chosen from hydrogen, alkyl, alkenyl and aryl,
the other substituants being as defined in Claim 1, as well as the optional N-oxides, geometrical and/or optical isomers, enantiomers and/or diastereoisomers, tautomeric forms, salts and metal and metalloid complexes thereof.

13. Compounds according to Claim 1, having the following characteristics:
Het represents pyridine,
n is 1 or 2,
X¹ and X² identical or different are independently chosen from hydrogen, -SO₂R⁷, and -C(O)OR⁷,
Y is chosen from -NH₂, -NR⁷R⁸, -C(O)NR⁷R⁸, -SO₂R⁷, -NHC(O)OR⁷, -CO₂H, -CO₂R⁷, and -NHC(O)R⁷,
G represents -O-,
Z is chosen from hydrogen, alkyl, alkanoyl, alkylsulphonyl, and arylsulphonyl,
P is chosen from -C(O)-, -SO₂-, and -P(O)(OR⁷)-,
Q¹ is chosen from -NR³R⁴, and O-R³,
T is a direct bond or is -O-,
Q² represents R³,
R³ is chosen from alkyl, and aryl,
R⁴ represents hydrogen,
R⁷ and R⁸, which are identical or different, are independently chosen from alkyl, alkenyl and aryl, or R⁷ and R⁸ may together form an unsaturated or saturated cycle,
the other substituants being as defined in Claim 1, as well as the optional N-oxides, geometrical and/or optical isomers, enantiomers and/or diastereoisomers, tautomeric forms, salts and metal and metalloid complexes thereof.

14. Compounds according to Claim 1, having the following characteristics:
Het is chosen from pyrimidine and pyridazine,
n represents 1,
X¹ is chosen from hydrogen, alkyl, -SR⁷ and -SO₂R⁷,
Y is chosen from -NH₂, -NR⁷R⁸, CO₂H, -C(O)OR⁷, -C(O)NR⁷R⁸, -SO₂R⁷, -NHC(O)OR⁷,
G represents a direct bond or is -O-,
Z is chosen from hydrogen, alkyl, alkanoyl, alkylsulphonyl, and arylsulphonyl,
P represents -C(O)-,
Q1 is chosen from -NR³R⁴, and -OR³,
T represents a direct bond or is -O-,
Q² is chosen from hydrogen and R³,
R³ is chosen from alkyl and aryl,
R⁴ represents hydrogen,
R⁷ and R⁸, which are identical or different, are independently chosen from alkyl, alkenyl and aryl, or R⁷ and R⁸ may together form an unsaturated or saturated cycle,
the other substituants being as defined in Claim 1, as well as the optional N-oxides, geometrical and/or optical isomers, enantiomers and/or diastereoisomers, tautomeric forms, salts and metal and metalloid complexes thereof.

15. Compounds according to Claim 1, having the following characteristics:
Het represents pyranone,
n is chosen from 1 and 2,
X¹,X² which are identical or different, are independently chosen from hydrogen, alkyl, and alkylthio,
Y is chosen from =O, and =NR⁷,
G represents a direct bond or is -O-,
Z is chosen from hydrogen, alkyl, alkanoyl, alkylsulphonyl, and arylsulphonyl,
P represents -C(O)-,
Q¹ represents -NR³R⁴,
T represents a direct bond or is -O-,
Q² is chosen from hydrogen and R³,
R³ is chosen from alkyl and aryl,
R⁴ represents hydrogen,
the other substituants being as defined in Claim 1, as well as the optional N-oxides, geometrical and/or optical isomers, enantiomers and/or diastereoisomers, tautomeric forms, salts and metal and metalloid complexes thereof.

16. Process for preparing the compounds according to one of Claims 1 to 15, **characterised by** the reaction Scheme 1 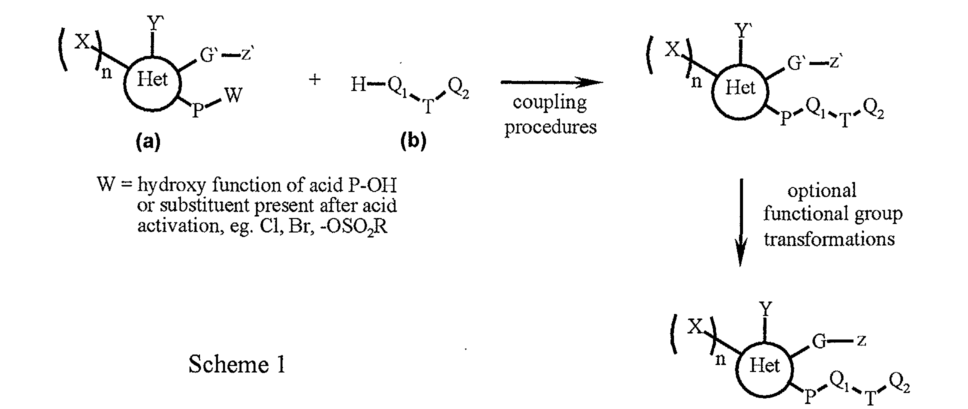 in which the various substituants are as defined in Claim 1,
by effecting a reaction between a substituted heterocyclic acid or acid derivative (a) with a nucleophilic compound of formula (b), wherein W represents a hydroxy group (-OH) present in the free acid -P-OH or a group obtained after activation of the free acid -P-OH.

17. Fungicidal compositions, comprising, as active material, an effective amount of at least one compound according to any one of Claims 1 to 15 or one of the N-oxides, geometrical and/or optical isomers, enantiomers and/or diastereoisomers, tautomeric forms, salts and metal and metalloid complexes thereof, that are agriculturally acceptable.

18. Fungicidal compositions according to Claim 17, comprising, besides the active material according to any one of Claims 1 to 15 or one of the N-oxides, geometrical and/or optical isomers, enantiomers and/or diastereoisomers, tautomeric forms, salts and metal and metalloid complexes thereof, that are agriculturally acceptable, an agriculturally acceptable solid or liquid support and/or a surfactant which is also agriculturally acceptable, as well as, optionally, one or more other fungicides, insecticides, herbicides, acaricides, attractants or pheromones and other compounds with biological activity.

19. Fungicidal compositions according to either of Claims 17 and 18, comprising from 0.05% to 99% by weight of active material.

20. Process for preventively or curatively combating the phytopathogenic fungi of crops, **characterised in that** an effective (agronomically effective) and non-phytotoxic amount of an active material of formula (I) according to one of Claims 1 to 15, or of a fungicidal composition comprising an active material of formula (I), is applied to the plant seeds or to the plant leaves and/or to the fruits of the plants or to the soil in which the plants are growing or in which it is desired to grow them.

21. Process according to Claim 20, in which rice, corn, cotton, cereals, fruit trees, oil-yielding crops, market-garden and vegetable crops, tomato, lettuce, protein-yielding crops, pea, solanacea plants, beetroot, flax and woodland trees, as well as genetically modified homologues of these crops, are treated.

22. Process according to either of Claims 20 and 21, in which the seeds of cereals, rice, corn, cotton, fruit trees, woodland trees, oil-yielding crops, protein-yielding crops, market-garden crops, solanacea plants, beetroot or flax, as well as genetically modified homologues of these crops, are treated.

23. Process according to any one of Claims 20 to 22, **characterised in that** the plants are genetically modified plants.

24. Process according to any one of Claims 20 to 23, in which the dose of active material applied is between 10 g and 800 g of active material per hectare, in the case of foliar treatments.

25. Process according to Claim 24, in which the dose of active material applied is between 50 g and 300 g of active material per hectare in the case of foliar treatments.

26. Process according to any one of Claims 20 to 23, in which the dose of active material applied is between 2 and 200 g of active material per 100 kg of seed, in the case of seed treatments.

27. Process according to Claim 26, in which the dose of active material applied is between 3 g and 150 g per 100 kg of seed, in the case of seed treatments.

28. Method for preventively or curatively treating lumber using the compounds according to one of Claims 1 to 15 or compositions containing them, against fungal attack, by direct application, sprinkling, dipping or injection.

29. Use of the compounds according to one of Claims 1 to 15 in human and animal therapy for the treatment of fungal diseases.
